# EUROPEAN PATENT SPECIFICATION

(11) **EP 3 356 556 B1**
(45) Date of publication and mention of the grant of the patent: **11.12.2019**
(21) Application number: 16787736.4
(22) Date of filing: 29.09.2016
(51) Int. Cl.: C12Q 1/6883

(54) **A METHOD FOR DIAGNOSING A DISEASE BY DETECTION OF CIRCRNA IN BODILY FLUIDS**
VERFAHREN ZUR DIAGNOSE EINER KRANKHEIT DURCH NACHWEIS VON CIRCRNA IN KÖRPERFLÜSSIGKEITEN
PROCÉDÉ DE DIAGNOSTIC D'UNE MALADIE PAR DÉTECTION D'ARNCIRC DANS LES FLUIDES BIOLOGIQUES

(30) Priority: 29.09.2015 EP 15187446
(43) Date of publication of application: 08.08.2018
(73) Proprietor: Max-Delbrück-Centrum für Molekulare Medizin in der Helmholtz-Gemeinschaft, 13125 Berlin (DE)
(72) Inventor: RAJEWSKY, Nikolaus, 10435 Berlin (DE); MEMCZAK, Sebastian, 10245 Berlin (DE); PAPAVASILEIOU, Panagiotis, 33602 Bielefeld (DE)
(74) Representative: Vossius & Partner Patentanwälte Rechtsanwälte mbB
(86) International application number: PCT/EP2016/073321
(87) International publication number: WO 2017/055487

(56) References cited:
- WO-A2-2012/050975
- AGNIESZKA RYBAK-WOLF ET AL: "Circular RNAs in the Mammalian Brain Are Highly Abundant, Conserved, and Dynamically Expressed", MOLECULAR CELL., vol. 58, no. 5, 4 June 2015 (2015-06-04), pages 870-885, XP055269076, US ISSN: 1097-2765, DOI: 10.1016/j.molcel.2015.03.027
- SEBASTIAN MEMCZAK ET AL: "Circular RNAs are a large class of animal RNAs with regulatory potency", NATURE, vol. 495, no. 7441, 27 February 2013 (2013-02-27), pages 333-338, XP055103972, ISSN: 0028-0836, DOI: 10.1038/nature11928
- JULIA SALZMAN ET AL: "Cell-Type Specific Features of Circular RNA Expression", PLOS GENETICS, vol. 9, no. 9, 5 September 2013 (2013-09-05), page e1003777, XP055200832, DOI: 10.1371/journal.pgen.1003777
- WALTER J LUKIW: "Circular RNA (circRNA) in Alzheimer's disease (AD)", FRONTIERS IN GENETICS, vol. 4, 31 December 2013 (2013-12-31), pages 1-2, XP055200738, ISSN: 1664-8021, DOI: 10.3389/fgene.2013.00307
- SUMAN GHOSAL ET AL: "Circ2Traits: a comprehensive database for circular RNA potentially associated with disease and traits", FRONTIERS IN GENETICS, vol. 4, 10 December 2013 (2013-12-10), XP055343806, DOI: 10.3389/fgene.2013.00283
- L CHENG ET AL: "Prognostic serum miRNA biomarkers associated with Alzheimer's disease shows concordance with neuropsychological and neuroimaging assessment", MOLECULAR PSYCHIATRY, 28 October 2014 (2014-10-28), XP055173008, ISSN: 1359-4184, DOI: 10.1038/mp.2014.127
- SEBASTIAN MEMCZAK ET AL: "Identification and Characterization of Circular RNAs As a New Class of Putative Biomarkers in Human Blood", PLOS ONE, vol. 10, no. 10, 20 October 2015 (2015-10-20), page e0141214, XP055344029, DOI: 10.1371/journal.pone.0141214

## Description

### Technical Field of the Invention

The present invention relates to the field of medicine and RNA biology, in particular it relates to the field of diagnosis of a disease using circRNAs, more particular the present invention relates to the field of diagnosis of Alzheimer's disease.

### Background of the Invention

Many diseases are associated with deregulation of gene expression. Such deregulation is in many cases detectable at early stages of the disease. In fact, detection of deregulated expression often serves as a biomarker for a disease or the risk for acquiring a disease before the disease manifests in terms of symptoms. However, diagnosis is often restricted to samples of the diseased tissue. In some cases, diagnosis also aims at the detection of biomarkers in easily accessible samples, such as blood. However, these methods are restricted to protein biomarkers and require cumbersome preparation of the samples, e.g. serum or plasma samples. The direct readout of expression, i.e. RNA, is in most cases not feasible in blood samples, as RNAs are prone to degradation. Hence, RNA nowadays is a poor biomarker in blood, in particular for diseases manifesting in tissues others than blood.

Regulatory RNAs such as microRNAs (miRNAs) or long non-coding RNAs (IncRNAs) have been implicated in many biological processes and human diseases such as cancer (reviewed in Batista PJ, Chang HY. Long Noncoding RNAs: Cellular Address Codes in Development and Disease. Cell. 2013;152(6):1298-1307; and Cech TR, Steitz JA. The Noncoding RNA Revolution-Trashing Old Rules to Forge New Ones. Cell. 2014;157(1):77-94). Recent studies have drawn attention to a new class of RNA that is endogenously expressed as single-stranded, covalently closed circular molecules (circRNA, reviewed in Jeck WR, Sharpless NE. Detecting and characterizing circular RNAs. Nature Biotechnology. 2014;32(5):453-461). Most circRNAs are probably products of a 'back-splice' reaction that joins a splice donor site with an upstream splice acceptor site *(see* Ashwal-Fluss R, Meyer M, Pamudurti NR, et al. circRNA Biogenesis Competes with Pre-mRNA Splicing. MOLCEL. 2014;1-12; and Starke S, Jost I, Rossbach O, et al. Exon Circularization Requires Canonical Splice Signals. Cell Reports. 2015;10(1):103-111). Circular RNA is known for several decades from viroids, viruses and plants, but until recently only few mammalian circRNAs were reported. Sequencing based studies lately revealed that circRNAs are abundantly and prevalently expressed across life, oftentimes in a tissue and developmental-stage specific manner (*see* Danan M, Schwartz S, Edelheit S, Sorek R. Transcriptome-wide discovery of circular RNAs in Archaea. Nucleic Acids Res. 2012; 40(7):3131-3142; Salzman J, Gawad C, Wang PL, Lacayo N, Brown PO. Circular RNAs are the predominant transcript isoform from hundreds of human genes in diverse cell types. PLoS ONE. 2012; 7(2):e30733; Jeck WR, Sorrentino JA, Wang K, et al. Circular RNAs are abundant, conserved, and associated with ALU repeats. RNA. 2013;19(2):141-157; Memczak S, Jens M, Elefsinioti A, et al. Circular RNAs are a large class of animal RNAs with regulatory potency. Nature. 2013;495(7441):333-338; Salzman J, Chen RE, Olsen MN, Wang PL, Brown PO. Cell-Type Specific Features of Circular RNA Expression. PLoS Genetics. 2013; 9(9):e1003777; Wang PL, Bao Y, Yee M-C, et al. Circular RNA is expressed across the eukaryotic tree of life. PLoS ONE. 2014;9(3):e90859; Guo JU, Agarwal V, Guo H, Bartel DP. Expanded identification and characterization of mammalian circular RNAs. Genome Biology. 2014;1-14; and You X, Vlatkovic I, Babic A, et al. Neural circular RNAs are derived from synaptic genes and regulated by development and plasticity. Nat Neurosci. 20117-25). The vast majority of circRNAs consists of 2-4 exons of protein coding genes, but they can also derive from intronic, non-coding, antisense, 5' or 3' untranslated or intergenic genomic regions (*see* Memczak S, Jens M, Elefsinioti A, et al. Circular RNAs are a large class of animal RNAs with regulatory potency. Nature. 2013; 495(7441):333-338; and Zhang Y, Zhang X-O, Chen T, et al. Circular Intronic Long Noncoding RNAs. MOLCEL. 2013; 1-15). Although not fully understood, the biogenesis of many mammalian circRNAs depends on complementary sequences within flanking introns *(see* Ashwal-Fluss R, Meyer M, Pamudurti NR, et al. circRNA Biogenesis Competes with Pre-mRNA Splicing. MOLCEL. 2014; 1-12; Rybak-Wolf A, Stottmeister C, Glažar P, et al. Circular RNAs in the Mammalian Brain Are Highly Abundant, Conserved, and Dynamically Expressed. MOLCEL. 2015;1-17; Zhang X-O, Wang H-B, Zhang Y, et al. Complementary Sequence-Mediated Exon Circularization. Cell. 2014; Liang D, Wilusz JE. Short intronic repeat sequences facilitate circular RNA production. Genes and Development. 2014; Conn SJ, Pillman KA, Toubia J, et al. The RNA Binding Protein Quaking Regulates Formation of circRNAs. Cell. 2015;160(6):1125-1134; and Ivanov A, Memczak S, Wyler E, et al. Analysis of Intron Sequences Reveals Hallmarks of Circular RNA Biogenesis in Animals. CellReports. 2015;10(2):170-177) and their expression can be modulated by antagonistic or activating trans-acting factors such as ADAR and Quaking (*see* Ivanov A, Memczak S, Wyler E, et al. Analysis of Intron Sequences Reveals Hallmarks of Circular RNA Biogenesis in Animals. Cell Reports. 2015; 10(2):170-177; and Conn SJ, Pillman KA, Toubia J, et al. The RNA Binding Protein Quaking Regulates Formation of circRNAs. Cell. 2015;160(6):1125-1134; respectively). Although the function of animal circRNAs is largely unknown, it was demonstrated that the circRNAs CDR1as (ciRS-7) and SRY can act as antagonists of specific miRNAs by functioning as miRNA sponges *(see* Memczak S, Jens M, Elefsinioti A, et al. Circular RNAs are a large class of animal RNAs with regulatory potency. Nature. 2013;495(7441):333-338; and Hansen TB, Jensen TI, Clausen BH, et al. Natural RNA circles function as efficient microRNA sponges. Nature. 2013;495(7441):384-388). Moreover, stable knockdown of CDRlas caused a migration defect in cell culture and a circRNA produced from the muscleblind transcript can bind muscleblind protein and likely regulate its expression levels *(see* Memczak S, Jens M, Elefsinioti A, et al. Circular RNAs are a large class of animal RNAs with regulatory potency. Nature. 2013;495(7441):333-338; and Ashwal-Fluss R, Meyer M, Pamudurti NR, et al. circRNA Biogenesis Competes with Pre-mRNA Splicing. MOLCEL. 2014;1-12). Besides these specific functions for the few in-depth analyzed circRNAs, a recent study uncovered a putatively more general competition mechanism between linear RNA splicing and co-transcriptional circular RNA splicing (*see* Ashwal-Fluss R, Meyer M, Pamudurti NR, et al. circRNA Biogenesis Competes with Pre-mRNA Splicing. MOLCEL. 2014;1-12). The lack of free ends, i.e. its circularity, renders circRNAs resistant to exonucleolytic activities within cells and in extracellular environments. Thus, circRNAs are stable molecules as demonstrated by their long half lives in cells a feature that distinguishes them from canonical linear RNA isoforms (*see* Cocquerelle C, Daubersies P, Majérus MA, Kerckaert JP, Bailleul B. Splicing with inverted order of exons occurs proximal to large introns. EMBO J. 1992;11(3):1095-1098; Jeck WR, Sorrentino JA, Wang K, et al. Circular RNAs are abundant, conserved, and associated with ALU repeats. RNA. 2013;19(2):141-157; and Memczak S, Jens M, Elefsinioti A, et al. Circular RNAs are a large class of animal RNAs with regulatory potency. Nature. 2013; 495(7441):333-338)

Lukiw, W.J., Frontiers in Genetics (2013)4(307):1-2 teaches the presence of a circRNA in hippocampal samples of a AD patient.

The inventors now for the first time show the presence of a plurality of ten thousands of circRNAs in standard clinical whole blood specimen of diseased subjects and thereby show that circRNAs function as biomarkers in human disorders, in particular neurodegenerative disorders, as exemplified by Alzheimer's disease. Strikingly, the mRNA transcripts which give rise to circRNAs were in hundreds of cases almost not detectable while the corresponding circRNAs were highly expressed, underlining the significance of circRNAs as novel biomarkers. Approaches have been performed to detect circRNAs as biomarkers in blood. However, these approaches use processed blood. Blood-exosomes have been postulated as comprising circRNAs (Li, Yan et al. (2015); Circular RNA is enriched and stable in exosomes: a promising biomarker for cancer diagnosis; Cell Res, 25(8): 981-984). However, blood-exosomes are difficultly obtainable and the cumbersome procedure renders the procedure susceptible to errors and the significance of the so obtained circRNA levels questionable. The present inventors however showed that circRNAs in unprocessed samples, e.g. whole blood, are detectable and are surprisingly well suited as biomarkers.

Alzheimer's disease (also referred to as "AD"), is the cause for 60% to 70% of cases of dementia. It is a chronic neurodegenerative disease starting slowly and getting worse over time. One of the first symptoms is a short-term memory loss. As the disease advances, symptoms can include problems with language, disorientation (including easily getting lost), mood swings, loss of motivation, not managing self care, and behavioural issues. As a person's condition declines, she or he often withdraws from family and society. Gradually, bodily functions are lost, ultimately leading to death. Although the speed of progression can vary, the average life expectancy following diagnosis is three to nine years. The cause of Alzheimer's disease is poorly understood. About 70% of the risk is believed to be genetic with many genes usually involved. Other risk factors include a history of head injuries, depression, or hypertension. The disease process is associated with plaques and tangles in the brain.

Currently, the diagnosis of Alzheimer's disease is based on the history of the illness and cognitive testing. These tests are often substituted by medical imaging and blood tests to rule out other possible causes. Initial symptoms are often mistaken for normal ageing. Today, examination of brain tissue is needed for a definite diagnosis. However, brain tissue is not easily accessible and the surgical intervention causes severe dangers. Mental and physical exercise, and avoiding obesity may decrease the risk of AD.

In 2010, there were between 21 and 35 million people worldwide with AD. It most often begins in people over 65 years of age, although 4% to 5% of cases are early-onset Alzheimer's which begin before this. It affects about 6% of people 65 years and older. In 2010, dementia resulted in about 486,000 deaths. In developed countries, AD is one of the most financially costly diseases. There is a long felt need for a direct, easy and reliable diagnosis of Alzheimer's disease to allow intervention and prevention of adverse effects of the beginning or progressing mental degeneration.

The molecular underpinnings of AD are controversially debated; although substantial research efforts were made and are currently ongoing (annual budget for AD of the NIH in 2015 is $566 million). In particular there is an urgent need for biomarkers for AD since it is believed that the molecular alterations involved in the disease precede the symptoms years or even decades, hindering therapeutic interventions.

### Summary of the Invention

The present invention provides for a method that overcomes the above outlined drawbacks. The inventors have found that it is possible to detect circRNAs in samples of a bodily fluid in a great amount. The inventors further have proven that the circRNAs are indicative for a disease that is not a disease of the bodily fluid. Thereby, a tool is given to directly diagnose a disease by determining presence or absence of one or more circRNAs in a bodily fluid. The invention therefore provides for a new class of biomarkers in bodily fluids, e.g. blood.

Hence, the present invention relates to a method for diagnosing Alzheimer's disease (AD) of a subject, comprising the step of:
- determining the presence or absence of one or more circular RNA (circRNA) in a sample of blood of said subject;
wherein the presence or absence of said one or more circRNA is indicative for the disease.

As has been shown by the inventors, certain circRNAs may be present in samples of a diseased subject at differing levels as compared to samples from healthy subjects. Hence, it may be desirable to decide on "presence" or "absence" of a circRNA when compared to a control level. Hence, in a preferred embodiment of the method according to the present invention the determination step comprises:
- determining the level of said one or more circRNA;
- comparing the determined level to a control level of said one or more circRNA; wherein differing levels between the determined and the control level are indicative for the disease. Hence, the invention also relates to a method for diagnosing a disease of a subject, comprising the step of:
- determining the level of said one or more circRNA;
- comparing the determined level to a control level of said one or more circRNA;
wherein differing levels between the determined and the control level are indicative for the disease.

The invention also relates to a method for diagnosing Alzheimer's disease, in a subject comprising the steps of:
- determining the level of one or more circRNA in a sample of blood of said subject;
- comparing the determined level to a control level of said one or more circRNA;
wherein differing levels between the determined and the control level are indicative for the disease.

The inventors, furthermore, identified circRNAs that were not previously known to be present in blood. These novel blood circRNAs are listed in Table 1. Hence, also described is a nucleic acid probe specifically hybridizing to the sequence of nucleotide 11 to 30 of a sequence selected from the group consisting of SEQ ID NO:1 to 910, and a RNA sequence encoded by a sequence of SEQ ID NO:1 to 910,or specifically hybridizing to a reverse complement sequence thereof; preferably specifically hybridizing to a sequence selected from the group consisting of SEQ ID NO:1 to 910, and a RNA sequence encoded by a sequence of SEQ ID NO:1 to 910, or specifically hybridizing to a reverse complement sequence thereof.

Furthermore, the invention relates to a kit for diagnosing AD as defined in the appendent claims and discloses a kit for diagnosing a neurodegenerative disease, comprising means for specifically detecting one or more nucleic acid sequence encoded by a sequence selected from the group consisting of SEQ ID NO:1 to 910 or SEQ ID NO:911 to SEQ ID NO:1820.

Furthermore described is an array for determining the presence or level of a plurality of nucleic acids, comprising a plurality of probes, wherein the plurality of probes specifically hybridize to the sequence of nucleotide 11 to 30 of a sequence selected from the group consisting of SEQ ID NO:1 to SEQ ID NO:910, and a RNA sequence encoded by a sequence of SEQ ID NO:1 to 910, or to a reverse complement sequence thereof; preferably the plurality of probes comprises probes specifically hybridizing to the sequence of nucleotide 11 to 30 of at least 100, preferably at least 150 more preferably at least 200 nucleic acid sequences selected from the group consisting of SEQ ID NO:1 to SEQ ID NO:910, and a RNA sequence encoded by a sequence of SEQ ID NO:1 to 910; or specifically hybridize to the reverse complement sequences thereof, preferably the plurality of probes comprises probes specifically hybridizing to the sequence of nucleotide 11 to 30 of SEQ ID NO:1 to SEQ ID NO:200, or a RNA sequence encoded by a sequence of SEQ ID NO:1 to 200, or the reverse complement sequences thereof.

Te invention also relates to the use of the kit of invention for the diagnosis of AD.

Furthermore, desribed is the use of a nucleic acid probe specifically hybridizing to the sequence of nucleotide 11 to 30 of a sequence selected from the group consisting of SEQ ID NO:1 to SEQ ID NO:910; and a RNA sequence encoded by a sequence of SEQ ID NO:1 to 910, or hybridizing to the reverse complement thereof, a kit according to the disclosure, or an array according to the disclosure for the diagnosis of a neurodegenerative disease, preferably for the diagnosis of Alzheimer's disease.

### Figure Legends

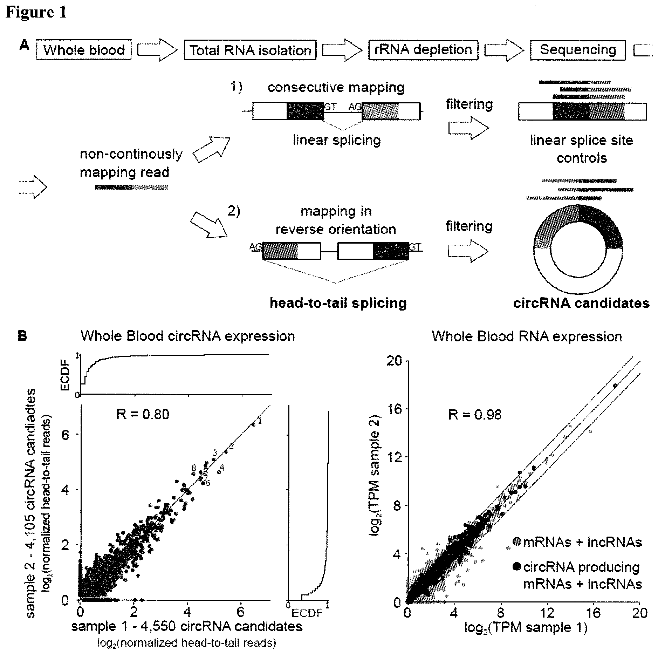

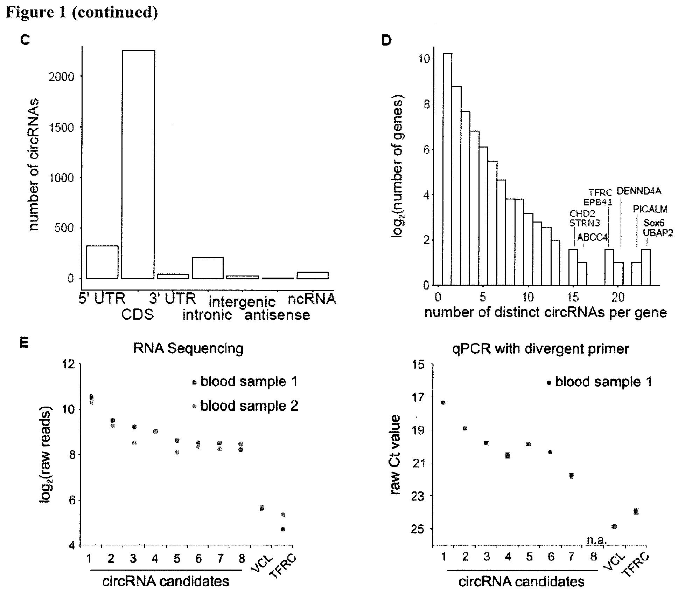

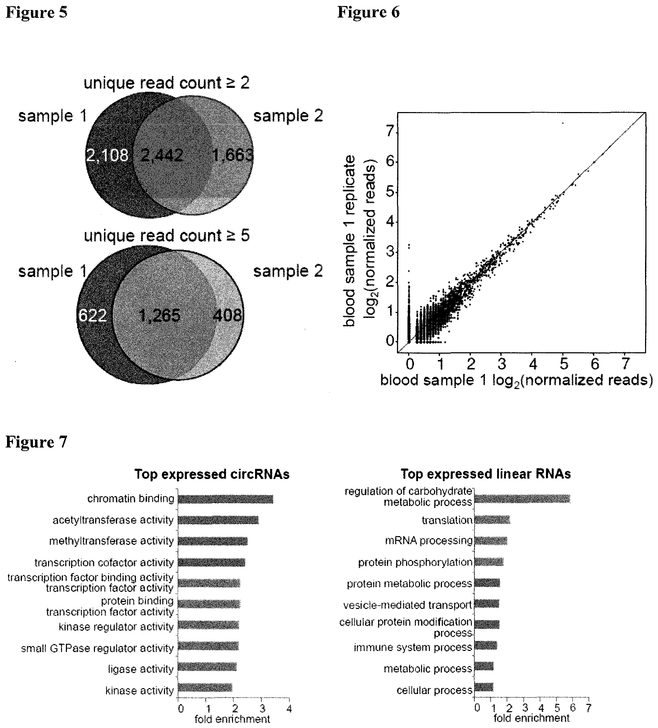

**Figure 1****: Thousands of circRNAs are reproducibly detected in human blood. (A)** Total RNA was extracted from human whole blood samples and rRNA was depleted. cDNA libraries were synthesized using random primers and subjected to sequencing. Raw sequencing reads were used for circRNA detection as previously described *(see* Memczak S, Jens M, Elefsinioti A, et al. Circular RNAs are a large class of animal RNAs with regulatory potency. Nature. 2013; 495(7441):333-338). Sequencing reads that map continuously to the human reference genome were disregarded. From unmapped reads anchors were extracted and independently mapped. Anchors that align consecutively indicate linear splicing events 1) whereas alignment in reverse orientation indicates head-to-tail splicing as observed for circular RNAs 2). After filtering of linear splicing events and circRNA candidates (see Methods in the Example section) the genomic coordinates and additional information such as read count, alignment quality and annotation are documented (Table 3). **(B)** circRNA candidate expression in human whole blood samples from two donors, ECDF=empirical cumulative distribution function. circRNA candidates tested in this study are annotated as numbers. Right panel: mRNA and long, non-coding RNA (lncRNA) (n=17,282) expression per gene in two blood samples in transcripts per million (TPM), RNAs with putative circular isoforms (n= 2,523) are highlighted in blue; R-values: Spearman correlation for RNAs found in both samples. **(C)** ENSEMBLE genome annotation for reproducibly detected circRNA candidates *(see* also Figure 5). Number of circRNAs with at least one splice site in each category is given. **(D)** Number of distinct circRNA candidates per gene. y-axis = log₂(circRNA frequency+1). Gene names with the highest numbers are highlighted. **(E)** Expression level of top 8 circRNA candidates measured with sequencing (left panel) and divergent primers in qPCR (right); Ct = cycle threshold linear control genes VCL and TFRC were measured with convergent primer.

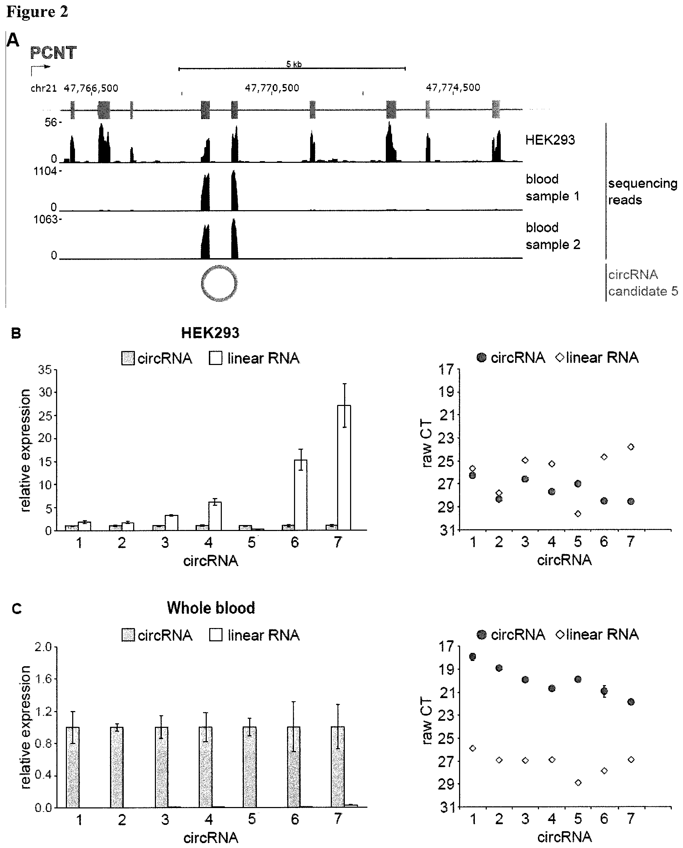

**Figure 2****: Top expressed blood circRNAs dominate over linear RNA isoforms. (A)** Example for the read coverage of a top expressed blood circRNA produced from the PCNT gene locus (http://genome.ucsc.edu; *see* Kent WJ, Sugnet CW, Furey TS, et al. The human genome browser at UCSC. Genome Research. 2002;12(6):996-1006). Data are shown for the human HEK293 cell line *(see* Ivanov A, Memczak S, Wyler E, et al. Analysis of Intron Sequences Reveals Hallmarks of Circular RNA Biogenesis in Animals. CellReports. 2015;10(2):170-177) and two biologically independent blood RNA preparations. **(B)** Relative expression and raw Ct values of top expressed blood circRNAs and corresponding linear isoforms in HEK293 cells and whole blood **(C).**

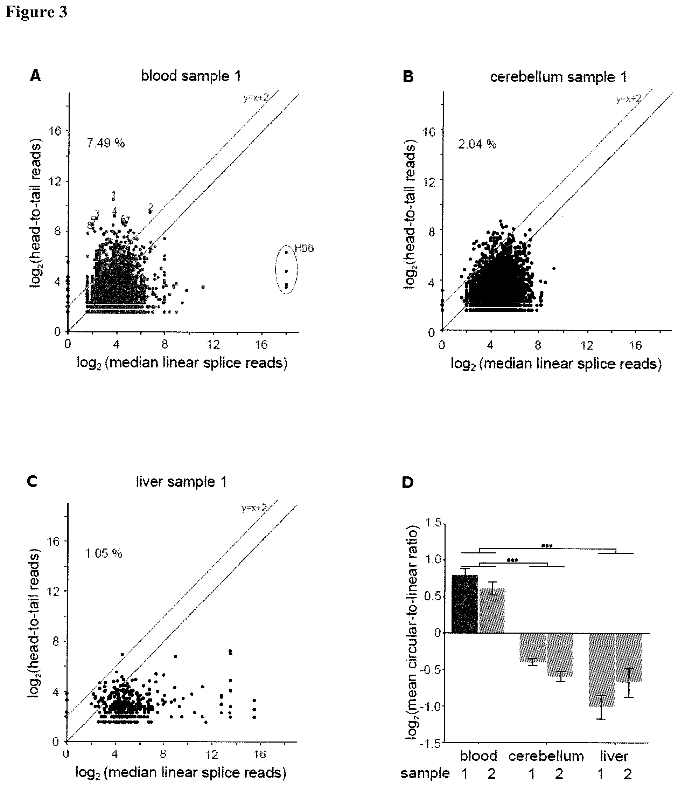

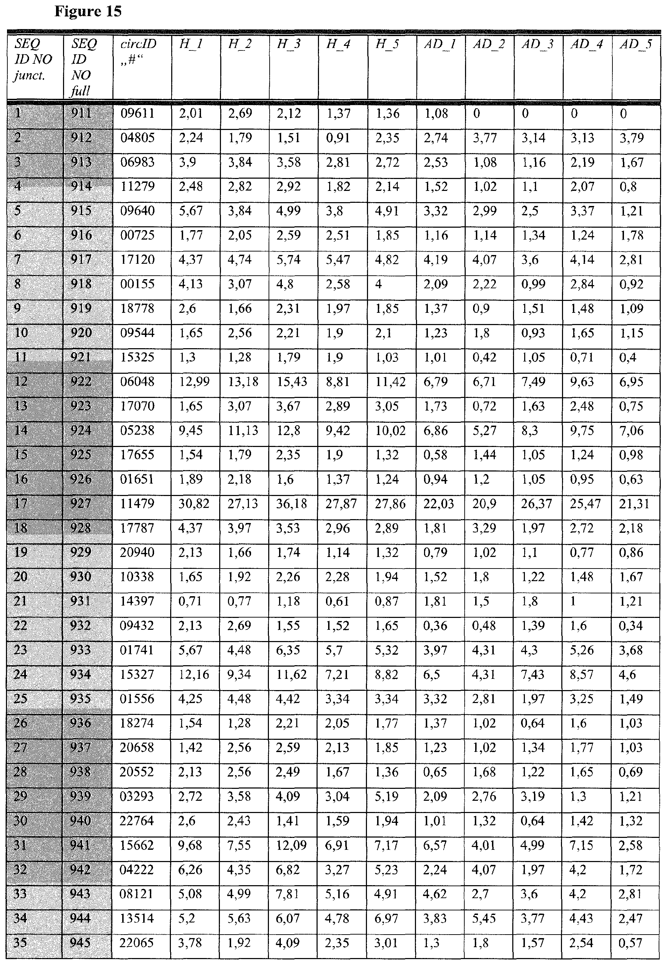

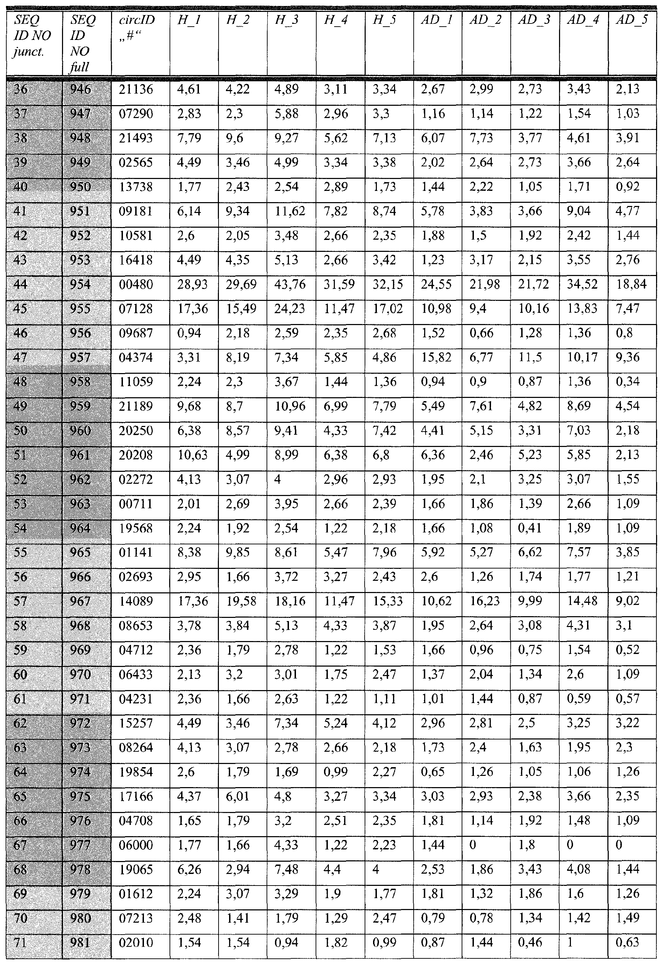

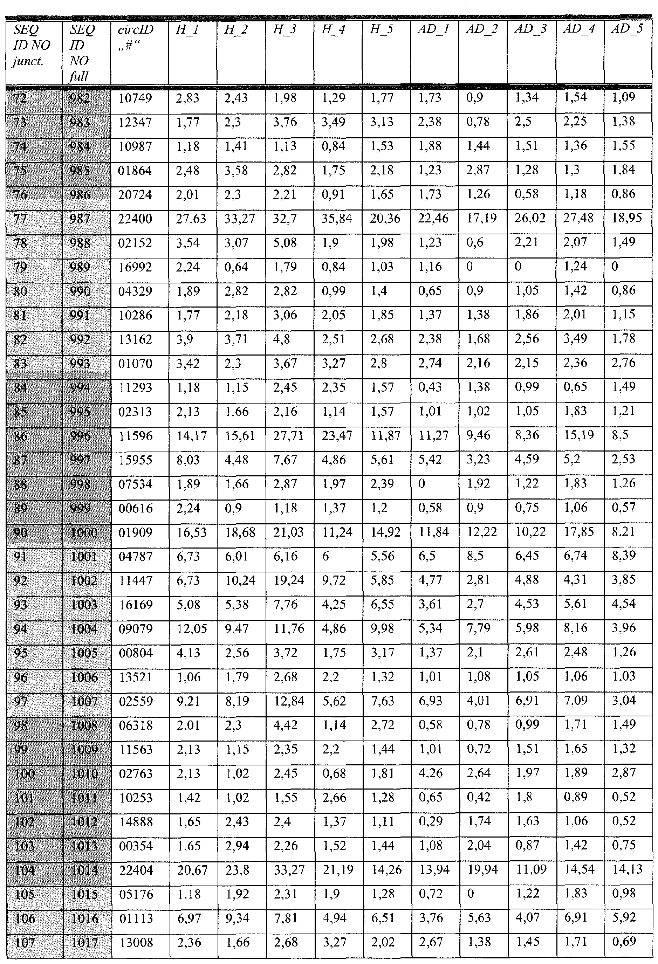

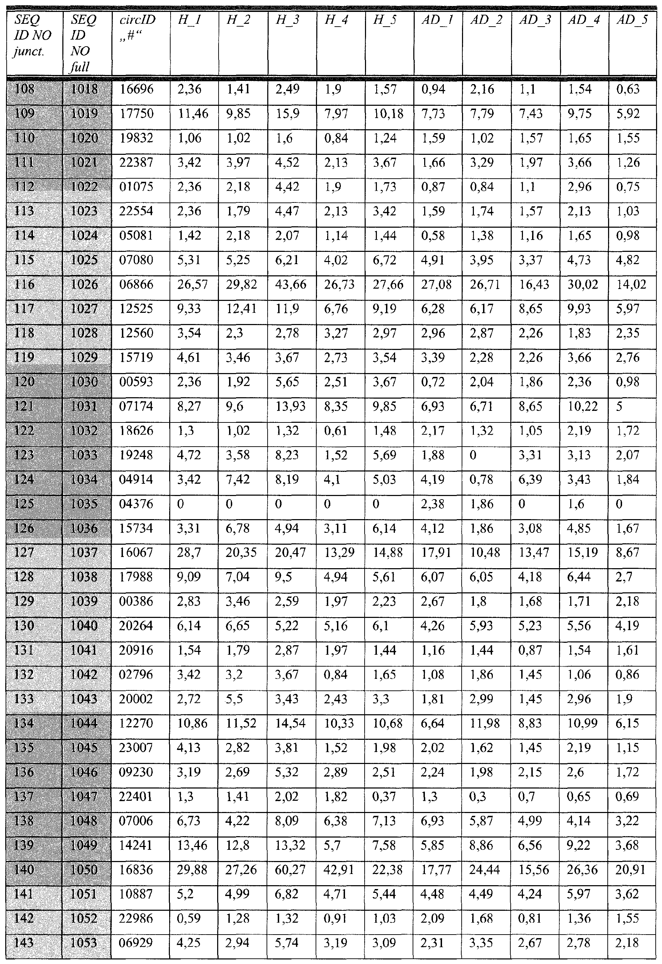

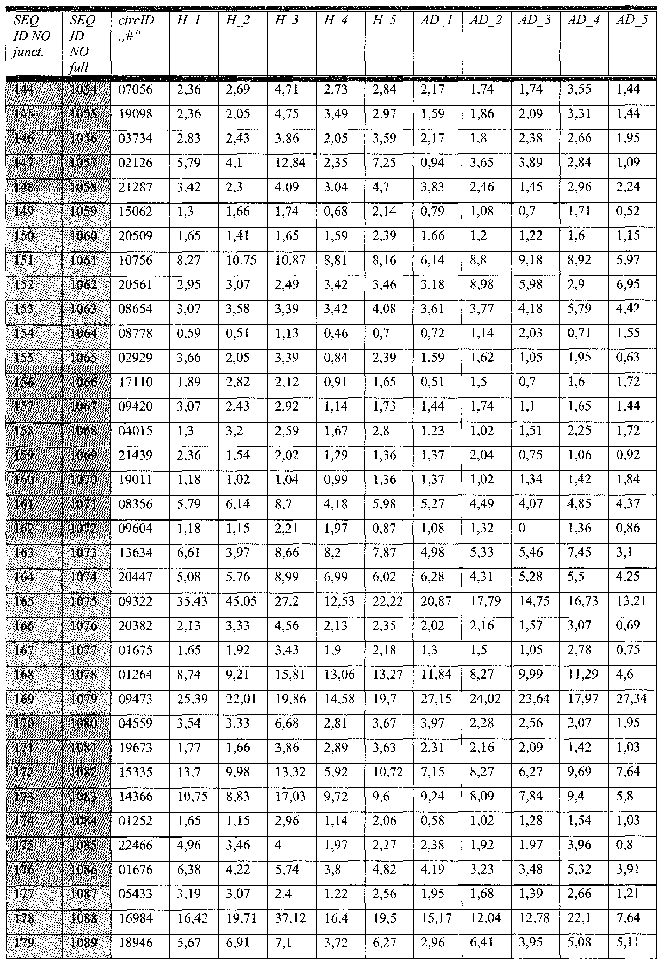

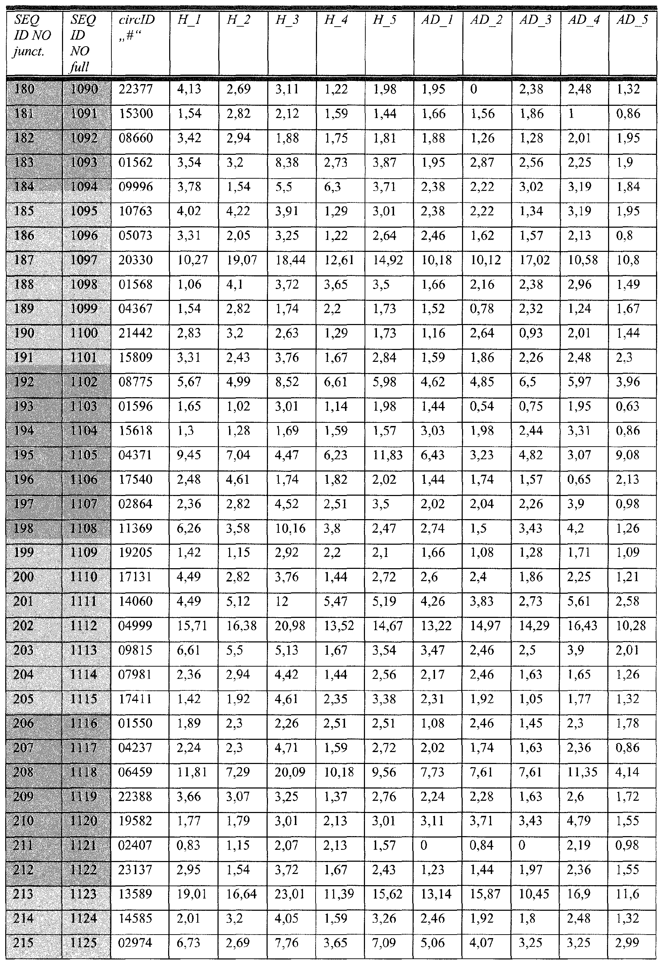

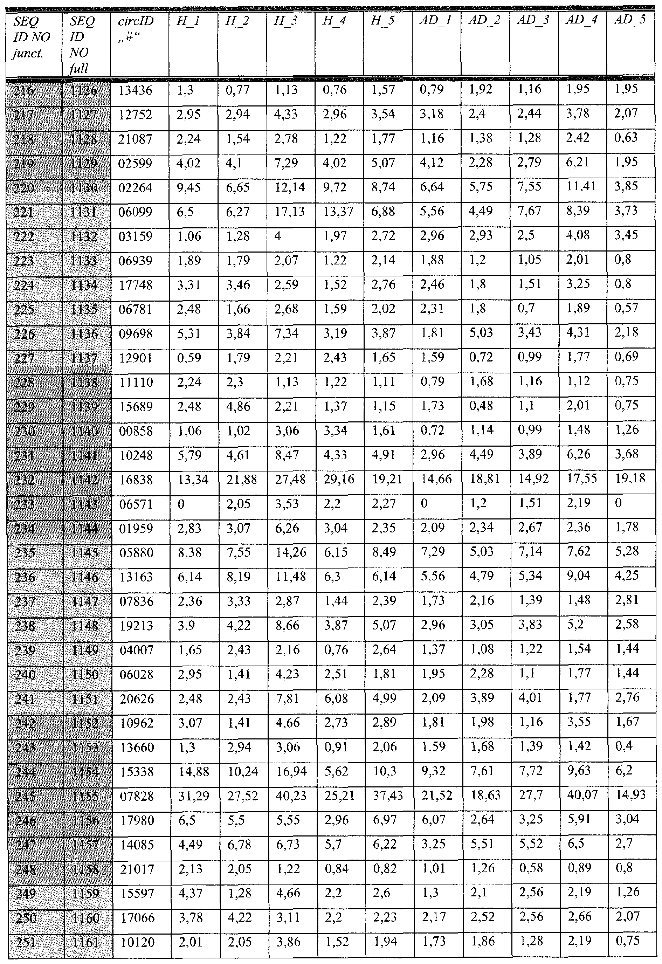

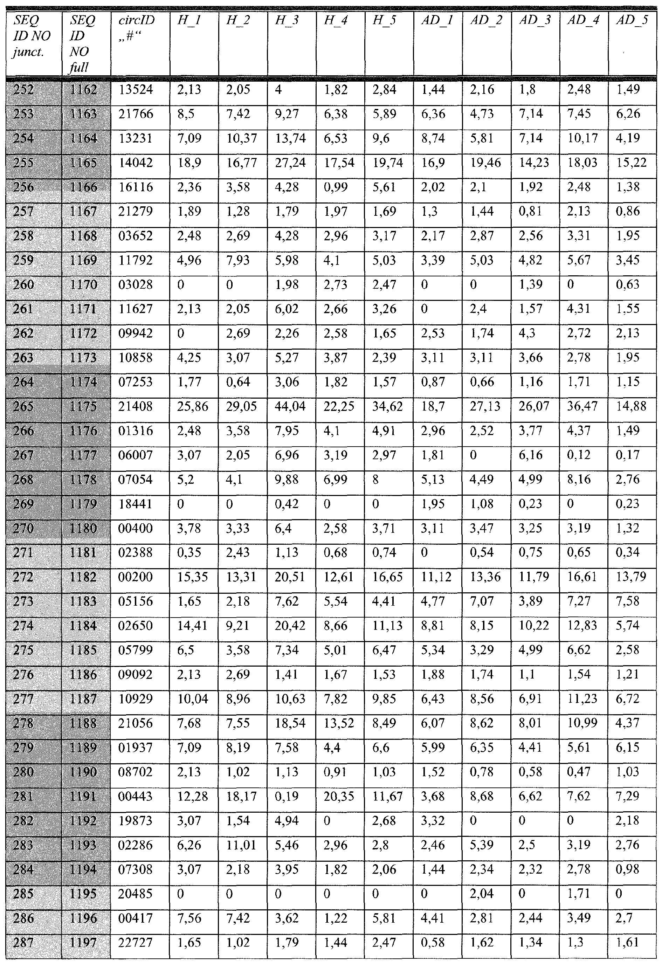

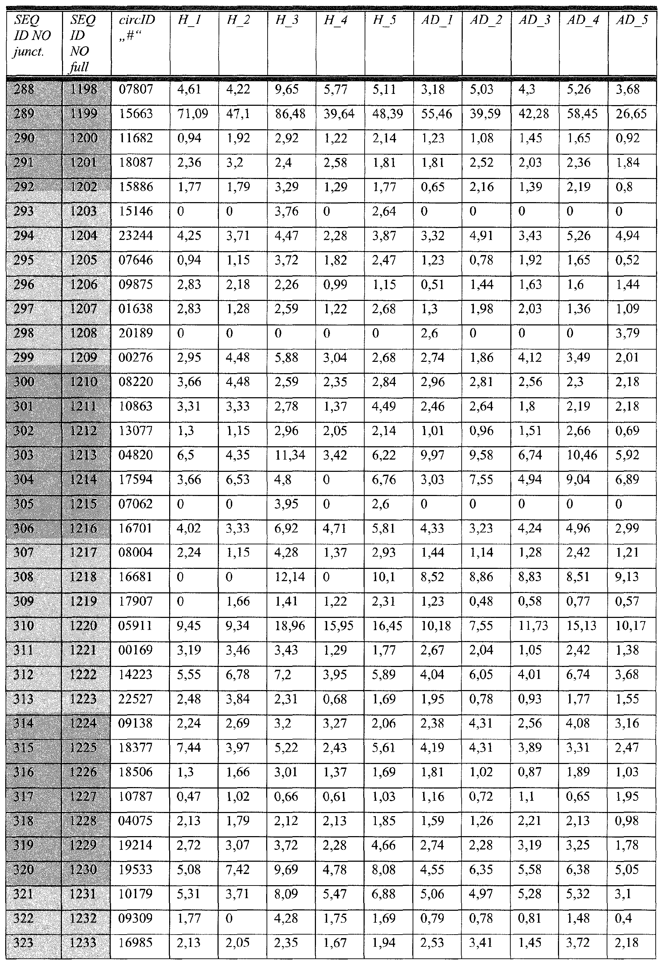

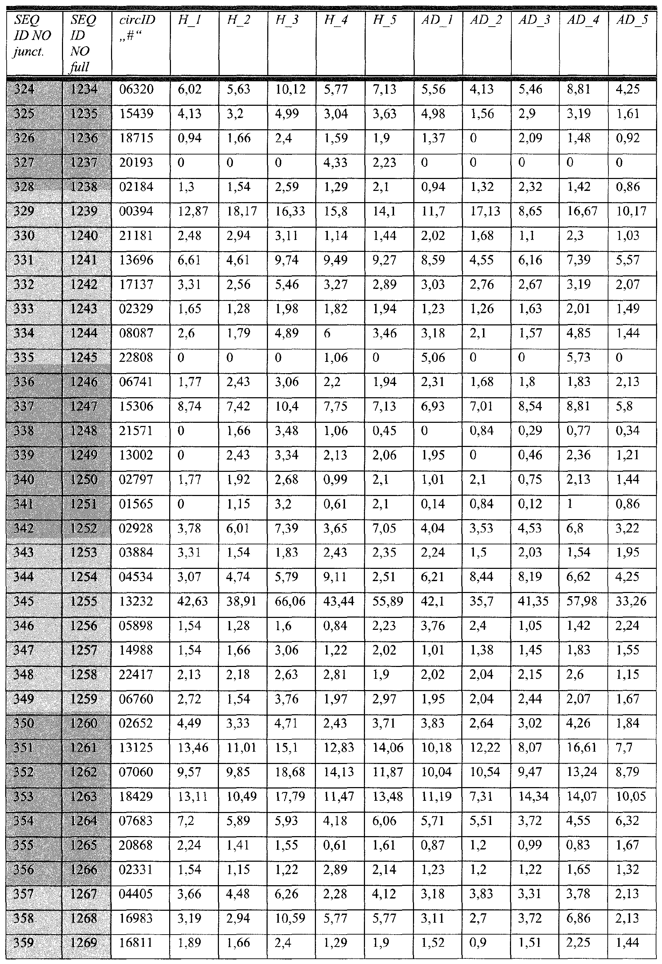

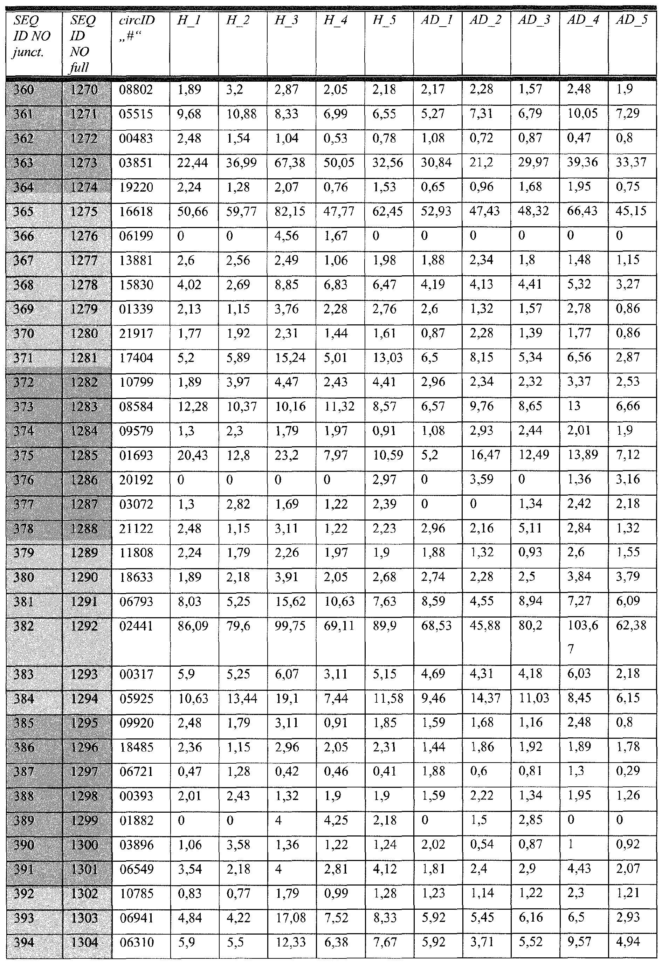

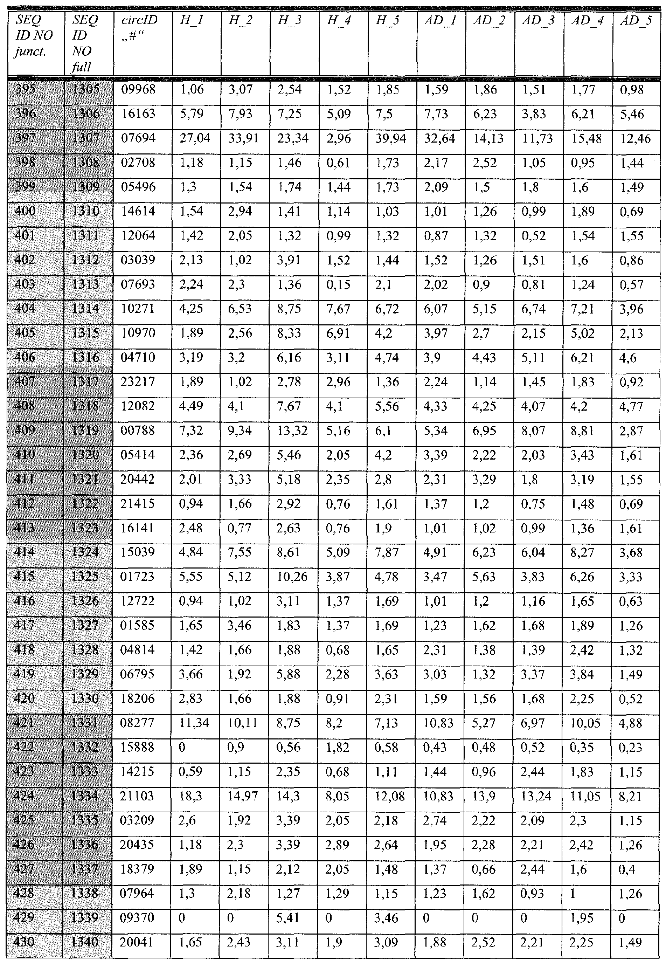

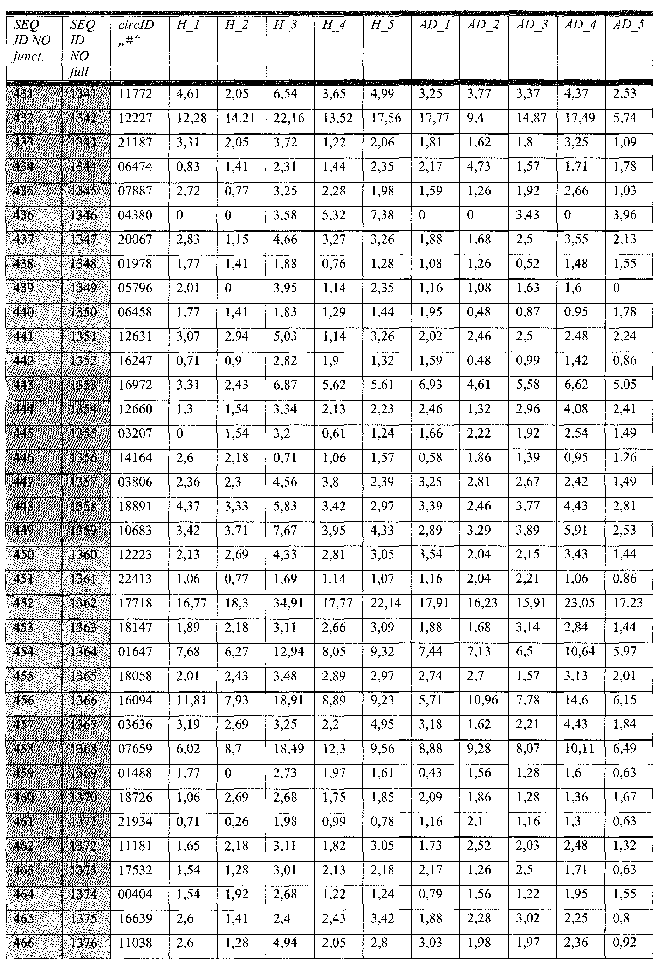

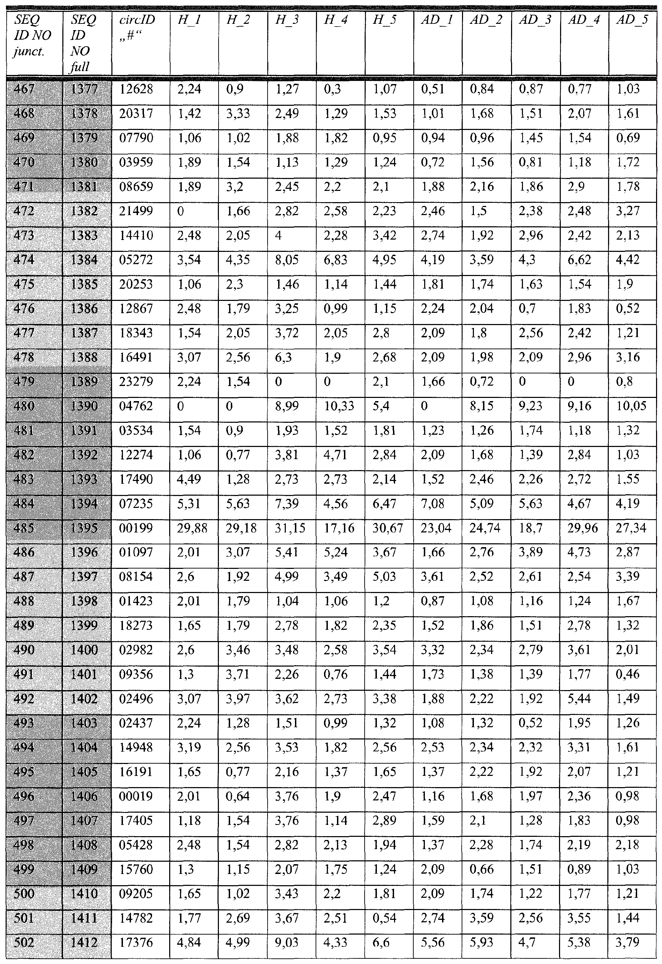

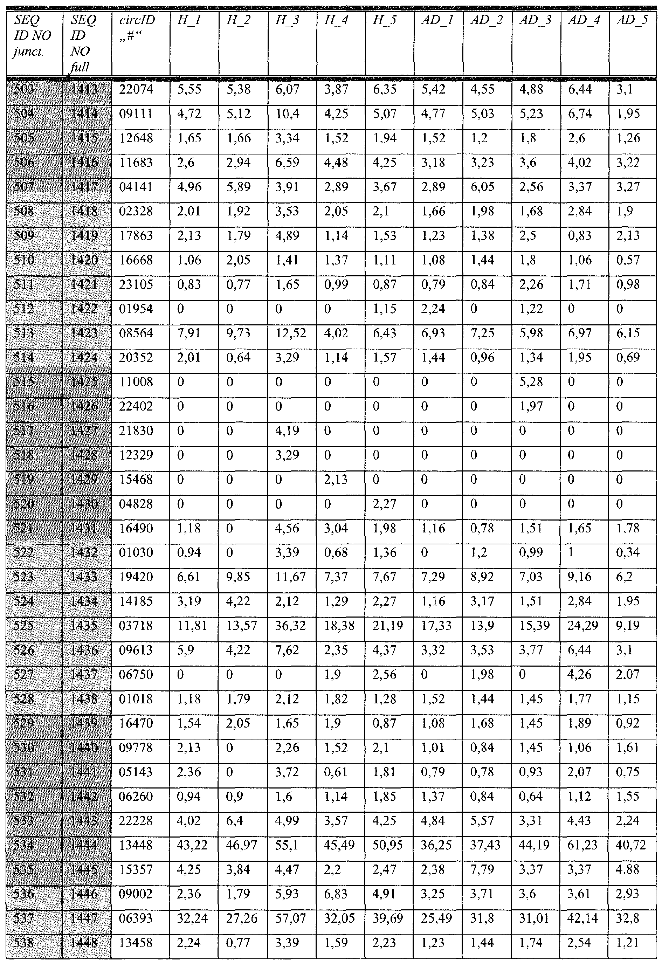

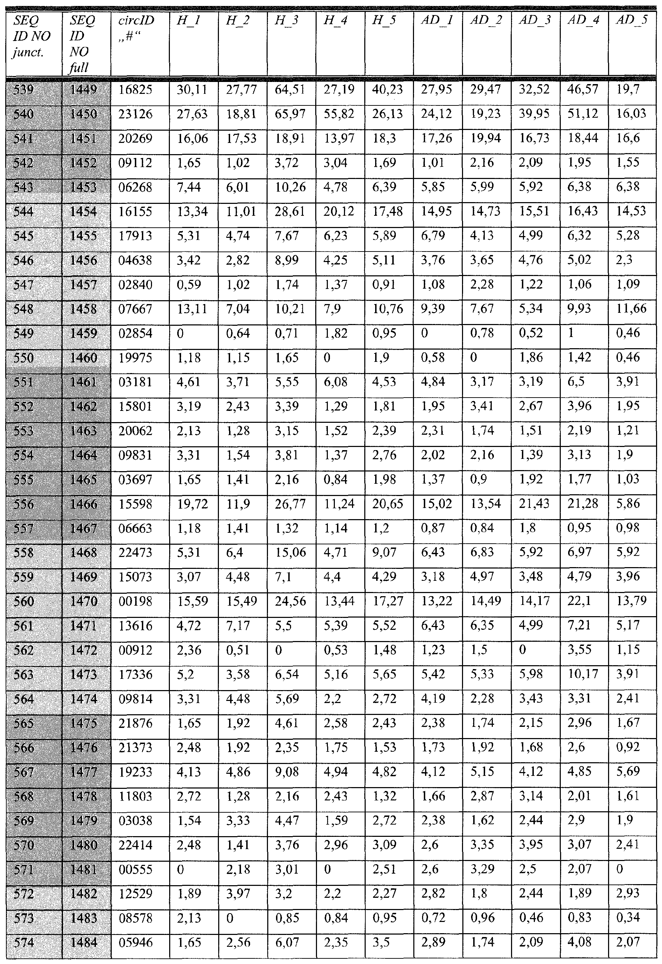

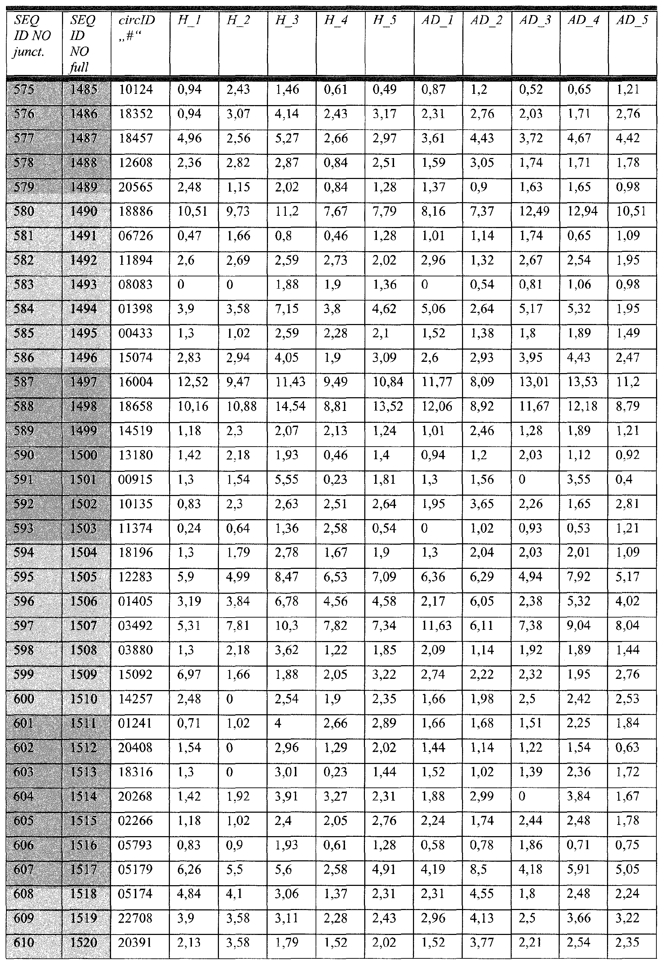

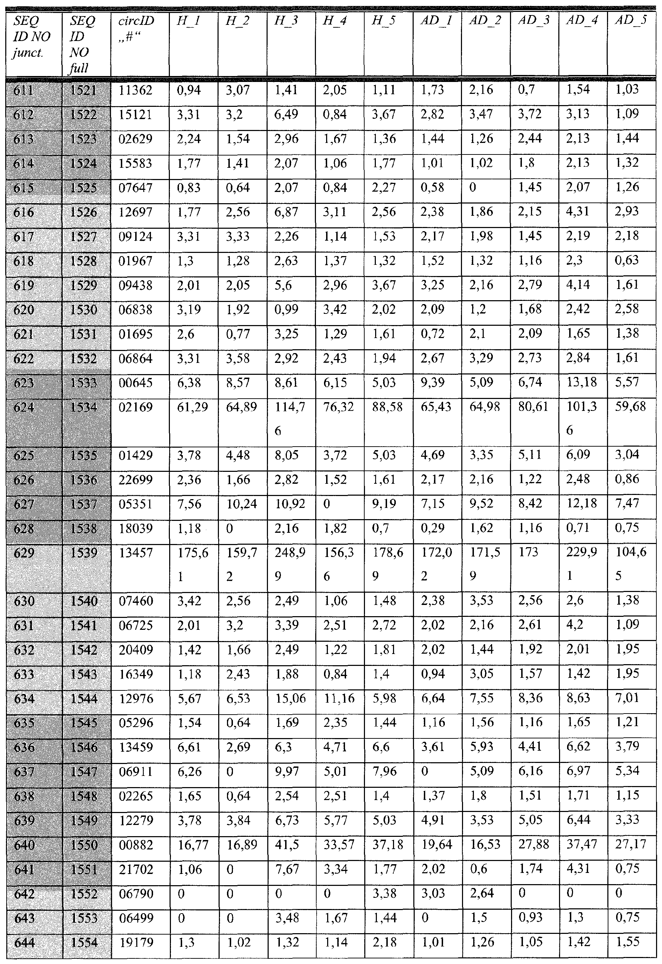

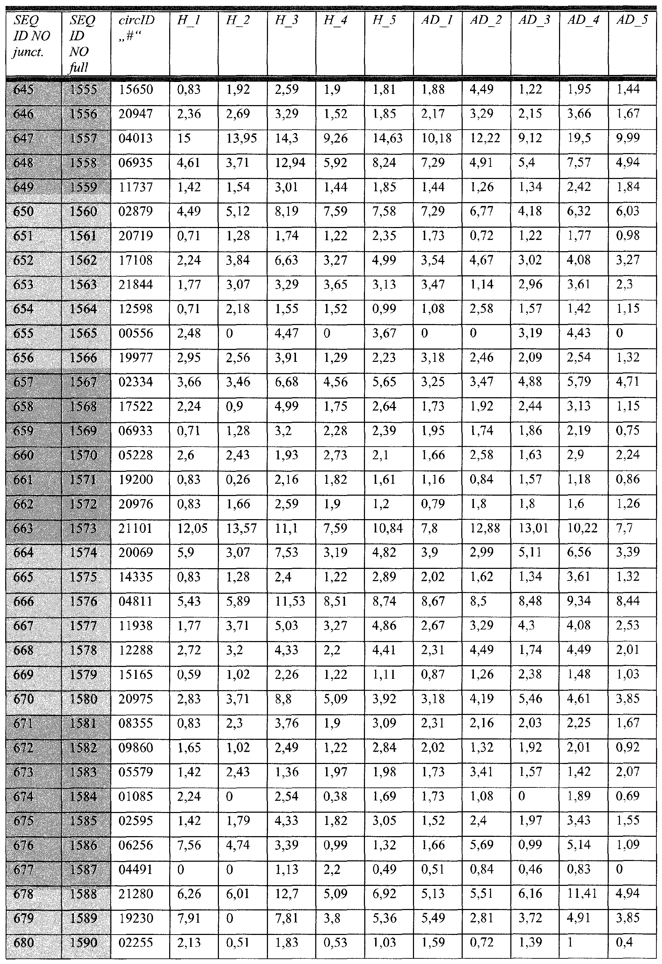

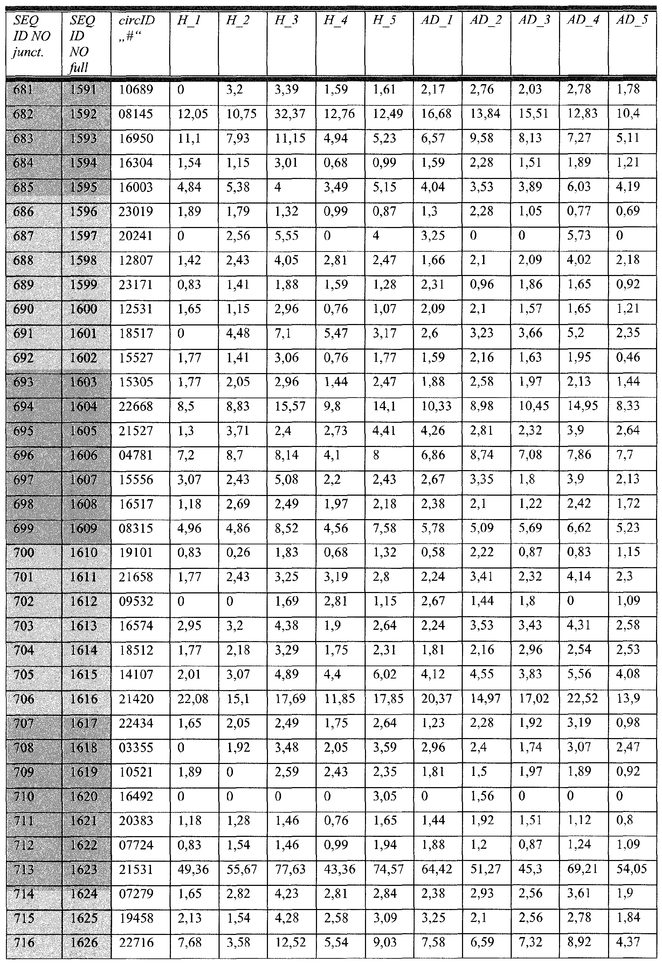

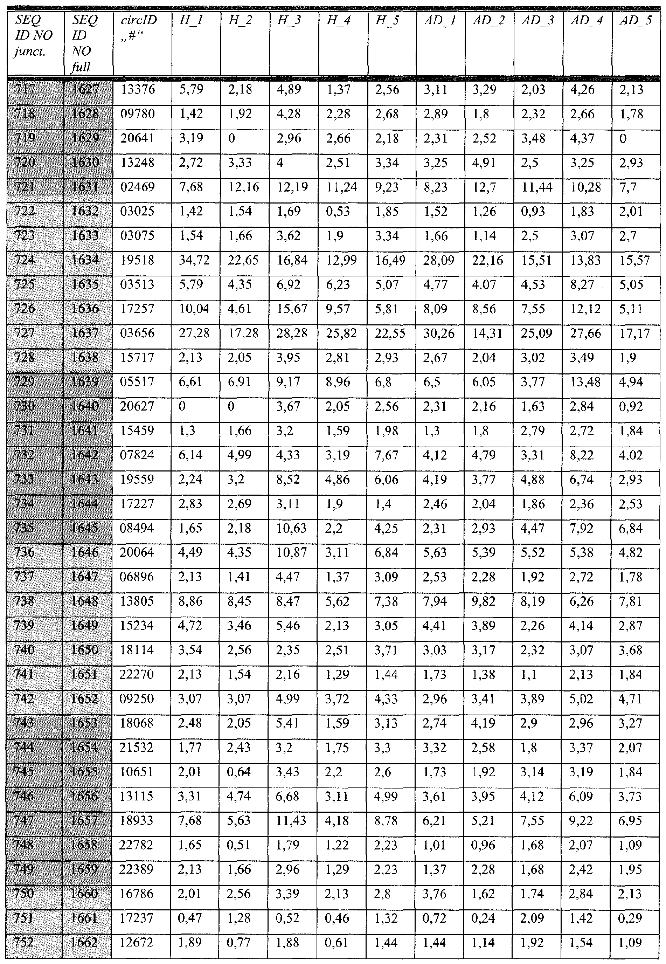

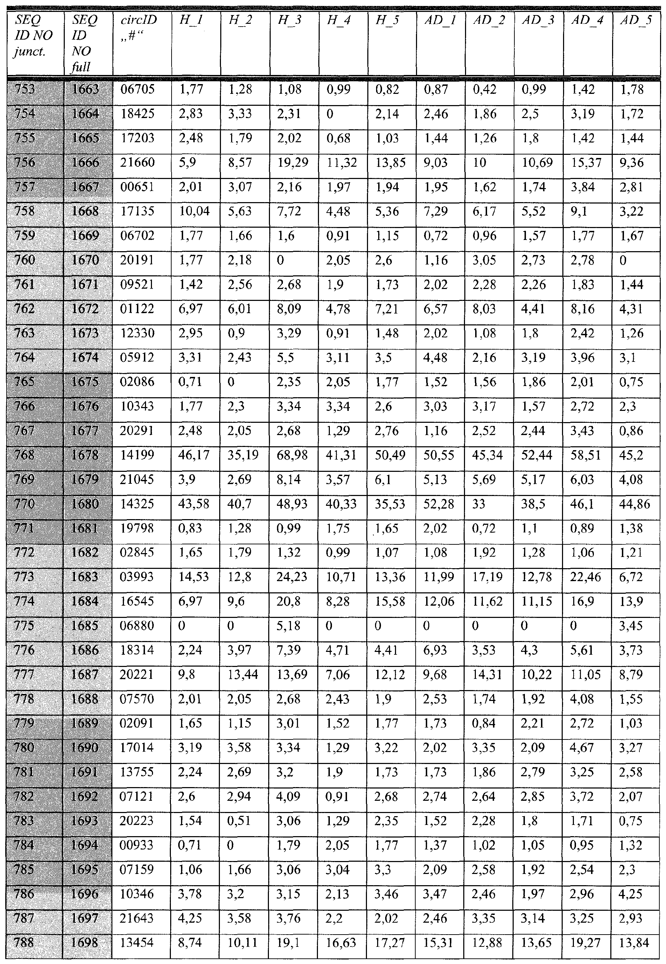

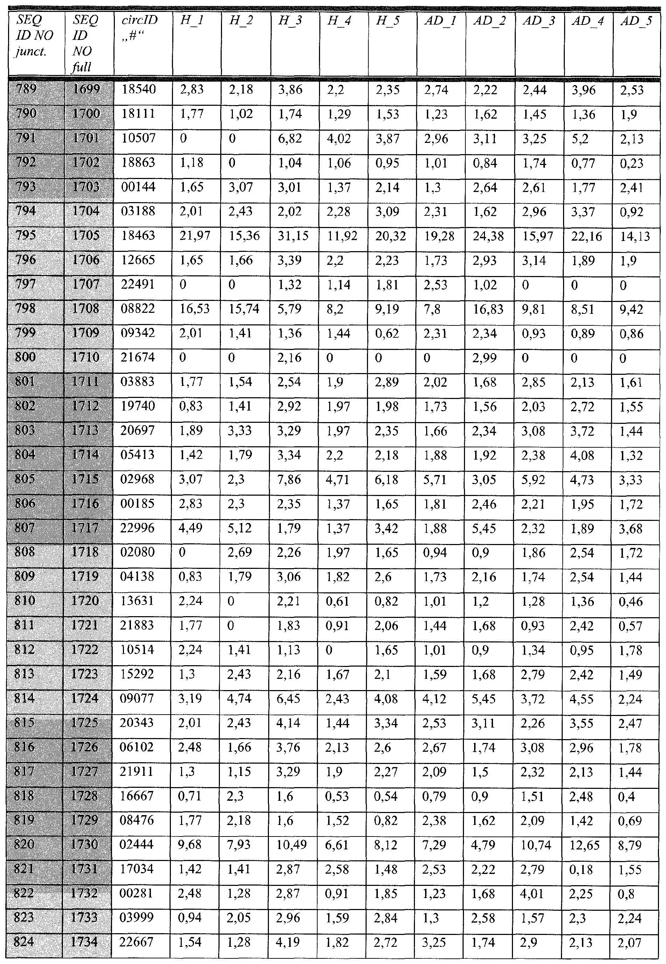

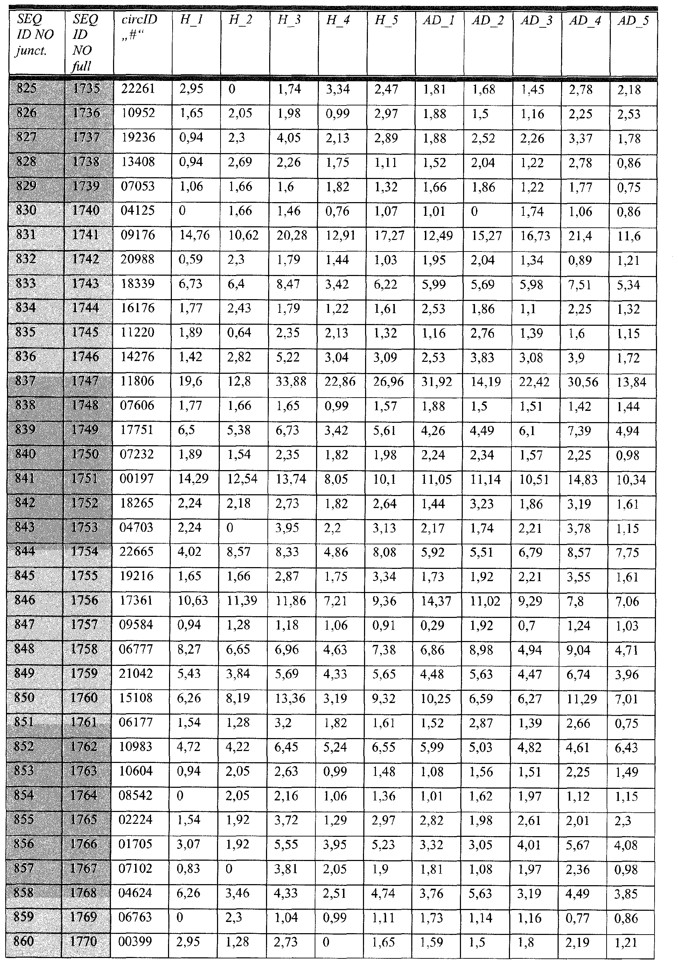

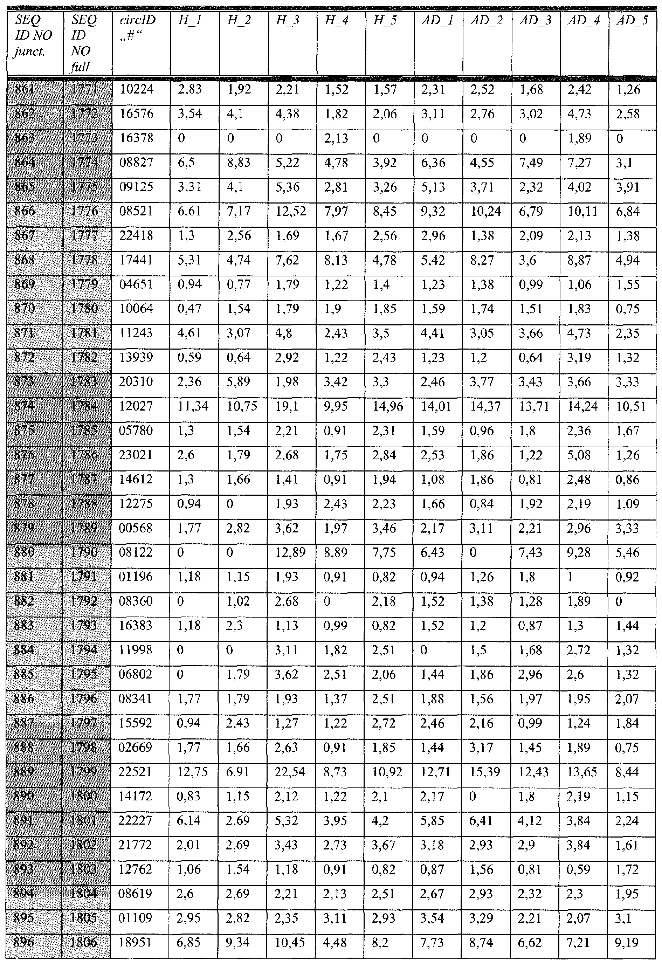

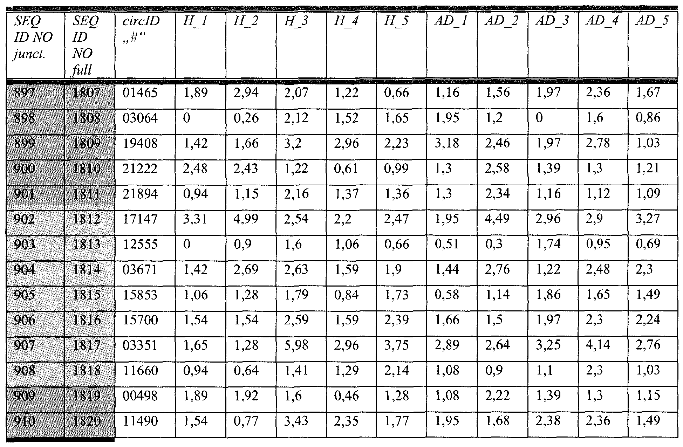

**Figure 3****: Circular to linear RNA isoform expression is high in blood compared to other tissues. (A)** Comparison of circular to linear RNA isoforms in blood. circRNAs measured by head-to-tail spanning reads. As a proxy for linear RNA expression median linear splice site spanning reads were counted. Data are shown for one replicate each of blood cerebellum **(B)** and liver **(C).** Relative fraction of circRNA candidates with >4x higher expression than linear isoforms are given as inset, eight tested candidates are indicated by numbers, circRNAs derived from hemoglobin are marked in (A). **(D)** mean circular-to-linear RNA expression ratio for the same samples, in two biological independent replicates. Error bars indicate the standard error of the mean, *** denotes P <0.001 permutation test on pooled replicate data (*see* Method section in the Examples). (A-D) represent expression datasets for one replicate per sample (Figure 15).

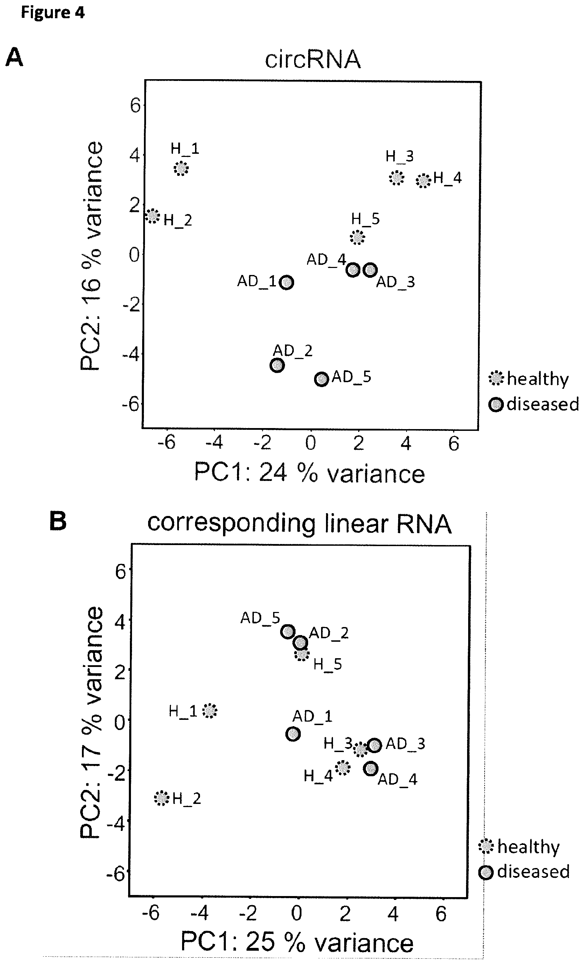

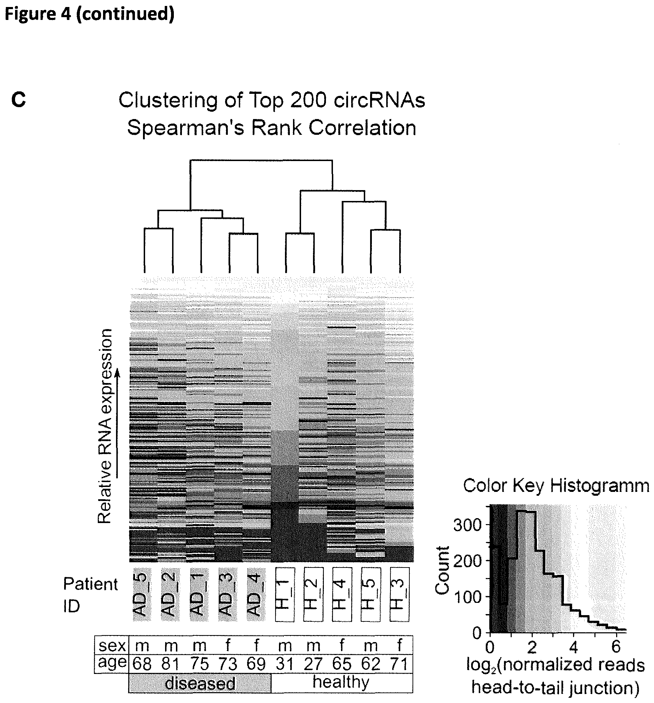

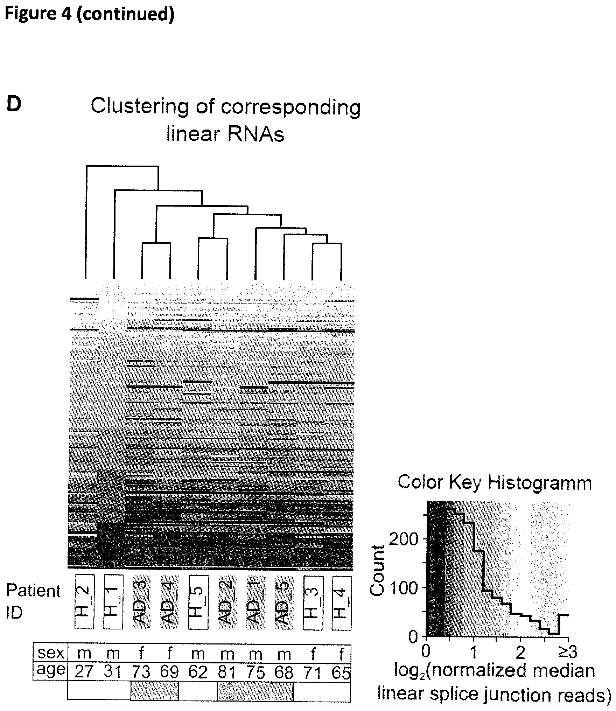

**Figure 4****: Comparative analysis of blood circRNA expression in Alzheimer's disease patients and controls. (A)** Principle Component Analysis (PCA) of circRNA expression for 5 control **(H)** and 5 Alzheimer's disease (AD) patients. A circRNA subset comprising the top 910 (out of 20,969) detected circRNAs was analyzed *(see* Results section in the Examples). **(B)** analysis as in (A) for the corresponding linear RNA isoforms measured by median read count of linear splice junctions. **(C)** Expression of 200 circRNA candidates with highest weight in PC2 (see A) were used for unsupervised clustering (Spearman's rank correlation as distance metric, *see* Method section in the Examples). PC2 represents the diseased/healthy principle component. Histograms show expression distribution. Patient details are given underneath each patient ID. **(D)** analysis as in (C) but for linear RNAs of the corresponding genes (n=167).
**Figure 5****: Reproducibility of circRNA candidate detection.** The overlap of 2,442 circRNAs found with at least 2 read counts in both samples is considered as reproducibly detected circRNA set.
**Figure 6****: Technical reproducibility of circRNA candidate detection.** A library of blood sample 1 (H_1) was sequenced twice (*see* Table 2).
**Figure 7****: GO annotation of circRNAs and linear RNAs in blood.** Significantly enriched GO terms (p<0.05) for circRNAs found in both samples (n=2,442) and for the same number of top expressed linear RNAs.
**Figure 8****: Predicted circRNA length.** Predicted spliced circRNA length distributions for circRNA candidates detected in liver, cerebellum and blood.

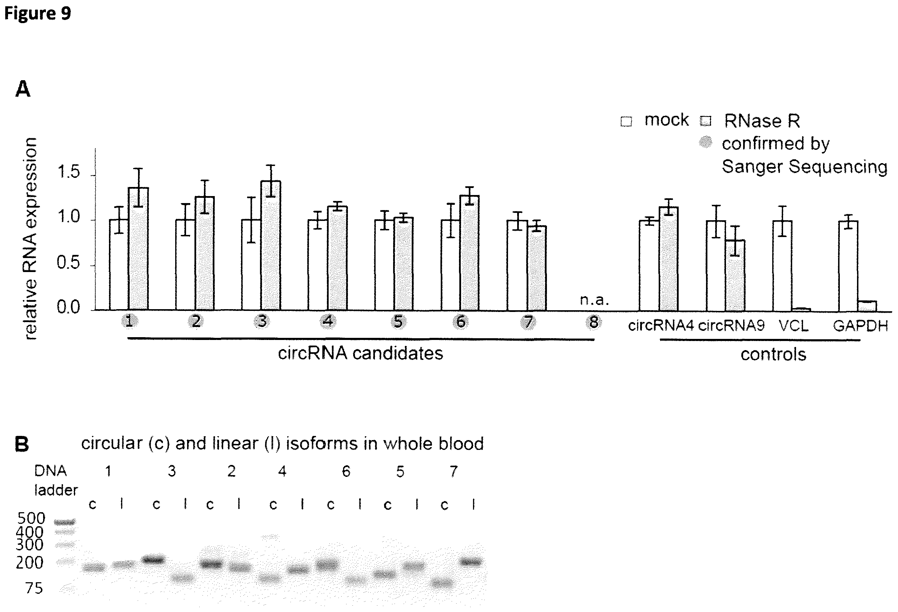

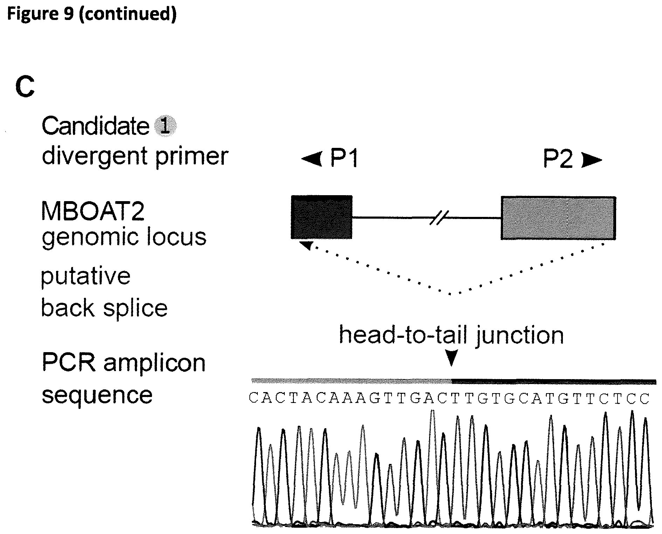

**Figure 9****: circRNA candidate validation. (A)** Top circRNA candidate expression was measured in qPCR using divergent primer on mock or RNase R treated total RNA preparation. 7/8 were successfully amplified while candidate 7 did not yield specific PCR products and is therefore excluded from further analysis. Linear RNAs and previously described circRNAs are shown as controls. **(B)** PCR amplicons for divergent and convergent primer sets (c - circular, l - linear) of the tested candidates, end point analysis after 40 cycles. **(C)** PCR amplicons were subjected to Sanger sequencing and checked for the presence of a head-to-tail junction, representative example result is shown.
**Figure 10****: Comparison of circRNA candidates in blood to liver and cerebellum. (A)** Comparison of circular RNA candidates detected in blood (sample 1) and cerebellum shown for the whole expression range. **(B)** fraction of circRNA candidates that overlap between the two samples binned by blood expression level. **(C, D)** analysis as before but for liver circRNA candidates.
**Figure 11****: Correlation of linear RNAs in cerebellum and blood and liver and blood.** Number of detected transcripts: blood=29,908; cerebellum=38,192; liver=27,880; TPM=transcripts per million.
**Figure 12****: Comparison circ-to-linear expression by RNA-Seq and qPCR.** Raw Ct values (Cycle threshold) and median linear splice junction spanning read counts are given for the respective RNA isoform.
**Figure 13****: Histogram of principle components.** Principle components (PC) were calculated from the analysis shown in Figure 4 (A, B).
**Figure 14****: Number of exons per circRNA in blood.** Histogram of number of exons per circRNA. Reproducible detected set (2,442) without intergenic circRNAs (n=27); median exon number: 2, mean exon number: 2.8.
**Figure 15****: List of circRNAs detected in human blood.** Genomic location, ENSEMBL gene identifier and symbols and gene biotype are given together in Table 1, *infra.* Here, raw read counts for each circRNA candidate in each sample of healthy subjects (H_1 to H_5) and subjects suffering from Alzheimer's disease (AD_1 to AD_5) are given.

### Detailed Description of the Invention

As outlined herein and exemplified in the Examples, the inventors for the first time provide evidence that circular RNAs (circRNA) is present in whole blood in great amounts and suited as biomarker for diseases in a subject. Hence, described is a method for diagnosing a disease of a subject, comprising the step of determining the presence or absence of one or more circular RNA (circRNA) in a sample of a bodily fluid of said subject; wherein the presence or absence of said one or more circRNA is indicative for the disease.

It was unexpectedly possible to show a correlation of differing levels of RNAs and a disease in a tissue other than the sample tissue, i.e. other than the bodily fluid tested. Hence, in a preferred embodiment said disease is not a disease of said bodily fluid. The gist of the present disclosure is that circRNAs in bodily fluids like blood are unexpectedly suited as biomarkers.

The term "biomarker" (biological marker) was introduced in 1989 as a Medical Subject Heading (MeSH) term: "measurable and quantifiable biological parameters (e.g., specific enzyme concentration, specific hormone concentration, specific gene phenotype distribution in a population, presence of biological substances) which serve as indices for health- and physiology-related assessments, such as disease risk, psychiatric disorders, environmental exposure and its effects, disease diagnosis, metabolic processes, substance abuse, pregnancy, cell line development, epidemiologic studies, etc." In 2001, an NIH working group standardized the definition of a biomarker as "a characteristic that is objectively measured and evaluated as an indicator of normal biological processes, pathogenic processes, or pharmacologic responses to a therapeutic intervention" and defined types of biomarkers. A biomarker may be measured on a biosample (as a blood, urine, or tissue test), it may be a recording obtained from a person (blood pressure, ECG, or Holter), or it may be an imaging test (echocardiogram or CT scan). Biomarkers can indicate a variety of health or disease characteristics, including the level or type of exposure to an environmental factor, genetic susceptibility, genetic responses to exposures, markers of subclinical or clinical disease, or indicators of response to therapy. Thus, a simplistic way to think of biomarkers is as indicators of disease trait (risk factor or risk marker), disease state (preclinical or clinical), or disease rate (progression). Accordingly, biomarkers can be classified as antecedent biomarkers (identifying the risk of developing an illness), screening biomarkers (screening for subclinical disease), diagnostic biomarkers (recognizing overt disease), staging biomarkers (categorizing disease severity), or prognostic biomarkers (predicting future disease course, including recurrence and response to therapy, and monitoring efficacy of therapy). The biomarkers of the present disclosure are preferably antecedent or screening biomarkers. Hence, the methods are methods for diagnosing the presence or the risk for acquiring a disease.

Biomarkers may also serve as surrogate end points. Although there is limited consensus on this issue, a surrogate end point is one that can be used as an outcome in clinical trials to evaluate safety and effectiveness of therapies in lieu of measurement of the true outcome of interest. The underlying principle is that alterations in the surrogate end point track closely with changes in the outcome of interest. Surrogate end points have the advantage that they may be gathered in a shorter time frame and with less expense than end points such as morbidity and mortality, which require large clinical trials for evaluation. Additional values of surrogate end points include the fact that they are closer to the exposure/intervention of interest and may be easier to relate causally than more distant clinical events. An important disadvantage of surrogate end points is that if the clinical outcome of interest is influenced by numerous factors (in addition to the surrogate end point), residual confounding may reduce the validity of the surrogate end point. It has been suggested that the validity of a surrogate end point is greater if it can explain at least 50% of the effect of an exposure or intervention on the outcome of interest.

A "sample" in the meaning of the disclosure can be all biological fluids of the subject, such as lymph, saliva, urine, cerebrospinal fluid or blood. The sample is collected from the patient or subjected to the diagnosis according to the disclosure. The sample of the bodily fluid is in a preferred embodiment selected from the group consisting of blood, cerebrospinal fluid, saliva, serum, plasma, and semen, the most preferred embodiment of the sample is a whole blood sample. A "sample" in the meaning of the disclosure may also be a sample originating from a biochemical or chemical reaction such as the product of an amplification reaction. Liquid samples may be subjected to one or more pre-treatments prior to use in the present disclosure.

Such pre-treatments include, but are not limited to dilution, filtration, centrifugation, concentration, sedimentation, precipitation or dialysis. Pre-treatments may also include the addition of chemical or biochemical substances to the solution, e.g. in order to stabilize the sample and the contained nucleic acids, in particular the circRNAs. Such addition of chemical or biochemical substances include acids, bases, buffers, salts, solvents, reactive dyes, detergents, emulsifiers, or chelators, like EDTA. The sample may for instance be taken and directly mixed with such substances. In a particularly preferred embodiment of the invention the sample is a whole blood sample. The whole blood sample is preferably not pre-treated by means of dilution, filtration, centrifugation, concentration, sedimentation, precipitation or dialysis. It is, however, preferred that substances are added to the sample in order to stabilize the sample until onset of analysis. "Stabilizing" in this context means prevention of degradation of the circRNAs to be determined. Preferred stabilizers in this context are EDTA, e.g. K₂EDTA, RNase inhibitors, alcohols e.g. ethanol and isopropanol, agents used to salt out proteins (such as RNAlater).

"Whole blood" is a venous, arterial or capillary blood sample in which the concentrations and properties of cellular and extra-cellular constituents remain relatively unaltered when compared with their *in vivo* state. In some embodiments, anticoagulation *in vitro* stabilizes the constituents in a whole blood sample.

In a preferred embodiment the sample comprises a nucleic acid or nucleic acids. The term "nucleic acid" is here used in its broadest sense and comprises ribonucleic acids (RNA) and deoxyribonucleic acids (DNA) from all possible sources, in all lengths and configurations, such as double stranded, single stranded, circular, linear or branched. All sub-units and subtypes are also comprised, such as monomeric nucleotides, oligomers, plasmids, viral and bacterial nucleic acids, as well as genomic and non-genomic DNA and RNA from the subject, circular RNA (circRNA), messenger RNA (mRNA) in processed and unprocessed form, transfer RNA (tRNA), heterogeneous nuclear RNA (hn-RNA), ribosomal RNA (rRNA), complementary DNA (cDNA) as well as all other conceivable nucleic acids. However, in the most preferred embodiment the sample comprises circRNAs.

"Presence" or "absence" of a circRNA in connection with the present invention means that the circRNA is present at levels above a certain threshold or below a certain threshold, respectively. In case the threshold is "0" this would mean that "presence" is the actual presence of circRNA in the sample and "absence" is the actual absence. However, "presence" in context with the present invention may also mean that the respective circRNA is present at a level above a threshold, e.g. the levels determined in a control. "absence" in this context then means that the level of the circRNA is at or below the certain threshold. Hence, it is preferred that the method of the present invention comprises determining of the level of one or more circRNA and comparing it to a control level of said one or more circRNA. In a preferred embodiment of the invention the determination step comprises: (i) determining the level of said one or more circRNA; and (ii) comparing the determined level to a control level of said one or more circRNA; wherein differing levels between the determined and the control level are indicative for the disease. In other words, the invention relates to a method for diagnosing a disease of a subject, comprising the step of (i) determining the level of said one or more circRNA; and (ii) comparing the determined level to a control level of said one or more circRNA; wherein differing levels between the determined and the control level are indicative for the disease.

The term "control level" relates to a level to which the determined level is compared in order to allow the distinction between "presence" or "absence" of the circRNA. The control level is preferably the level which is determinant for the deductive step of making the actual diagnose. Control level in a preferred embodiment relates to the level of the respective circRNA in a healthy subject or a population of healthy subjects, i.e. a subject not having the disease to be diagnosed, e.g. not having a neurodegenerative disease, such as Alzheimer's disease. The skilled person with the disclosure of the present application is in the position to determine suited control levels using common statistical methods.

In the context of the present invention, the levels of the one or more circRNA may be analyzed in a number of fashions well known to a person skilled in the art. For example, each assay result obtained may be compared to a "normal" or "control" value, or a value indicating a particular disease or outcome. A particular diagnosis/prognosis may depend upon the comparison of each assay result to such a value, which may be referred to as a diagnostic or prognostic "threshold". In certain embodiments, assays for one or more diagnostic or prognostic indicators are correlated to a condition or disease by merely the presence or absence of the circRNAs in the assay. For example, an assay can be designed so that a positive signal only occurs above a particular threshold level of interest, and below which level the assay provides no signal above background.

The sensitivity and specificity of a diagnostic and/or prognostic test depends on more than just the analytical "quality" of the test, they also depend on the definition of what constitutes an abnormal result, i.e. when a level may be regarded as differing from a control level. In practice, Receiver Operating Characteristic curves (ROC curves), are typically calculated by plotting the value of a variable versus its relative frequency in "normal" (i.e. apparently healthy individuals not having ovarian cancer) and "disease" populations. For any particular marker, a distribution of marker levels for subjects with and without a disease will likely overlap. Under such conditions, a test does not absolutely distinguish normal from disease with 100% accuracy, and the area of overlap indicates where the test cannot distinguish normal from disease. A threshold is selected, below which the test is considered to be abnormal and above which the test is considered to be normal. The area under the ROC curve is a measure of the probability that the perceived measurement will allow correct identification of a condition. ROC curves can be used even when test results don't necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" or "control" population, and a ROC curve created. These methods are well known in the art. See, *e.g.,* Hanley et al. 1982. Radiology 143: 29-36*.* Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5% of a given measurement.

The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

In other embodiments, a positive likelihood ratio, negative likelihood ratio, odds ratio, or hazard ratio is used as a measure of a test's ability to predict risk or diagnose a disease. In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group.

The skilled artisan will understand that associating a diagnostic or prognostic indicator, with a diagnosis or with a prognostic risk of a future clinical outcome is a statistical analysis. For example, a marker level of lower than X may signal that a patient is more likely to suffer from an adverse outcome than patients with a level more than or equal to X, as determined by a level of statistical significance. For another marker, a marker level of higher than X may signal that a patient is more likely to suffer from an adverse outcome than patients with a level less than or equal to X, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value. See, *e.g.,* Dowdy and Wearden, Statistics for Research, John Wiley & Sons, New York, 1983*.* Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

Suitable threshold levels for the diagnosis of the disease can be determined for certain combinations of circRNAs. This can e.g. be done by grouping a reference population of patients according to their level of circRNAs into certain quantiles, e.g. quartiles, quintiles or even according to suitable percentiles. For each of the quantiles or groups above and below certain percentiles, hazard ratios can be calculated comparing the risk for an adverse outcome, i.e. a "disease" or "Alzheimer's disease", between those patients who have a certain disease and those who have not. In such a scenario, a hazard ratio (HR) above 1 indicates a higher risk for an adverse outcome for the patients. A HR below 1 indicates beneficial effects of a certain treatment in the group of patients. A HR around 1 (e.g. +/- 0.1) indicates no elevated risk for the particular group of patients. By comparison of the HR between certain quantiles of patients with each other and with the HR of the overall population of patients, it is possible to identify those quantiles of patients who have an elevated risk and those who benefit from medication and thereby stratify subjects according to the present invention.

In some cases presence of the disease will not affect patients with levels (e.g. in the fifth quintile) of a circRNA different from the "control level", while in other cases patients with levels similar to the control level will be affected (e.g. in the first quintile). However, with the above explanations and his common knowledge, a skilled person is able to identify those groups of patients having a disease, e.g. a neurodegenerative disease as Alzheimer's disease. Exemplarily, some combinations of levels of circRNAs are listed for Alzheimer's disease in the appended examples. In another embodiment of the invention, the diagnosis is determined by relating the patient's individual level of marker peptide to certain percentiles (e.g. 97.5^{th} percentile (in case increased levels being indicative for a disease) or the 2.5^{th} percentile (in case decreased levels being indicative for a disease)) of a healthy population.

Kaplan-Meier estimators may be used for the assessment or prediction of the outcome or risk (e.g. diagnosis, relapse, progression or morbidity) of a patient.

"Equal" level in context with the present invention means that the levels differ by not more than ± 10 %, preferably by not more than ± 5 %, more preferably by not more than ± 2 %."Decreased" or "increased" level in the context of the present invention mean that the levels differ by more than 10 %, preferably by more than 15 %, preferably more than 20 %.
The term "subject" relates to a subject to be diagnosed, preferably a subject suspected to have or to have a risk for acquiring a disease, preferably a neurodegenerative disease, more preferably a subject suspected to have or to have a risk for acquiring Alzheimer's disease. The subject is preferably an animal, more preferably a mammal, most preferably a human.

The inventors have found that differential abundance of circRNA in samples of a bodily fluid is suited as a biomarker. It has been found that differing levels of circRNAs are correlating with a disease. This has been proven for Alzheimer's disease, a disease of neuronal tissue. Without being bound by reference, the correlation may be due to a passage of the circRNAs through the blood-brain-barrier, i.e. the circRNAs detected in the method according to the present disclosure are differentially expressed in the tissue of the disease, i.e. the tissue of interest. In case of the neurodegenerative disease (e.g. Alzheimer's disease) the tissue of interest is neuronal tissue, e.g. the brain. Hence, in one embodiment of the present disclosure said one or more circRNA, the differing levels of which in the bodily fluid are attributed to the presence of the disease, is differentially expressed between the diseased and non-diseased state in the tissue of interest. Alternatively, the circRNA levels may be of secondary nature, e.g. arise due to an immune response in the bodily fluid. Hence, in a further embodiment said one or more circRNA, the differing levels of which in the bodily fluid are attributed to the presence of the disease, is not differentially expressed between the diseased and non-diseased state in the tissue of interest.

Circular RNA" (circRNA) has been previously described. However, not in connection with their detection in a bodily fluid, e.g. blood. The skilled person is able to determine whether a detected RNA is a circular RNA. In particular, a circRNA does not contain a free 3'-end or a free 5' end, i.e. the entire nucleic acid is circularized. The circRNA is preferably a circularized, single stranded RNA molecule. Furthermore, the circRNA according to the present disclosure is a result of a head-to-tail splicing event that results in a discontinuous sequence with respect to the genomic sequence encoding the RNA. This means that a first sequence being present 5'-upstream of a second sequence in the genomic context, on the circRNA said first sequence at its 5'-end is linked to the 3' end of said second sequence and thereby closing the circle. The consequence of this arrangement is that at the junction where the 5'-end of said first sequence is linked to the 3'-end of said second sequence a unique sequence is build that is neither present in the genomic context nor in the normally transcribed RNA, e.g. mRNA. It has been found by the inventors that these junctions in all identified circRNAs, in the genomic context, are flanked by the canonical splice sequence, the GT/AG splice signal known by the skilled person. Hence, the circRNAs according to the present disclosure preferably contain an exon-exon junction in a head-to-tail arrangement, as visualized in Figure 1A as a result of a back-splicing reaction. The skilled person will recognize that a usual mRNA transcript contains exon-exon junctions in a tail-to-head arrangement, i.e. the 3'end (tail) of exon being upstream in the genomic context is linked to the 5'end (head) of the exon being downstream in the genomic context. The actual junction, i.e. the point at which the one exon is linked to the other is also referred to herein as "breakpoint". In a preferred embodiment the presence or absence of a circRNA or the level of a circRNA is determined by detection of an exon-exon-junction in a head-to-tail arrangement. One possible approach is exemplified in the enclosed examples. The detection of circular RNA has been previously described in Memczak S, Jens M, Elefsinioti A, et al. Circular RNAs are a large class of animal RNAs with regulatory potency. Nature. 2013; 495(7441):333-338, in particular as relates to the detection and annotation of circRNAs. The biogenesis of many mammalian circRNAs depends on complementary sequences within flanking introns (*see* Ashwal-Fluss R, Meyer M, Pamudurti NR, et al. circRNA Biogenesis Competes with Pre-mRNA Splicing. MOLCEL. 2014; 1-12; Rybak-Wolf A, Stottmeister C, Glažar P, et al. Circular RNAs in the Mammalian Brain Are Highly Abundant, Conserved, and Dynamically Expressed. MOLCEL. 2015;1-17; Zhang X-O, Wang H-B, Zhang Y, et al. Complementary Sequence-Mediated Exon Circularization. Cell. 2014; Liang D, Wilusz JE. Short intronic repeat sequences facilitate circular RNA production. Genes and Development. 2014; Conn SJ, Pillman KA, Toubia J, et al. The RNA Binding Protein Quaking Regulates Formation of circRNAs. Cell. 2015;160(6):1125-1134; and Ivanov A, Memczak S, Wyler E, et al. Analysis of Intron Sequences Reveals Hallmarks of Circular RNA Biogenesis in Animals. CellReports. 2015;10(2):170-177). Hence, in one embodiment the two introns upstream and downstream of and direct adjacent in the genomic context to the exons of the exon-exon junction (i.e. forming the exon-exon junction) in a head to tail arrangement often contain complementary sequences, e.g. a complementary sequence stretch of at least 15 nucleotides, preferably 500 nucleotides, more preferably 1000 nucleotides. For detection of circRNA in principle, the RNA of a sample is sequenced after reverse transcription and library preparation. Afterwards, the sequences are analyzed for the presence of exon-exon junctions in a head-to-tail arrangement. For instance RNA sequenced can be mapped to a reference genome using common mapping programs and software, e.g. bowtie2 (version 2.1.0; *see* Langmead B, Salzberg SL. Fast gapped-read alignment with Bowtie 2. Nature Methods. 2012; 9(4):357-359). Human reference genomes are known to the skilled person and include the human reference genome hg19 (Feb 2009, GRCh37; downloadable from the UCSC genome browser; *see* Kent WJ, Sugnet CW, Furey TS, et al. The human genome browser at UCSC. Genome Research. 2002; 12(6):996-1006). Although circRNA detection in blood is possible without any preprocessing of the total RNA sample, it is preferred to deplete ribosomal RNAs (rRNA), preferably the majority of rRNA, to increase the sensitivity of circRNA detection, in particular when using RNA Sequencing approaches. To this end, the content of rRNAin the sample should be depleted to less than 20 %, preferably less than 10 %, more preferably less than 2 % with respect to the total RNA content. The rRNA depletion may performed as known in the art, e.g. it may be facilitated by commercially available kits (e.g. Ribominus, Themo Scientific) or enzymatic methods (Xian Adiconis et al. Comprehensive comparative analysis of RNA sequencing methods for degraded or low input samples Nat Methods. 2013 Jul; 10(7): 10.1038/nmeth.2483.).

Further, in a preferred embodiment RNA sequences which map continuously to the genome by aligning without any trimming (end-to-end mode) are neglected. Reads not mapping continuously to the genome are preferably used for circRNA candidate detection. The terminal sequences (anchors) from the sequences, e.g. 20 nt or more, may be extracted and re-aligned independently to the genome. From this alignment the sequences may be extended until the full circRNA sequence is covered, i.e. aligned. Consecutively aligning anchors indicate linear splicing events whereas alignment in reverse orientation indicates head-to-tail splicing as observed in circRNAs *(see* Figure 1A). The so identified resulting splicing events are filtered using the following criteria 1) GT/AG signal flanking the splice sites in the genomic context; 2) the breakpoint, i.e. the exon-exon-junction can be unambiguously detected; and 3) no more than 100 kilobases distance between the two splice sites in the genomic context. Furthermore, further optional criteria may be used, depending on the method chosen; e.g. a maximum of two mismatches when extending the anchor alignments; a breakpoint no more than two nucleotides inside the alignment of the anchors; at least two independent reads supporting the head-to-tail splice junction; and/or a minimum difference of 35 in the bowtie2 alignment score between the first and the second best alignment of each anchor.

The circRNAs according to the present disclosure may be detected using different techniques.

As outlined herein, the exon-exon junction in a head-to-tail arrangement is unique to the circRNAs. Hence, the detection of these is preferred. Nucleic acid detection methods are commonly known to the skilled person and include probe hybridization based methods, nucleic acid amplification based methods, and nucleic acid sequencing, or combinations thereof. Hence, in a preferred embodiment of the present invention circRNA is detected using a method selected from the group consisting of probe hybridization based methods, nucleic acid amplification based methods, and nucleic acid sequencing.

Probe hybridization based method employ the feature of nucleic acids to specifically hybridize to a complementary strand. To this end nucleic acid probes may be employed that specifically hybridize to the exon-exon junction in a head-to-tail arrangement of the circRNA, i.e. to a sequence spanning the exon-exon junction, preferably to the region extending from 10 nt upstream to 10 nt downstream of the exon-exon junction, preferably to the region from 20 nt upstream to 20 nt downstream of the exon-exon junction, or even a greater region spanning the exon-exon junction. The skilled person will recognize that hybridization probes specifically hybridizing to the respective sequence of the circRNA may be used, as well as hybridization probes specifically hybridizing to the reverse complement sequence thereof, e.g. in case the circRNA is previously reverse transcribed to cDNA and/or amplified.

Hybridization can also be used as a measure of homology between two nucleic acid sequences. A nucleic acid sequence hybridizing specifically to an exon-exon junction in a head-to-tail arrangement according to the present disclosure may be used as a hybridization probe according to standard hybridization techniques. The hybridization of the probe to DNA or RNA from a test source (e.g., the bodily fluid, like whole blood, or amplified nucleic acids from the sample of the bodily fluid) is an indication of the presence of the relevant circRNA in the test source.

Hybridization conditions are known to those skilled in the art and can be found in Current Protocols in Molecular Biology, John Wiley & Sons, N.Y., 6.3.1-6.3.6, 1991. Preferably, specific hybridization refers to hybridization under stringent conditions. "Stringent conditions" are defined as equivalent to hybridization in 6X sodium chloride/sodium citrate (SSC) at 45°C, followed by a wash in 0.2 X SSC, 0.1 % SDS at 65°C; or as equivalent to hybridization in commercially available hybridization buffers (e.g. ULTRAHyb, ThermoScientific) for blotting techniques and 5x SSC 0.5% SDS (750 mM NaCl, 75 mM sodium citrate, 0.5% sodiumdodecylsulfate, pH 7.0) for array based detection methods at 65°C.

The means and methods of the present invention preferably comprise the use of nucleic acid probes. A nucleic acid probe according to the present invention is an oligonucleotide, nucleic acid or a fragment thereof, which is substantially complementary to a specific nucleic acid sequence. "substantially complementary" refers to the ability to hybridize to the specific nucleic acid sequence under stringent conditions.

The skilled person knows means and methods to determine the levels of nucleic acids in a sample and compare them to control levels. Such methods may employ labeled nucleic acid probes according to the invention. "Labels" include fluorescent or enzymatic active labels as further defined herein below. Such methods include real-time PCR methods and microarray methods, like Affimetrix®, nanostring and the like.

The determination of the circRNAs or their level may also be detected using sequencing techniques. The skilled person is able to use sequencing techniques in connection with the present invention. Sequencing techniques include but are not limited to Maxam-Gilbert Sequencing, Sanger sequencing (chain-termination method using ddNTPs), and next generation sequencing methods, like massively parallel signature sequencing (MPSS), polony sequencing, 454 pyrosequencing, Illumina (Solexa) sequencing, SOLiD sequencing, or ion torrent semiconductor sequencing or single molecule, real-time technology sequencing (SMRT).

The detection/determination of the circRNAs and the respective level may also employ nucleic acid amplification method alone or in combination with the sequencing and/or hybridization method. Nucleic acid amplification may be used to amplify the sequence of interest prior to detection. It may however also be used for quantifying a nucleic acid, e.g. by real-time PCR methods. Such methods are commonly known to the skilled person. Nucleic acid amplification methods for example include rolling circle amplification (such as in Liu, et al., "Rolling circle DNA synthesis: Small circular oligonucleotides as efficient templates for DNA polymerases," J. Am. Chem. Soc. 118:1587-1594 (1996).), isothermal amplification (such as in Walker, et al., "Strand displacement amplification--an isothermal, in vitro DNA amplification technique," Nucleic Acids Res. 20(7):1691-6 (1992)), ligase chain reaction (such as in Landegren, et al., "A Ligase-Mediated Gene Detection Technique," Science 241:1077-1080, 1988, or, in Wiedmann, et al., "Ligase Chain Reaction (LCR)--Overview and Applications," PCR Methods and Applications (Cold Spring Harbor Laboratory Press, Cold Spring Harbor Laboratory, NY, 1994) pp. S51-S64.)). Nucleic-acid amplification can be accomplished by any of the various nucleic-acid amplification methods known in the art, including but not limited to the polymerase chain reaction (PCR), ligase chain reaction (LCR), transcription-based amplification system (TAS), nucleic acid sequence based amplification (NASBA), rolling circle amplification (RCA), transcription-mediated amplification (TMA), self-sustaining sequence replication (3SR) and Qβ amplification. It will be readily understood that the amplification of the circRNA may start with a reverse transcription of the RNA into complementary DNA (cDNA), optionally followed by amplification of the so produced cDNA.

It may be desirable to reduce or diminish non circRNA prior to the determination or the presence or level of the circRNAs. To this end RNA degrading agents may be added to the sample and/or the isolated total nucleic acids, e.g. total RNA, thereof, wherein said RNA degrading agent does not degrade circRNAs or does degrade circRNAs only at lower rates as compared to linear RNAs. One such agent is RNase R. RNase R is a 3'-5' exoribonuclease closely related to RNase II, which has been shown to be involved in selective mRNA degradation, particularly of non stop mRNAs in bacteria (*see* Cheng; Deutscher, MP et al. (2005). "An important role for RNase R in mRNA decay". Molecular Cell 17(2):313-318; and Venkataraman, K; Guja, KE; Garcia-Diaz, M; Karzai, AW (2014). "Non-stop mRNA decay: a special attribute of trans-translation mediated ribosome rescue."; Frontiers in microbiology 5:93. Suzuki H1, Zuo Y, Wang J, Zhang MQ, Malhotra A, Mayeda A; Characterization of RNase R-digested cellular RNA source that consists of lariat and circular RNAs from pre-mRNA splicing; Nucleic Acids Res. 2006 May 8;34(8):e63.). RNase R has homologues in many other organisms. When a part of another larger protein has a domain that is very similar to RNase R, this is called an RNase R domain. Hence, in a preferred embodiment the sample is treated with RNase R before determination of the circRNA to deplete linear RNA isoforms from the total RNA preparation and thereby increase detection sensitivity.

As outlined herein, the diagnostic or prognostic value of a single circRNA may not be sufficient in order to allow a diagnosis or prognosis with a reliable result. In such case it may be desirable to determine the presence or level of more than one circRNA in the sample and optionally comparing them to the respective control level. The skilled person will acknowledge that these more than one circRNAs may be chosen from a predetermined panel of circRNAs. Such panel usually includes the minimum number of circRNAs necessary to allow a reliable diagnosis or prognosis. The number of circRNAs of the panel may vary depending on the desired reliability and/or the prognostic or diagnostic value of the included circRNAs, e.g. when determined alone. Hence, the method according to the present invention in a preferred embodiment determines more than one circRNA from a panel of circRNAs, e.g. their presence or absence, or level, respectively.

The panel for obtaining the desired may be chosen according to the needs. In particular the skilled person may apply statistical approaches as outlined herein in order to validate the diagnostic and/or prognostic significance of a certain panel. The inventors have herein shown for a neurodegenerative disease the development of a certain panel of circRNAs giving a reasonable degree of certainty. The skilled person may apply common statistical techniques in order to develop a panel of circRNAs. Such statistical techniques include cluster analysis (e.g. hierarchical or k-means clustering), principle component analysis or factor analysis.

In principle, the statistical methods aim the identification of circRNAs or panels of circRNAs that exhibit differing presence and/or levels in samples of diseased and healthy/normal subjects. As outlined, the panel is preferably a panel of more than one circRNA, i.e. a plurality. In a preferred embodiment of the invention said panel comprises a plurality of circRNAs that have been identified as being present at differing levels in bodily fluid samples of patients having the disease and patients not having the disease. The panel of circRNAs has been preferably identified by principle component analysis or clustering.

The "principle component analysis" (PCA) (as also used exemplified herein) regards the analysis of factors differing between diseased and healthy subjects. PCA is known to the skilled person (*see* Pearson K., "On lines and planes of closest fit to systems of points in space", The London, Edinburgh, and Dublin Philosophical Magazine and Journal of Science 2.11 559-572 (1901), and Hotelling H., "Analysis of a complex of statistical variables into principal components" Journal of educational psychology 24.6 417 (1933)). The circRNAs to be chosen for the principle component analysis may be those previously determined in samples of healthy and/or diseased subject. Thresholds may be incorporated in order to consider a circRNA for further analysis, in a preferred embodiment only circRNAs having an expression value of at least 6.7 after variance stabilizing transformation of raw read counts in one of the samples. PCA may be performed on circRNAs included in the analysis using the prcomp function of the standard package "stats" of the "R" programming language (R Core Team (2013). R: A language and environment for statistical computing. R Foundation for Statistical Computing, Vienna, Austria. ISBN 3-900051-07-0). Depending on the circRNAs chosen, the disease and other factors, the weights can vary. However, the skilled artisan will acknowledge that these circRNAs with the highest weight as regards the principle component of interest, i.e. disease/healthy state, shall be chosen in order to obtain the circRNAs with the highest predictive absolute values. PCA is used to visualize and measure the amount of variation in a data set. Mathematically, PCA is an orthogonal linear transformation that transforms the data to a new coordinate system such that the greatest variance by some projection of the data lies on the first coordinate which is called Principal Component 1 (PC1) and so on. The before mentioned calculated weight represents the distance of each circular RNA to this specific projection. Thus, the higher the absolute value the more relevant is for this projection.

"Hierarchical Clustering" (also referred to herein as "clustering") may be performed as known in the art (*reviewed in* Murtagh, F and Conteras, P "Methods of hierarchical clustering" arXiv preprint arXiv:1105.0121 (2011)). Samples may be clustered on log₂ transformed normalized circRNA expression profiles (log₂(nᵢ + 1)). Hierarchical, agglomerative clustering may be performed with complete linkage and optionally by further using Spearman's rank correlation as distance metric (1 - {corr [log(nᵢ + 1)]}). "The goal of cluster analysis is to partition observations (here circRNA expression) into groups ("clusters") so that the pairwise dissimilarities between those assigned to the same cluster tend to be smaller than those in different clusters" (*see* Friedman J, Hastie T, and Tibshiriani R, "The elements of statistical learning", Vol. 1. Soringer, Berlin: Springer series in statistics (2001)). Here, the measure for dissimilarity is defined as the Spearman's rank correlation. A visualization and complete description of the hierarchical clustering is provided by a dendrogram.

The inventors have exemplified the method outlined above for a neurodegenerative disease, in particular Alzheimer's disease. A neurodegenerative disease in context with the present disclosure is to be understood as a disease associated with neurodegeneration. Neurodegeneration means a progressive loss of structure or function of neurons, including death of neurons. Many neurodegenerative diseases including ALS, Parkinson's, Alzheimer's, and Huntington's occur as a result of neurodegenerative processes. Nowadays, many similarities exist that relate these diseases to one another on a sub-cellular level. There are many parallels between different neurodegenerative disorders including atypical protein assemblies (protein misfolding and/or agglomeration) as well as induced cell death. Neurodegeneration can be found in many different levels of neuronal circuitry ranging from molecular to systemic.

Alzheimer's disease has been identified as a protein misfolding disease (proteopathy), causing plaque accumulation of abnormally folded amyloid beta protein, and tau protein in the brain. Plaques are made up of small peptides, 39-43 amino acids in length, called amyloid beta (Aβ). Aβ is a fragment from the larger amyloid precursor protein (APP). APP is a transmembrane protein that penetrates through the neuron's membrane. APP is critical to neuron growth, survival, and post-injury repair. In Alzheimer's disease, an unknown enzyme in a proteolytic process causes APP to be divided into smaller fragments. One of these fragments gives rise to fibrils of amyloid beta, which then form clumps that deposit outside neurons in dense formations known as senile plaques. AD is also considered a tauopathy due to abnormal aggregation of the tau protein. In AD, tau undergoes chemical changes, becoming hyperphosphorylated; it then begins to pair with other threads, creating neurofibrillary tangles and disintegrating the neuron's transport system. A patient, is classified as having Alzheimer's disease according to the criteria as set by the National Institute of Neurological and Communicative Disorders and Stroke (NINCDS) and the Alzheimer's disease and Related Disorders Association (ADRDA, now known as the Alzheimer's Association), the NINCDS-ADRDA Alzheimer's Criteria for diagnosis in 1984, extensively updated in 2007 (*see* McKhann G, Drachman D, Folstein M, et al. Clinical Diagnosis of Alzheimer's disease: Report of the NINCDS-ADRDA Work Group under the Auspices of Department of Health and Human Services Task Force on Alzheimer's disease. Neurology. 1984;34(7):939-44; and Dubois B, Feldman HH, Jacova C, et al. Research Criteria for the Diagnosis of Alzheimer's disease: Revising the NINCDS-ADRDA Criteria. Lancet Neurology. 2007;6(8):734-469). These criteria require that the presence of cognitive impairment, and a suspected dementia syndrome, be confirmed by neuropsychological testing for a clinical diagnosis of possible or probable Alzheimer's disease. A histopathologic confirmation including a microscopic examination of brain tissue is required for a definitive diagnosis. Good statistical reliability and validity have been shown between the diagnostic criteria and definitive histopathological confirmation (*see* Blacker D, Albert MS, Bassett SS, et al. Reliability and validity of NINCDS-ADRDA criteria for Alzheimer's disease. The National Institute of Mental Health Genetics Initiative. Archives of Neurology. 1994;51(12):1198-204). Eight cognitive domains are most commonly impaired in AD-memory, language, perceptual skills, attention, constructive abilities, orientation, problem solving and functional abilities. These domains are equivalent to the NINCDS-ADRDA Alzheimer's Criteria as listed in the Diagnostic and Statistical Manual of Mental Disorders (DSM-IV-TR) published by the American Psychiatric Association.

The present invention relates to a method for diagnosing AD in a subject comprises the steps of:
- determining the level of one or more circRNA in a sample of a blood of said subject;
- comparing the determined level to a control level of said one or more circRNA;
wherein differing levels between the determined and the control level are indicative for Alzheimer's disease.

The inventors have identified specific circRNAs that have a predictive or diagnostic value as regards the neurodegenerative disease. In particular 910 highly expressed circRNAs have been identified that are differentially present in samples of patients with a neurodegenerative disease as compared to the healthy controls. These 910 circRNAs are particularly characterized by their exon-exon junction in a head-to-tail arrangement, as outlined herein above. The sequences encoding the 20 nucleotides upstream and 20 nucleotides downstream of said exon-exon junction in the respective circRNAs are given in SEQ ID NOs: 1 to 910. However, it may be sufficient to determine only 10 nucleotides upstream and 10 nucleotides downstream of the junction in order to detect the circRNAs specifically. Hence, in a preferred embodiment said one or more circRNA in the method for diagnosing the neurodegenerative disease comprises a sequence encoded by a sequence selected from the group consisting of nucleotides 11 to 30 of any of the sequences of SEQ ID NO:1 to SEQ ID NO:910. The circRNA may for instance be detected through determining the presence or levels of RNA comprising the respective sequences, e.g. by hybridization, sequencing and/or amplification methods as outlined herein.

SEQ ID NO:1 to 1820 list the DNA sequences encoding the sequences of the exon-exon junctions or the complete sequences of the circRNAs used in the present invention. "Encoded" in this regard means that the RNA encoded by the DNA sequence has the sequence of nucleotides as set out in the DNA sequence with the thymidines "T" being exchanged by uracils "U", the backbone being ribonucleic acid instead of deoxyribonucleic acid. X

The inventors found that the circRNAs are indicative for the presence or the risk of acquiring a neurodegenerative disease when present at increased or decreased levels. Whether the presence of the specific circRNA at decreased or increased levels is indicative for the neurodegenerative disease is given in Table 1. Hence, in a particular preferred embodiment the presence of increased or decreased levels as defined in Table 1 under "diseased" for the circRNA comprising the respectively encoded sequence are indicative for the presence of or risk of acquiring a neurodegenerative disease, preferably for Alzheimer's disease. As outlined in the Table's legend, "+" denotes that increased levels and/or the presence of the respective circRNA are indicative for the presence or risk of acquiring Alzheimer's disease, while "-" denotes that decreased levels and/or the absence of the respective circRNA are indicative for the presence or risk of acquiring Alzheimer's disease.

As mentioned, the circRNAs may be detected through the unique sequences occurring at the exon-exon junction in the head-to-tail arrangement. However, in one embodiment the circRNA may be detected through detection of a larger portion of their sequence. In one embodiment of the method for diagnosing a neurodegenerative disease, preferably Alzheimer's disease, said one or more circRNA has a sequence as encoded by a sequence selected from the group consisting of SEQ ID NO:911 to SEQ ID NO:1820. Preferably the presence of increased or decreased levels as defined in Table 1 under "disease" for the circRNA having the respective encoded sequence are indicative for the presence of a neurodegenerative disease, preferably Alzheimer's disease.

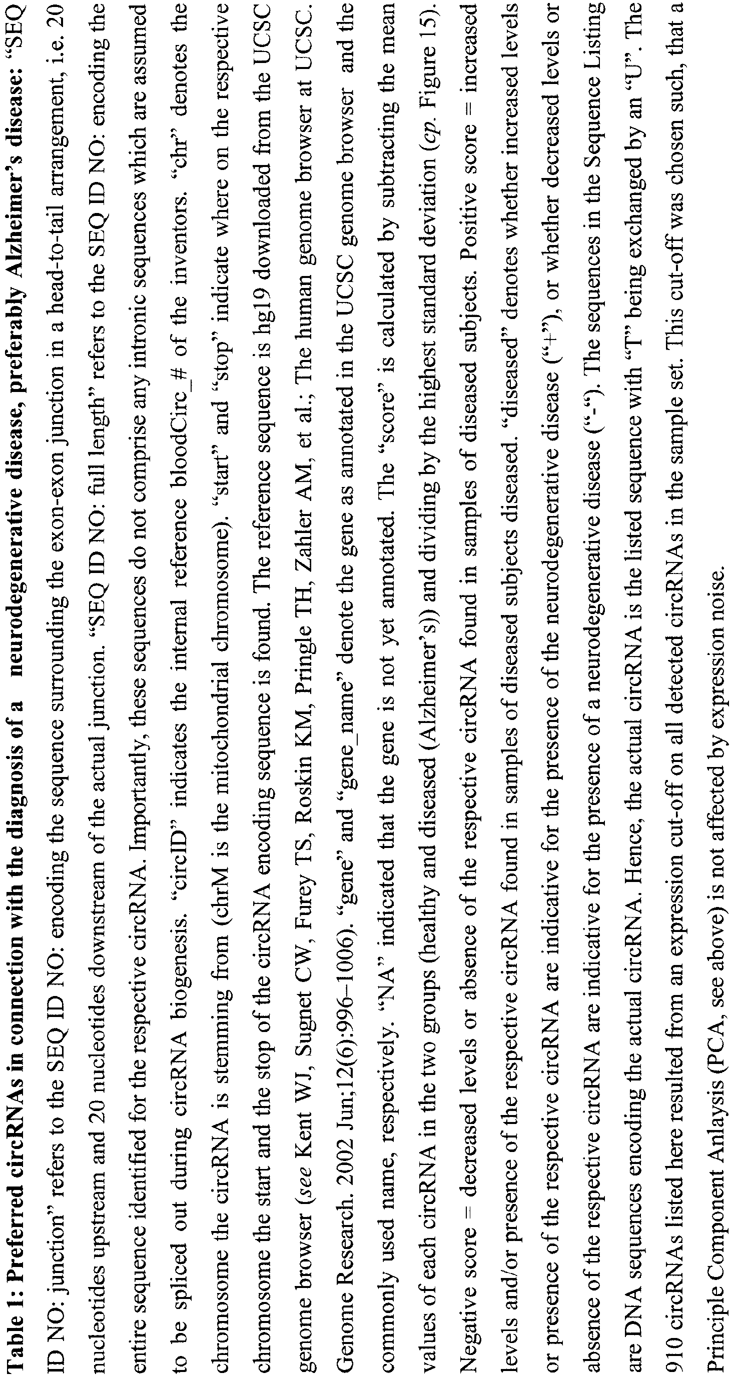

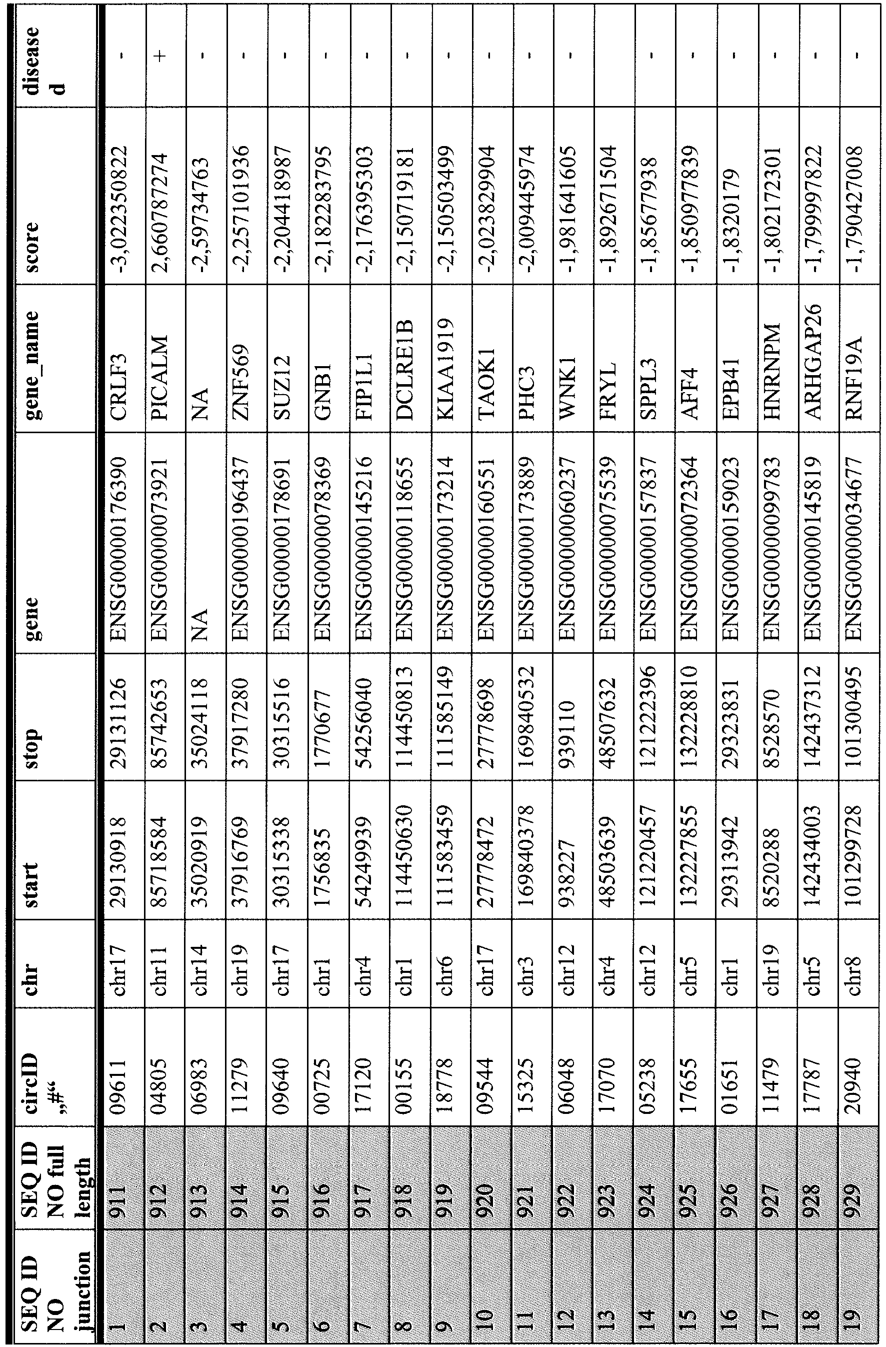

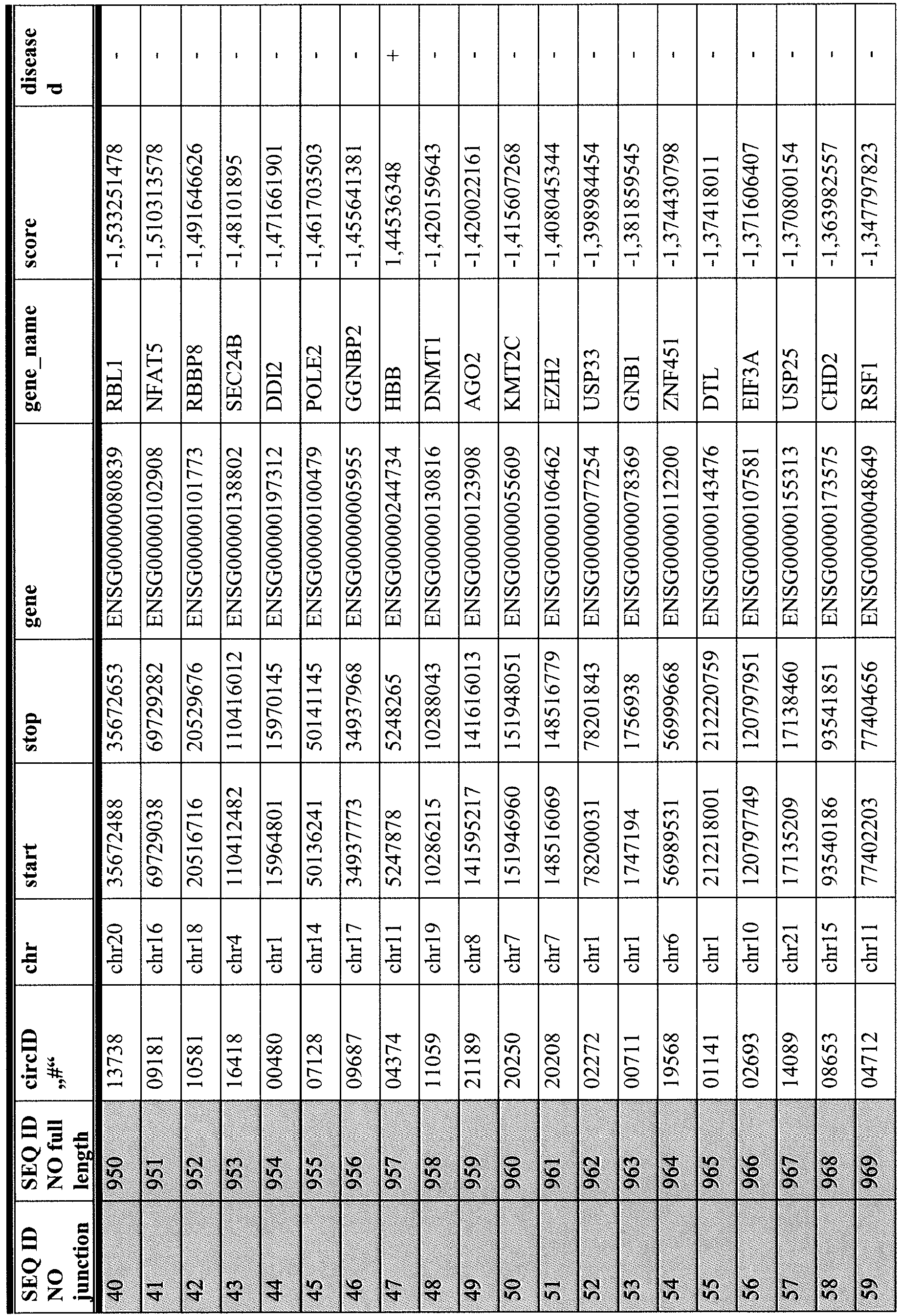

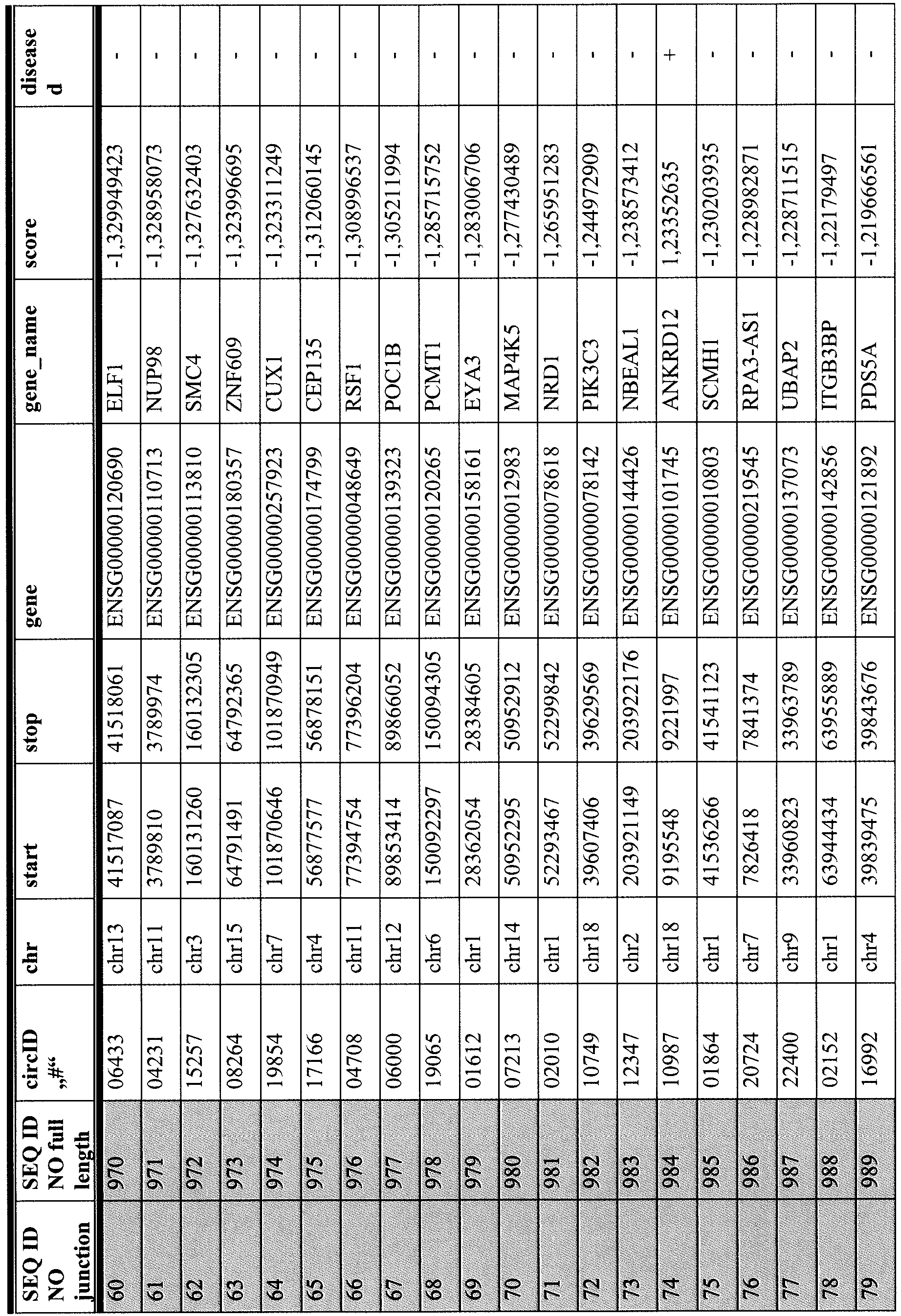

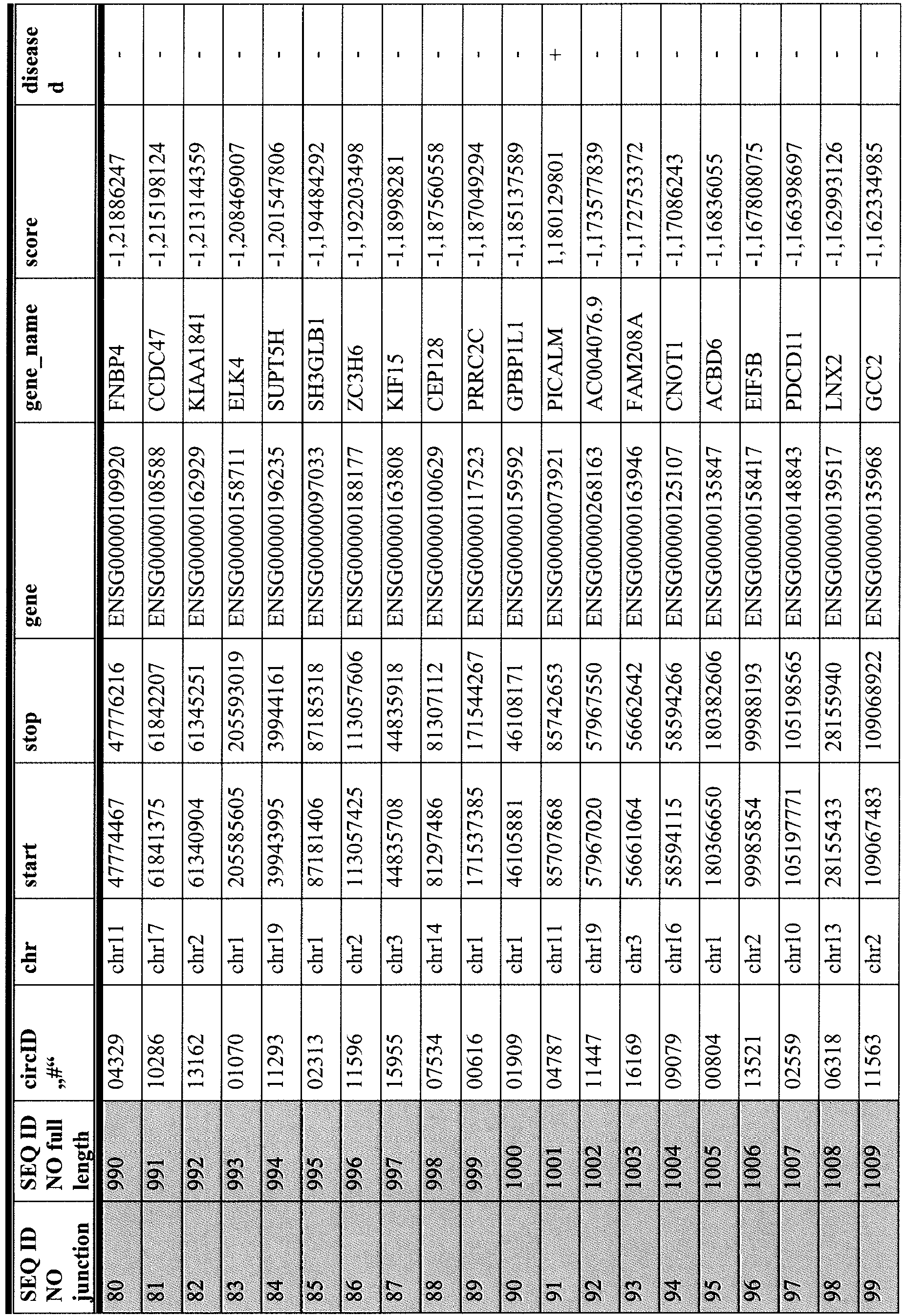

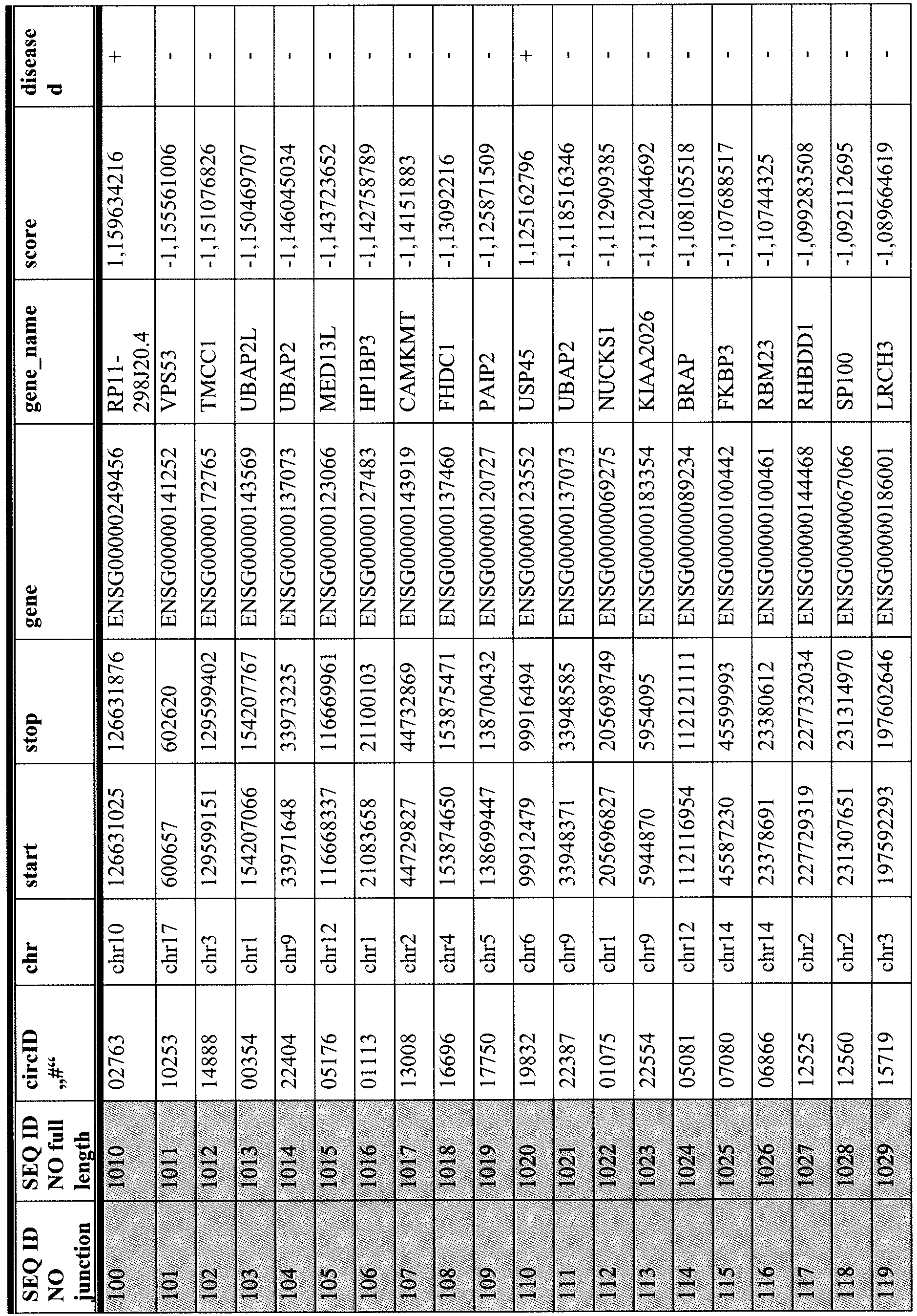

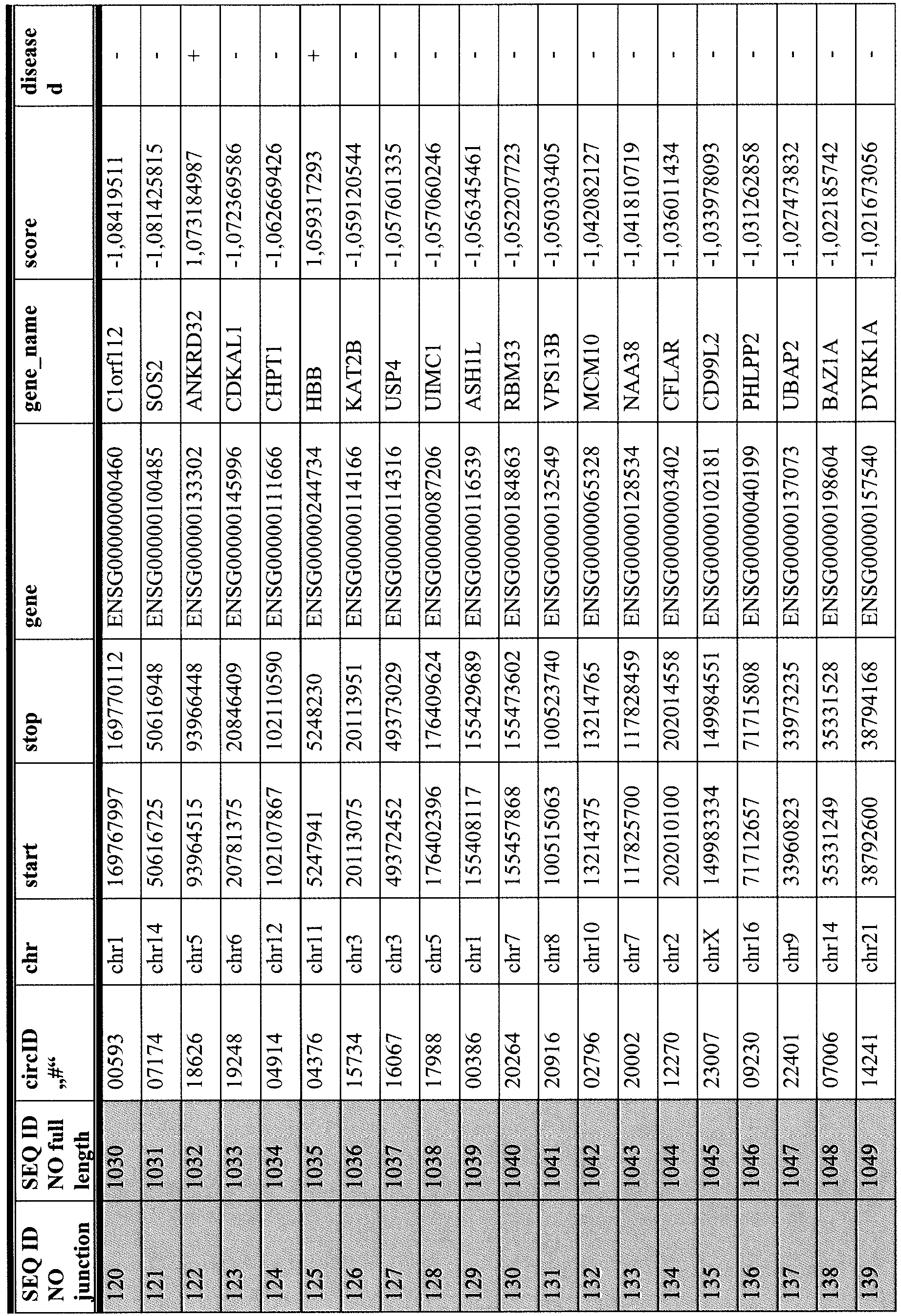

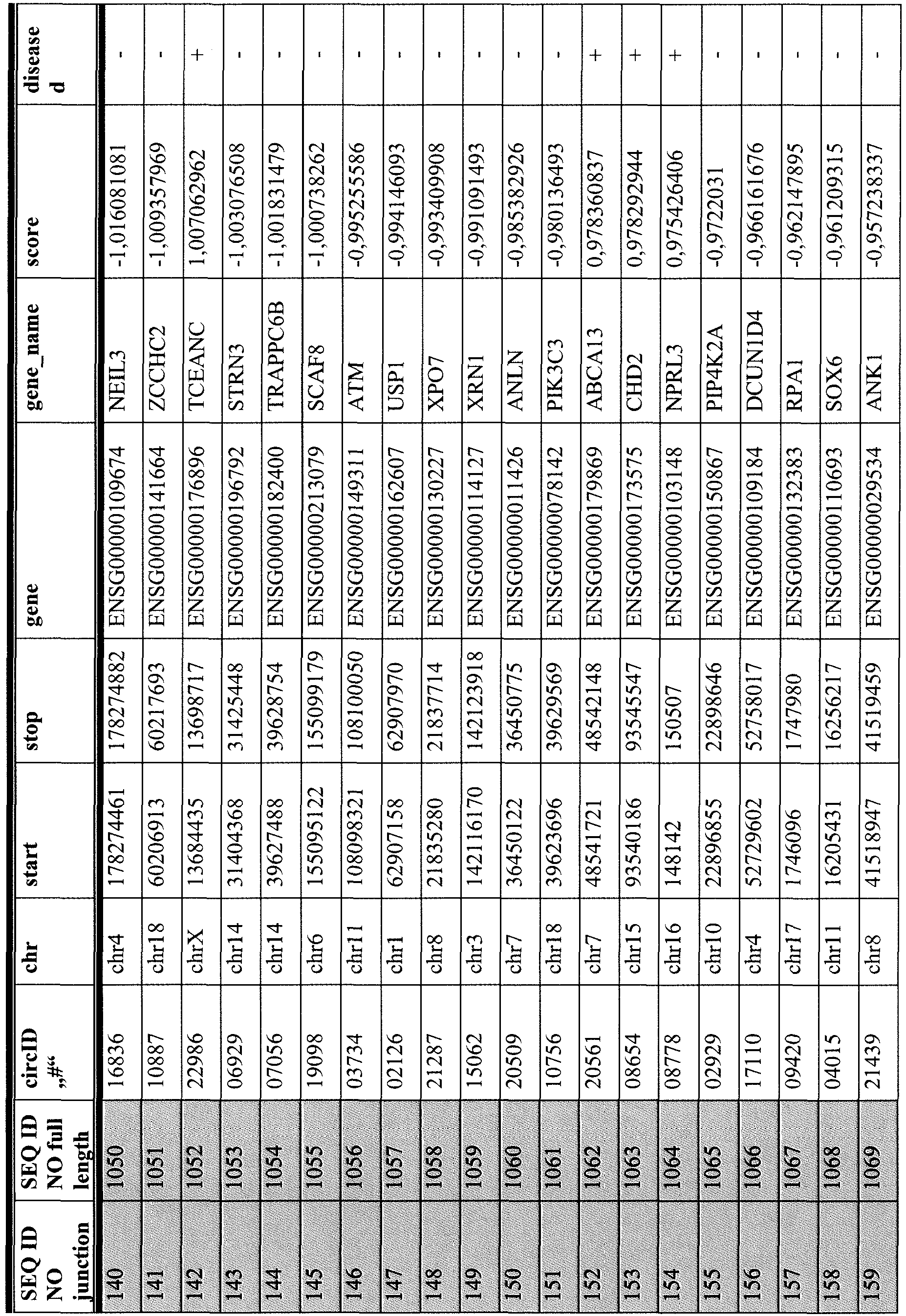

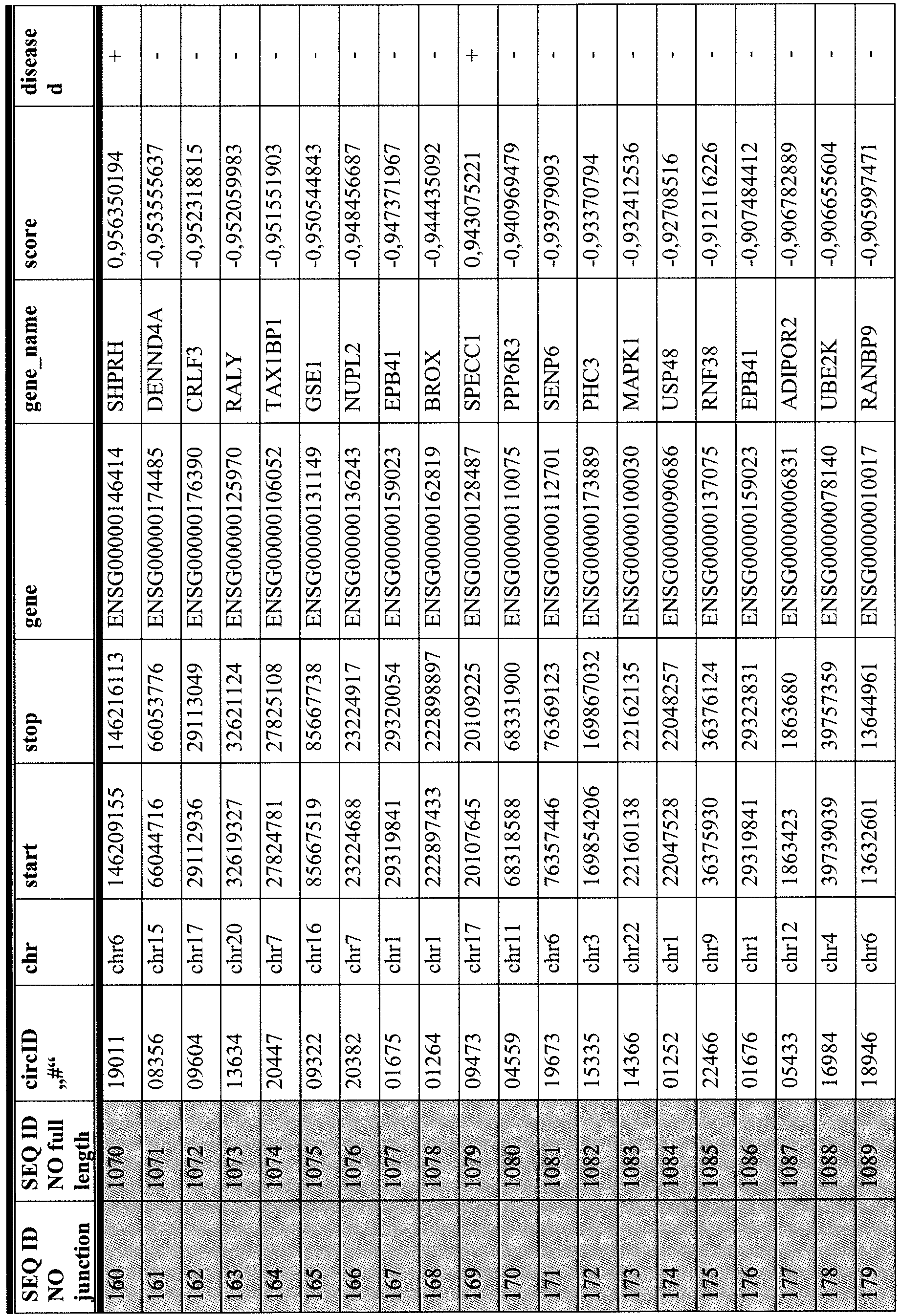

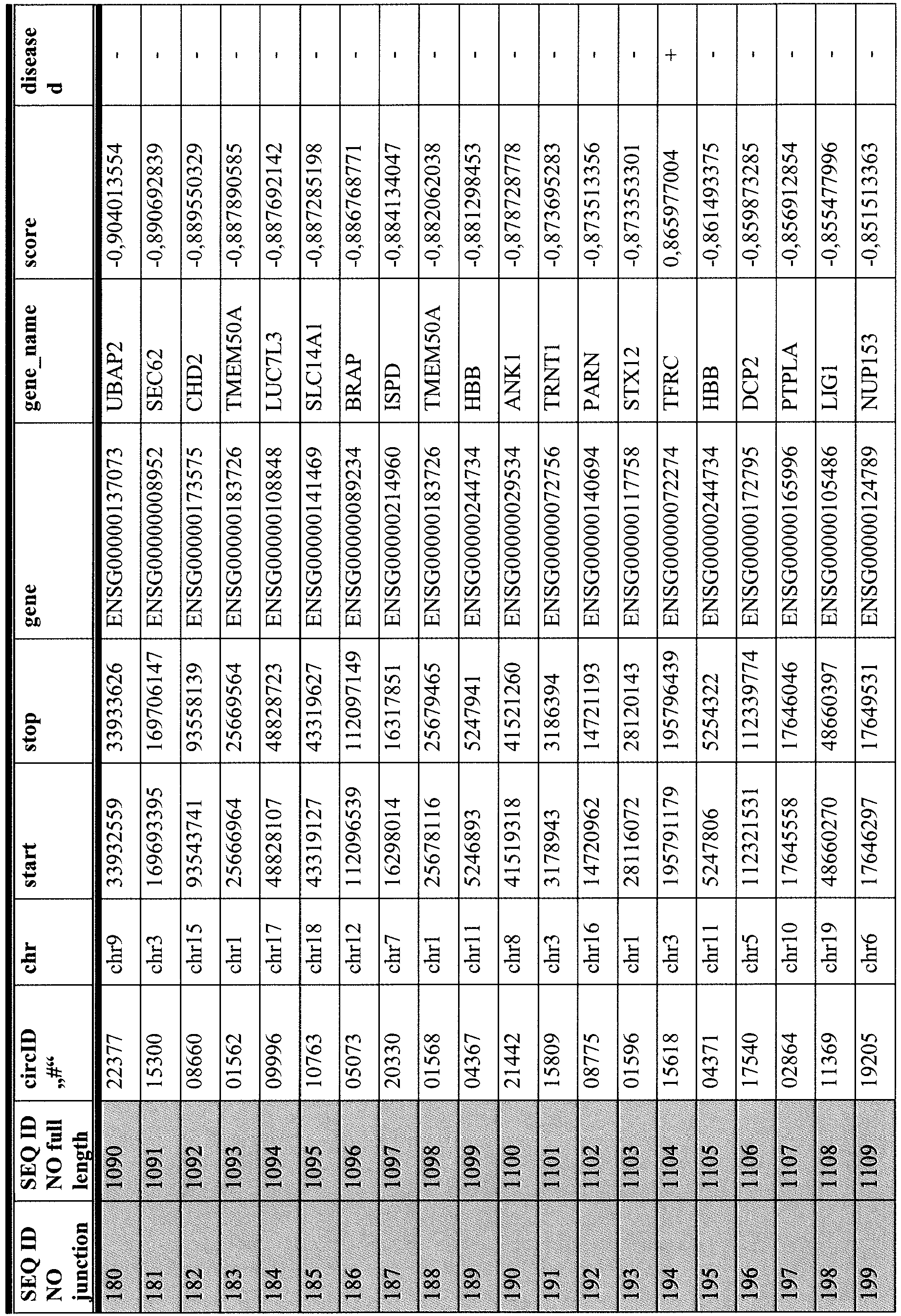

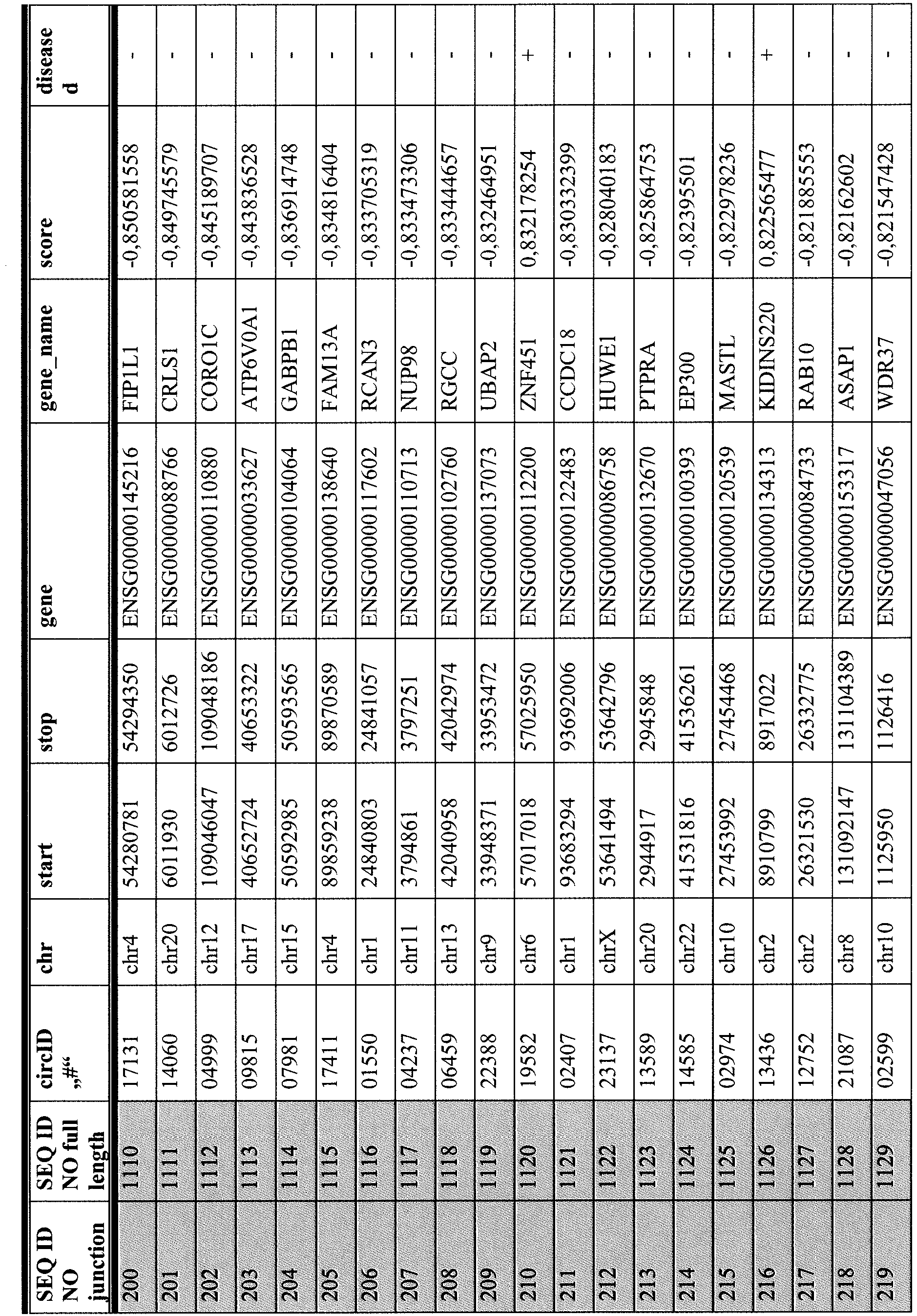

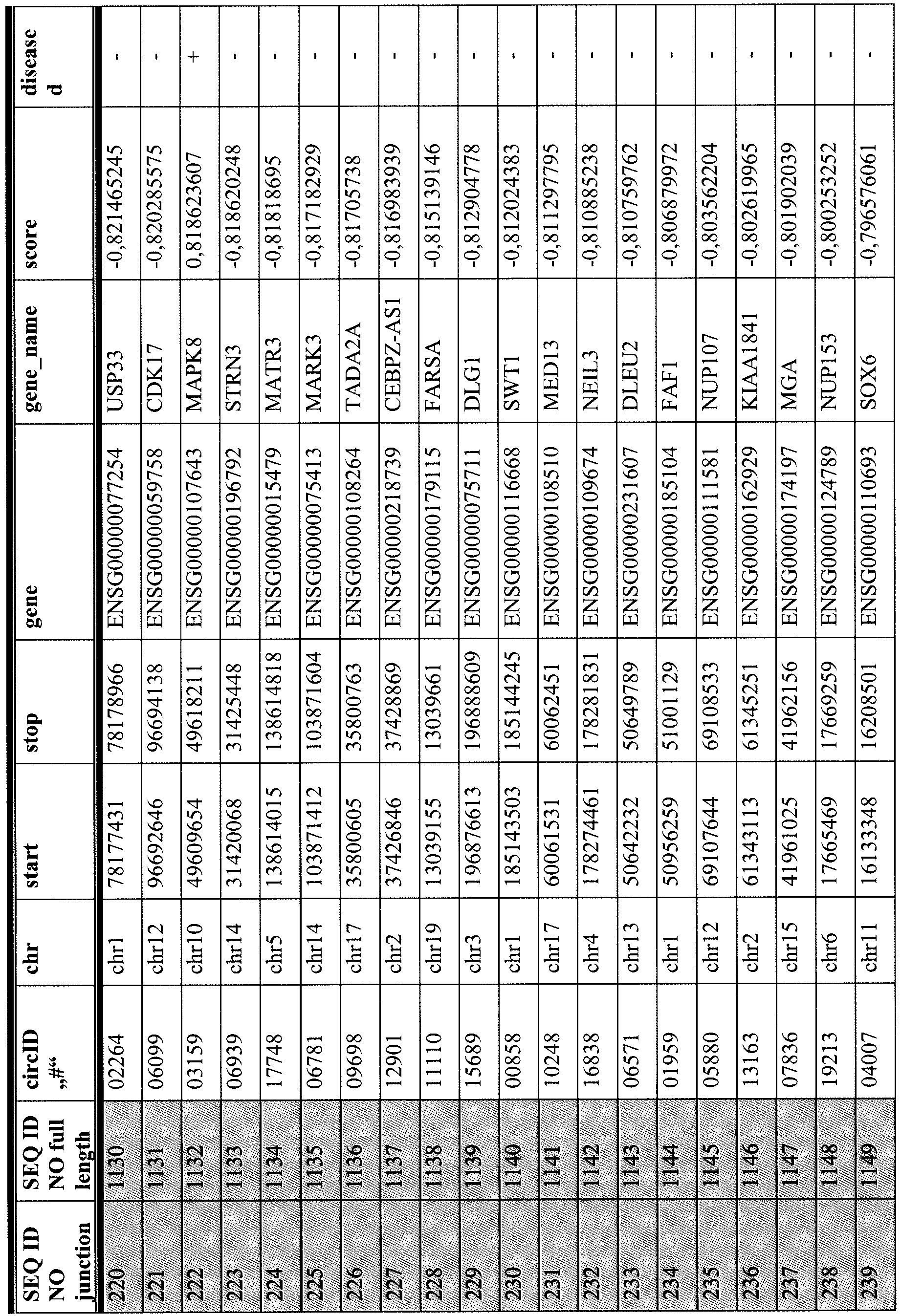

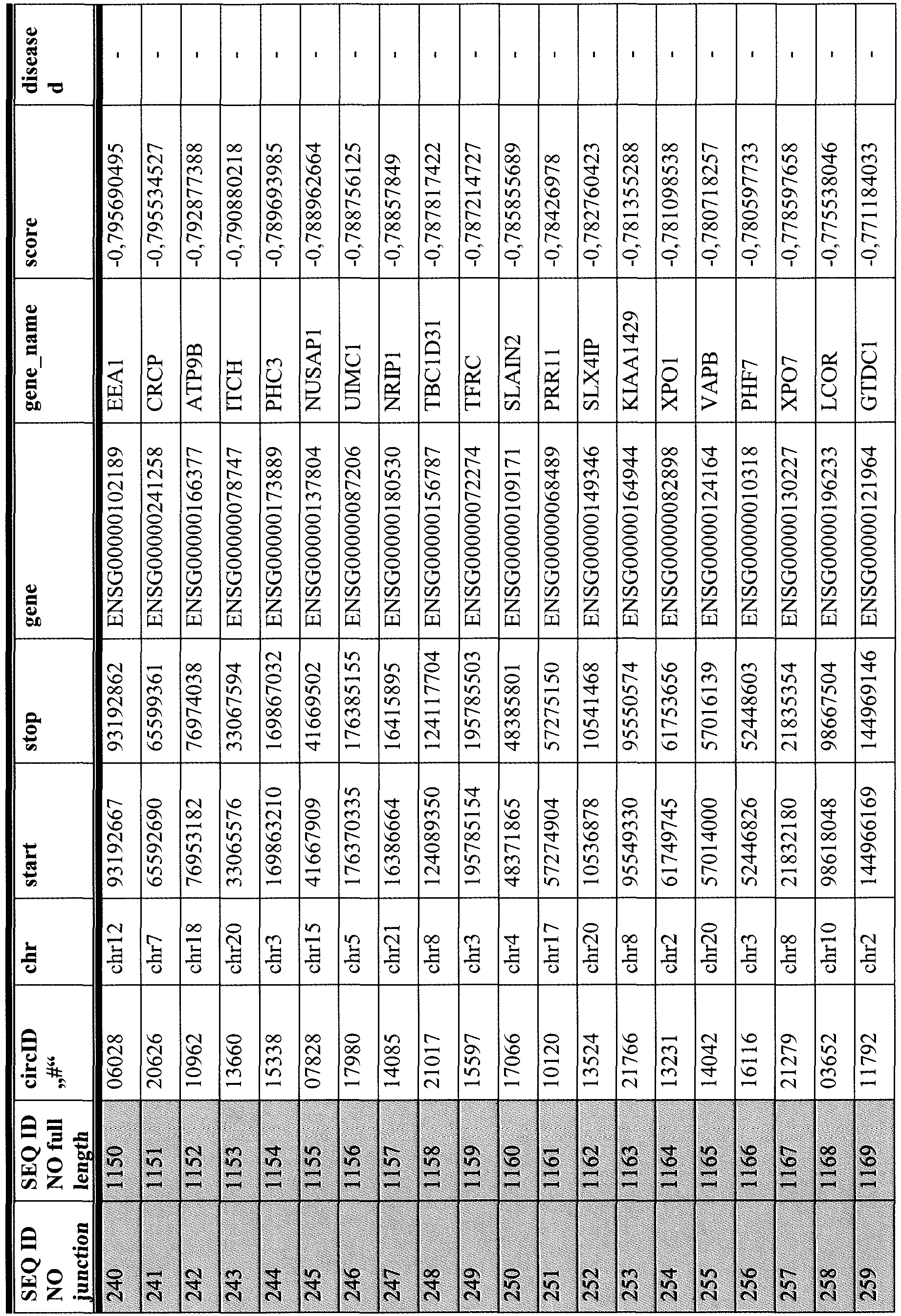

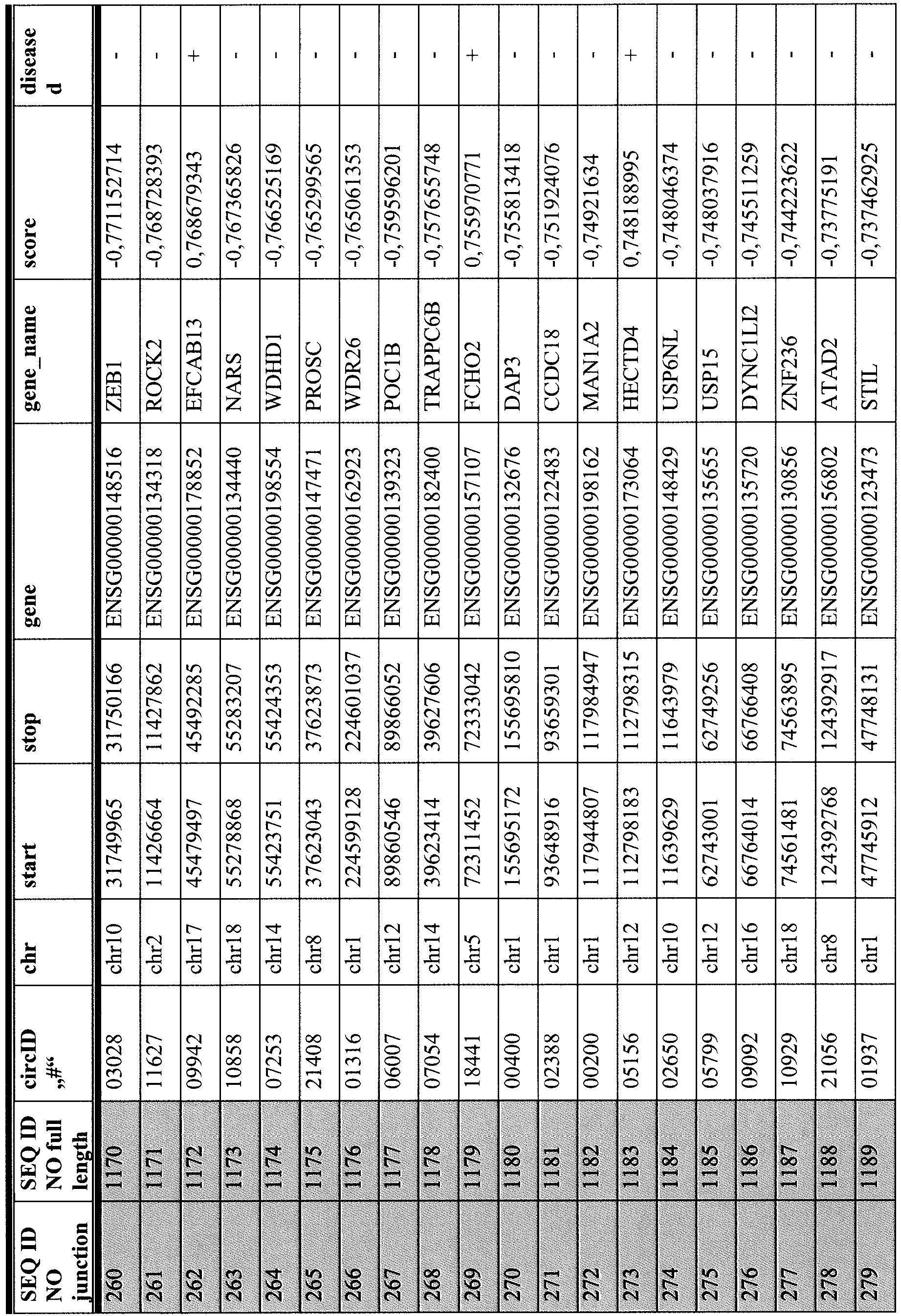

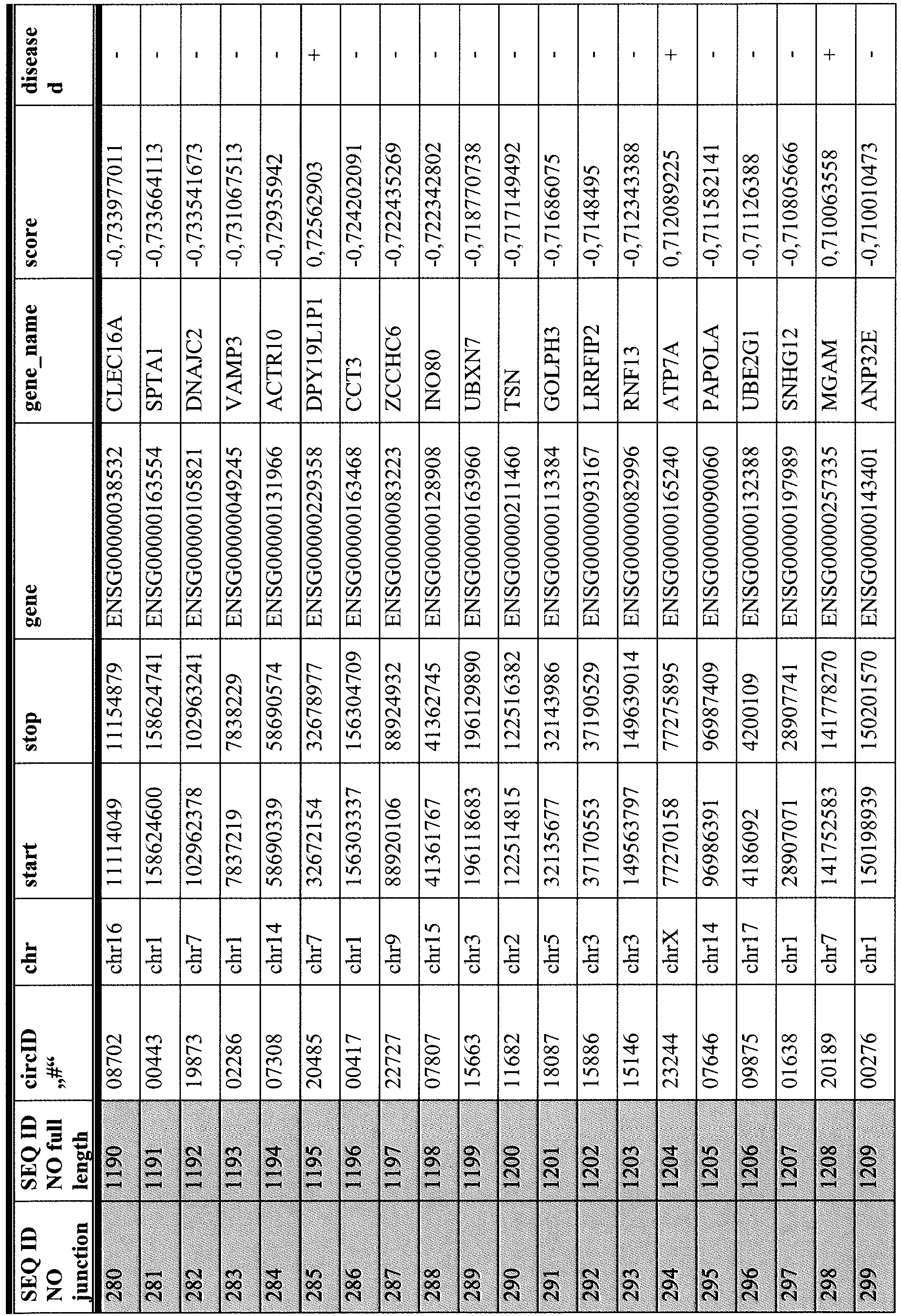

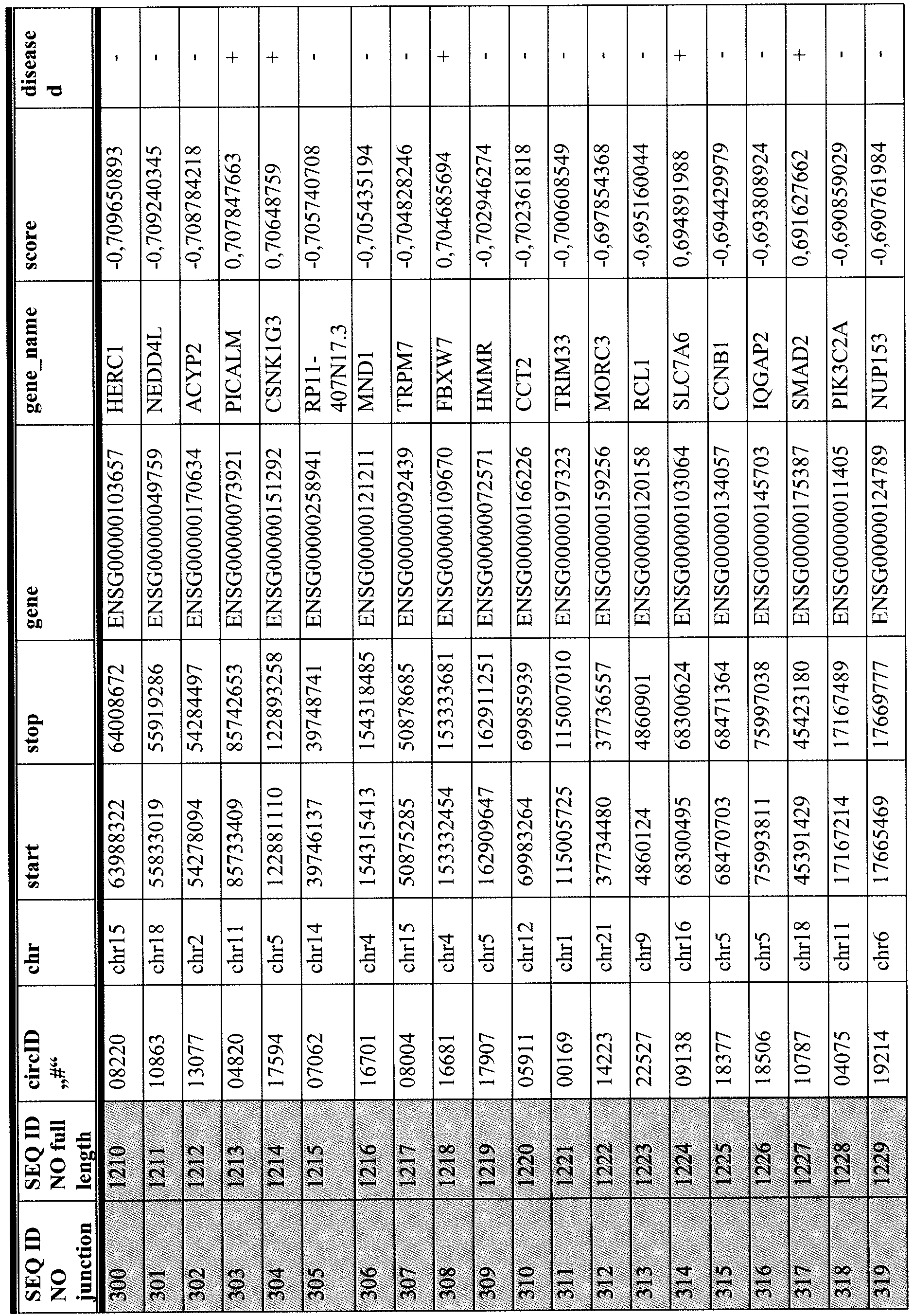

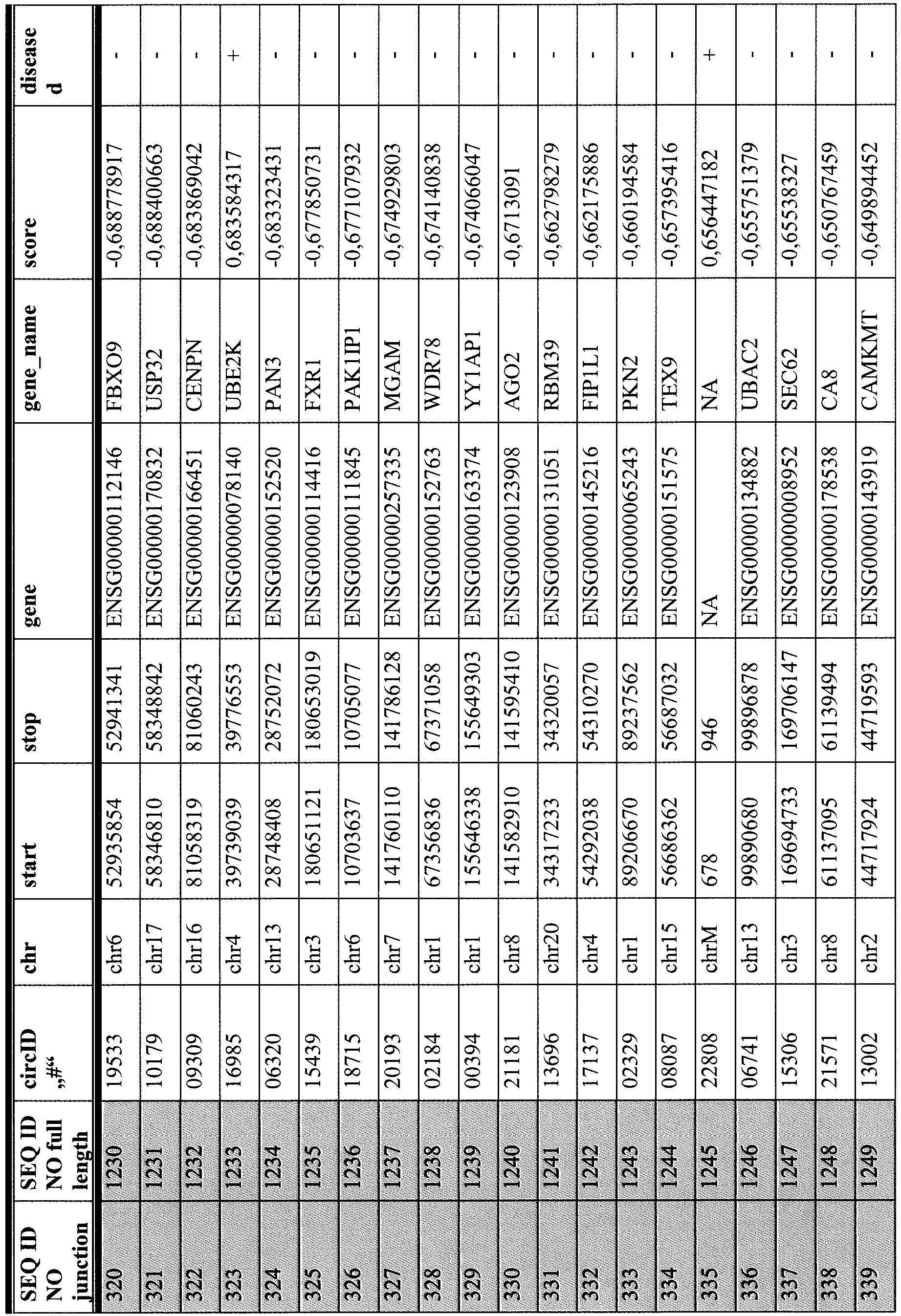

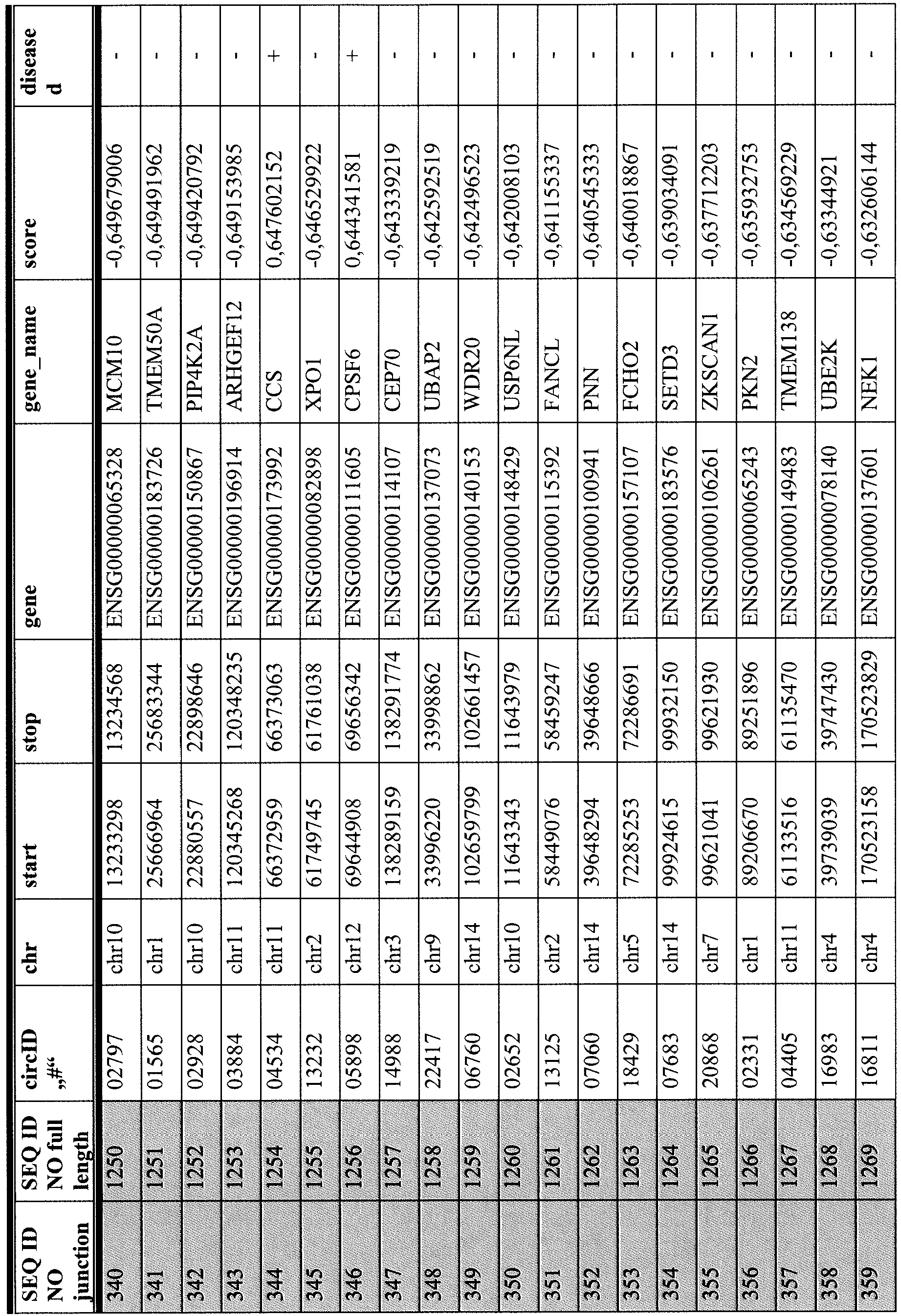

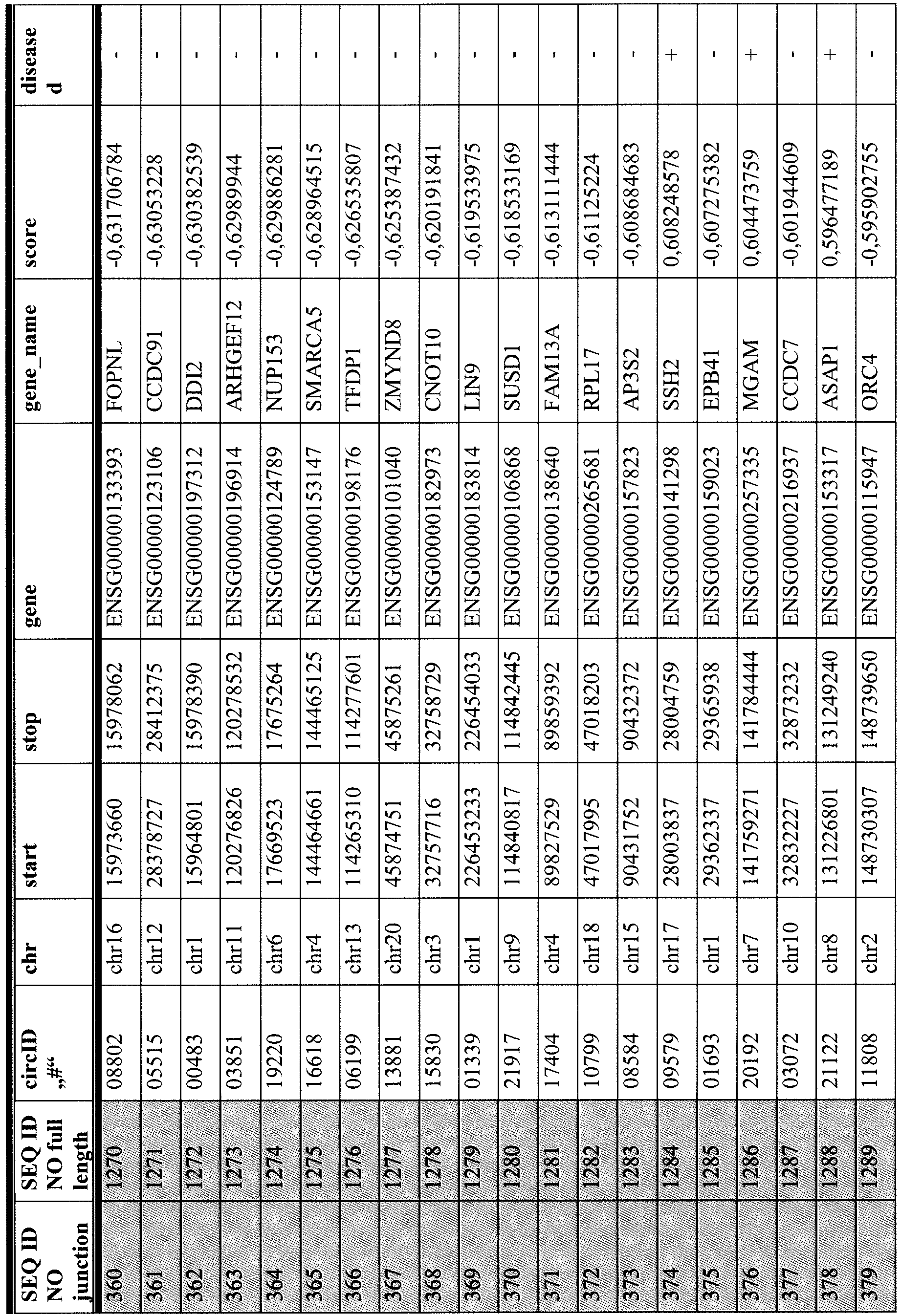

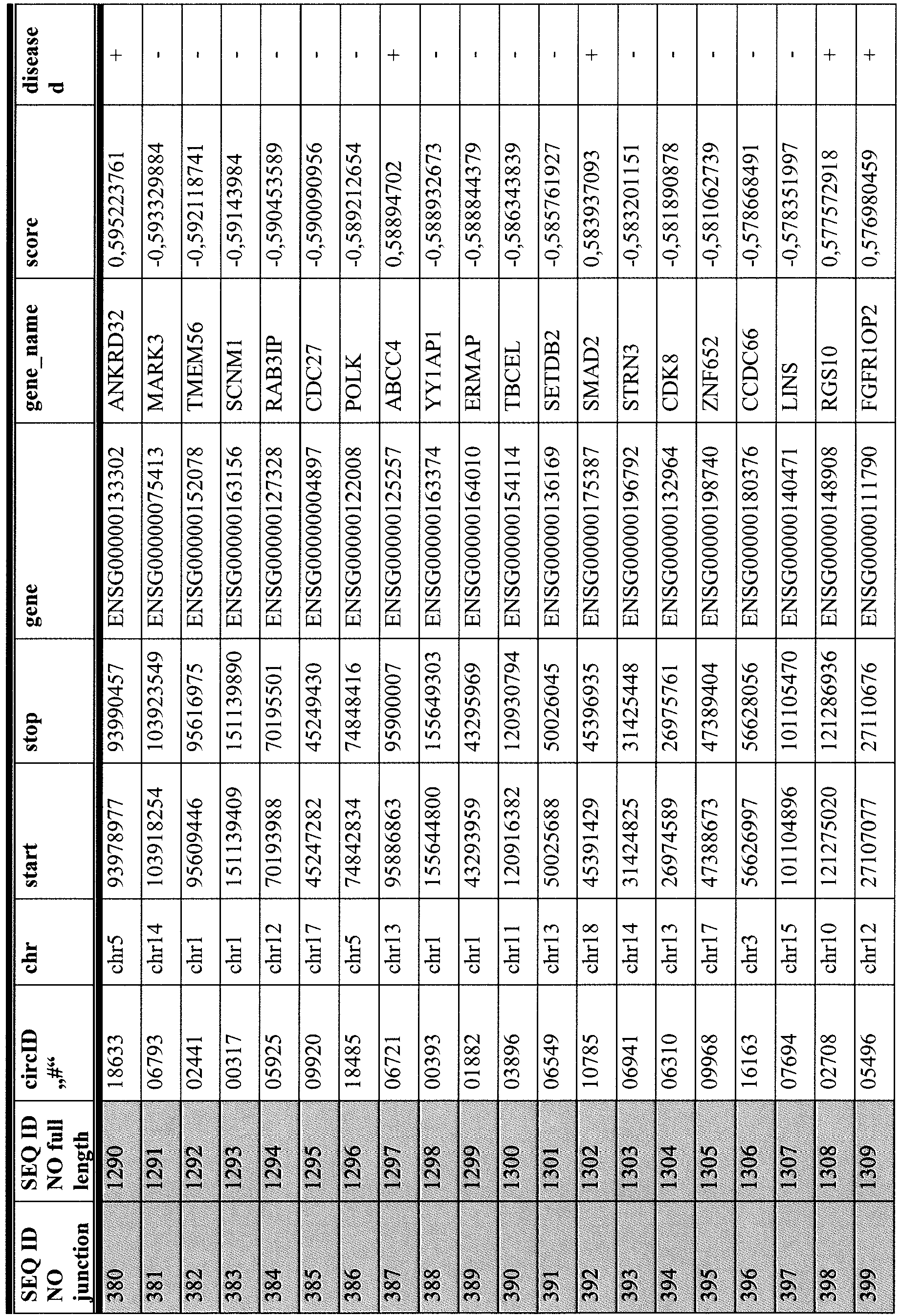

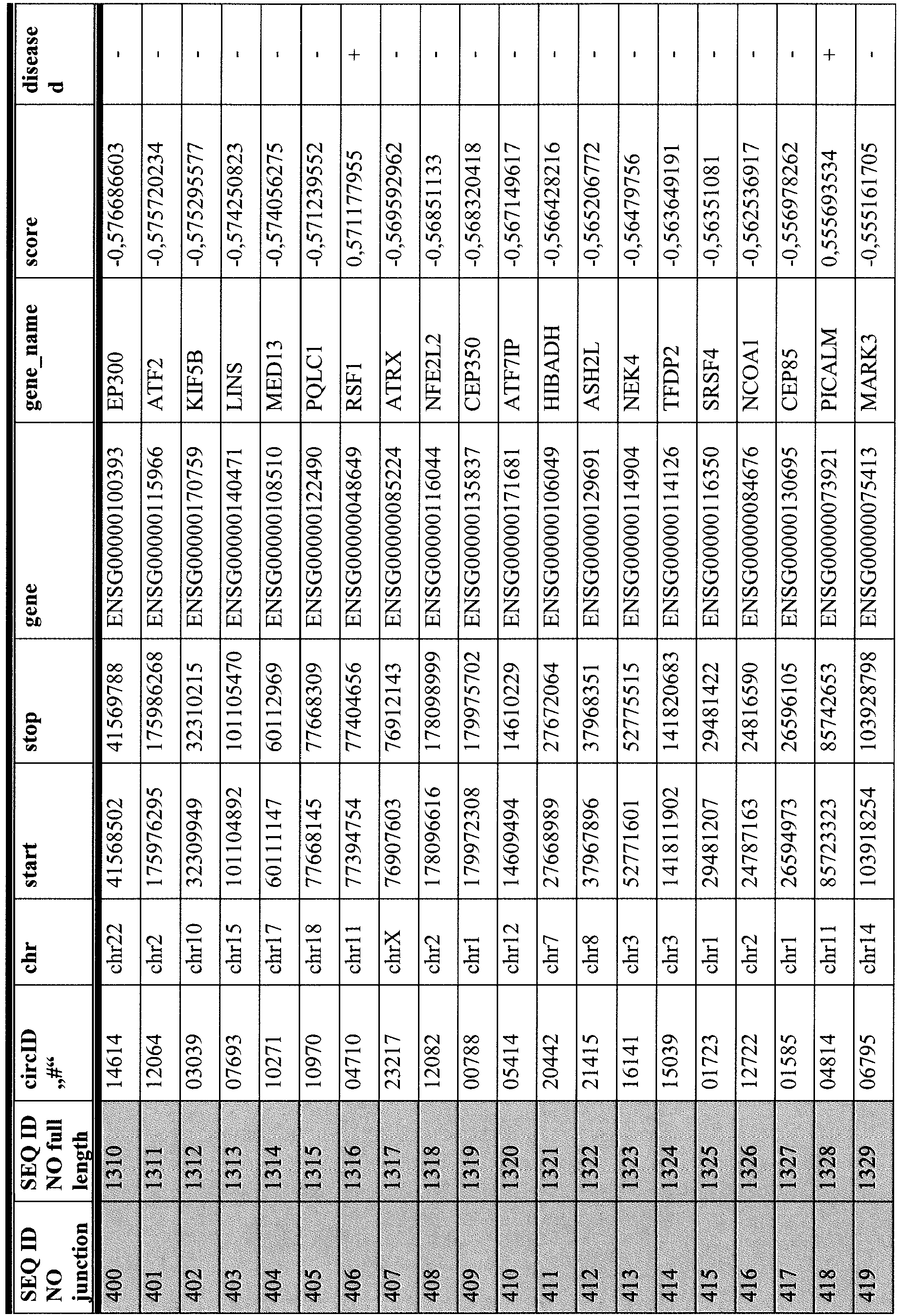

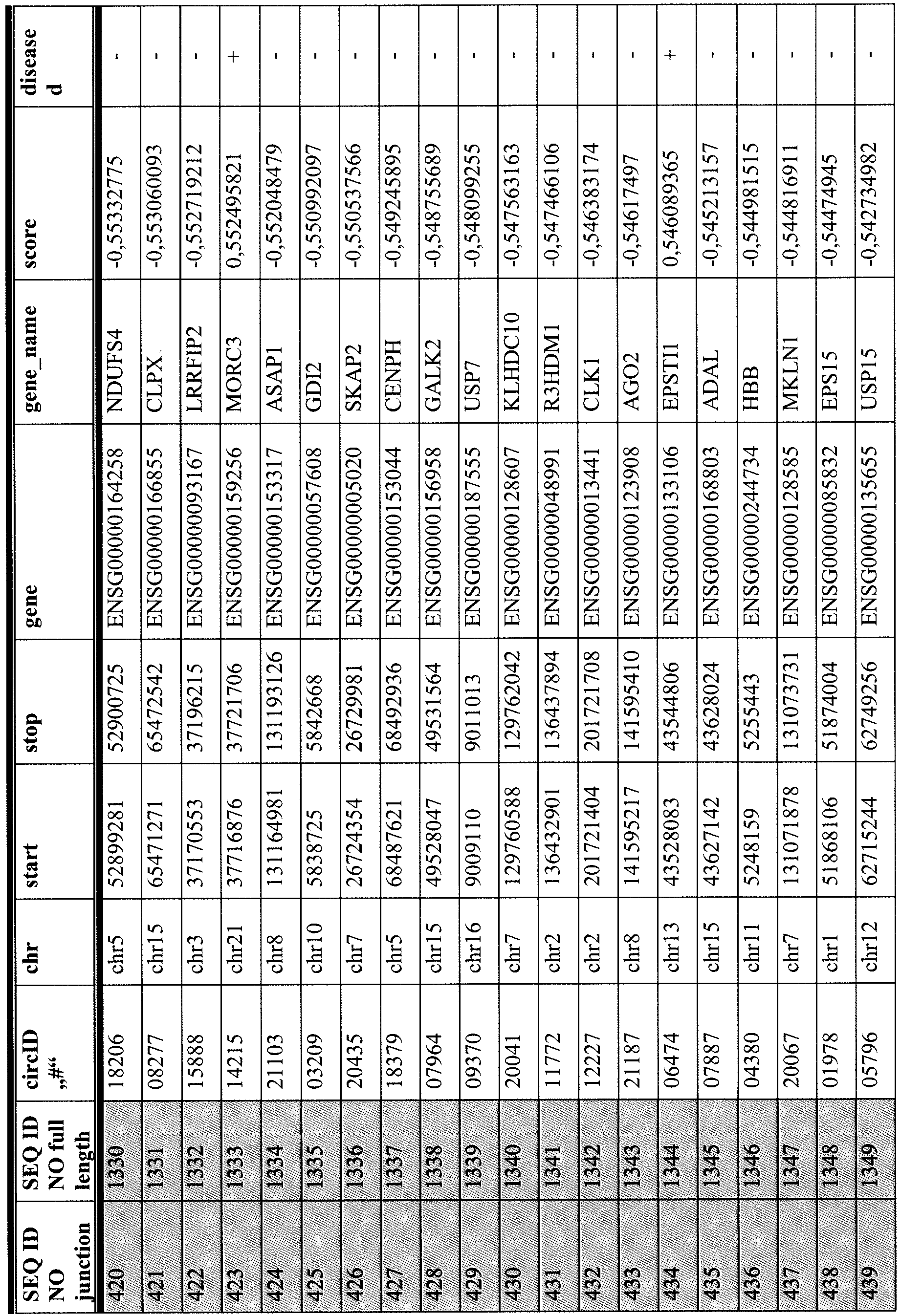

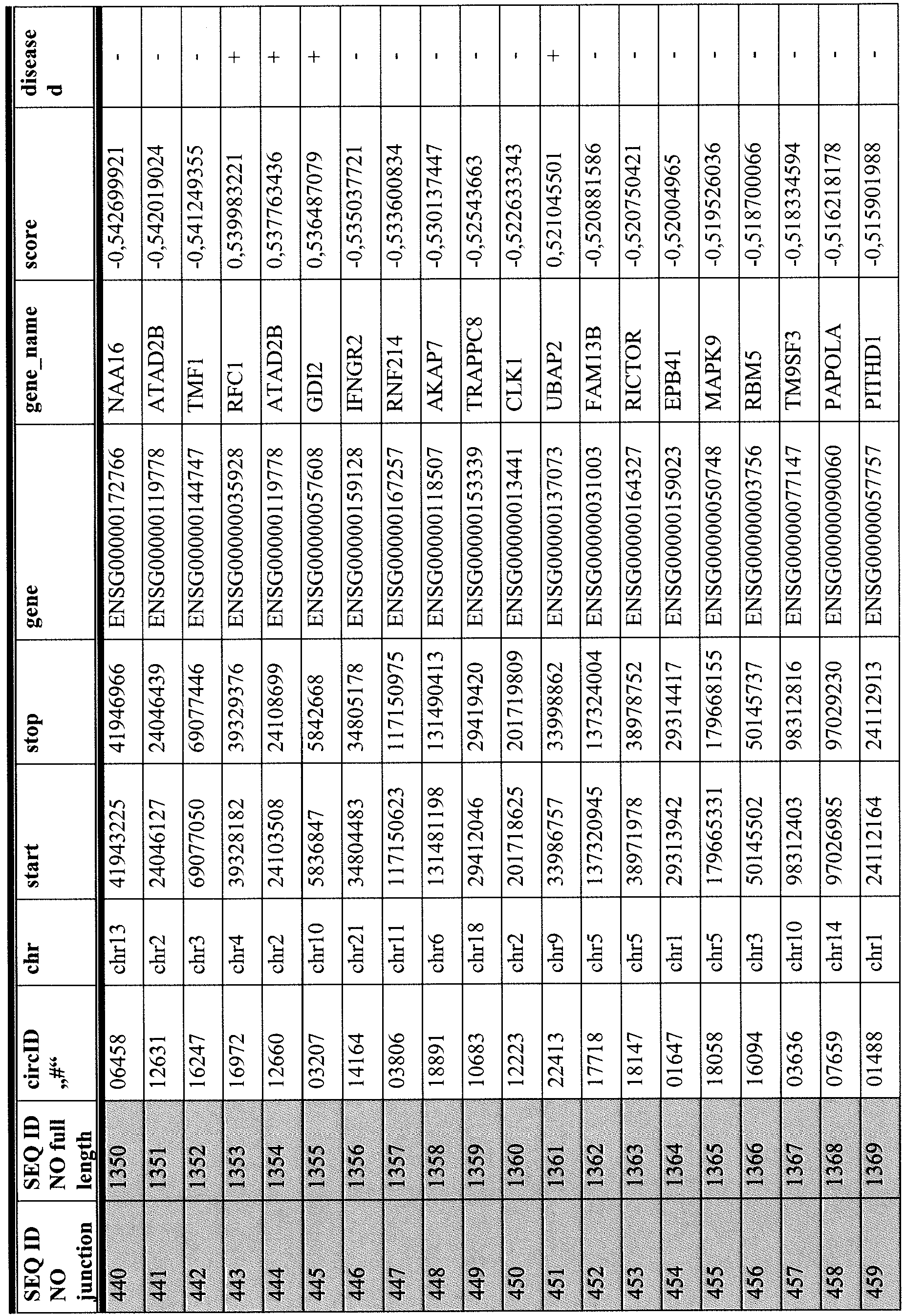

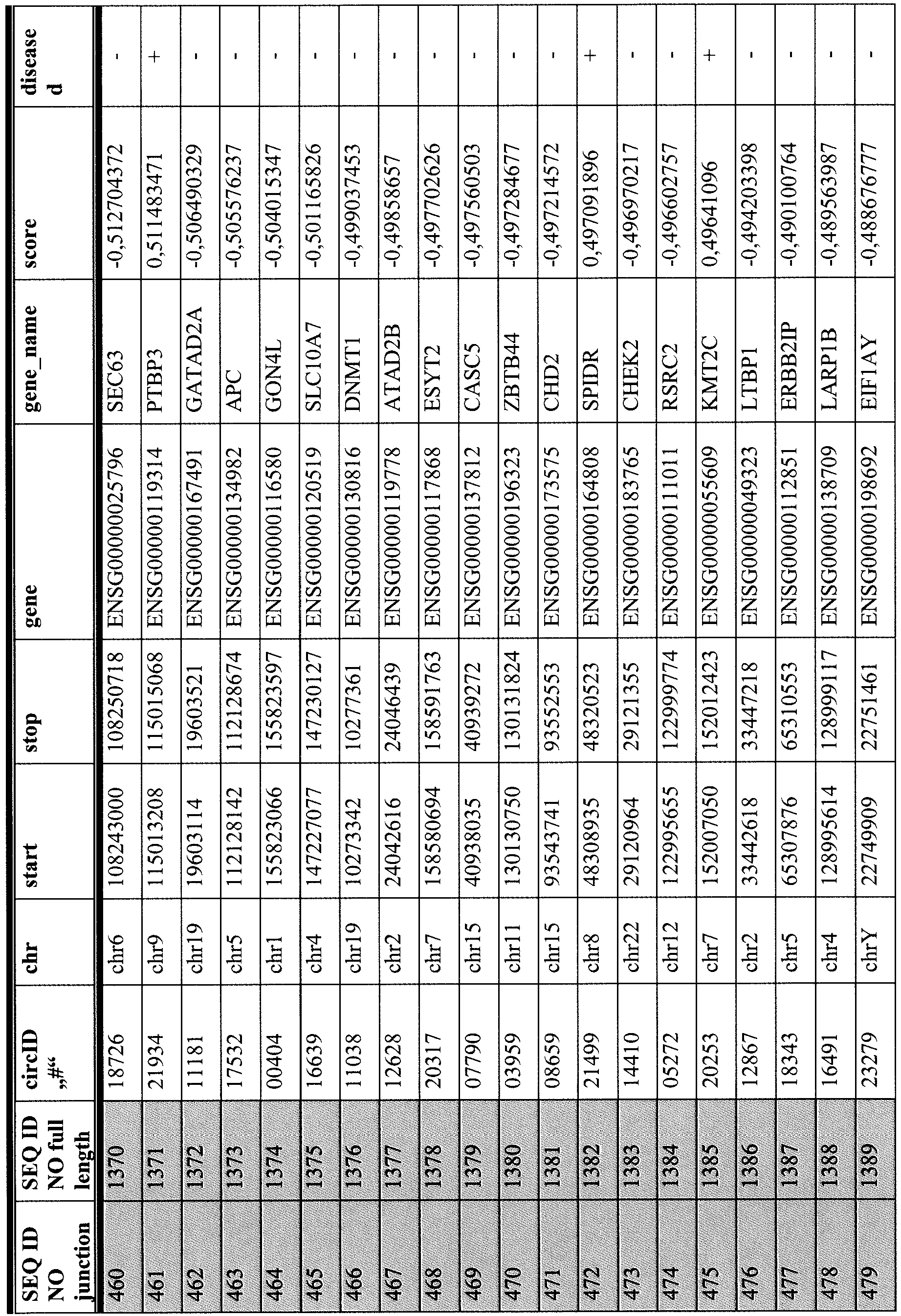

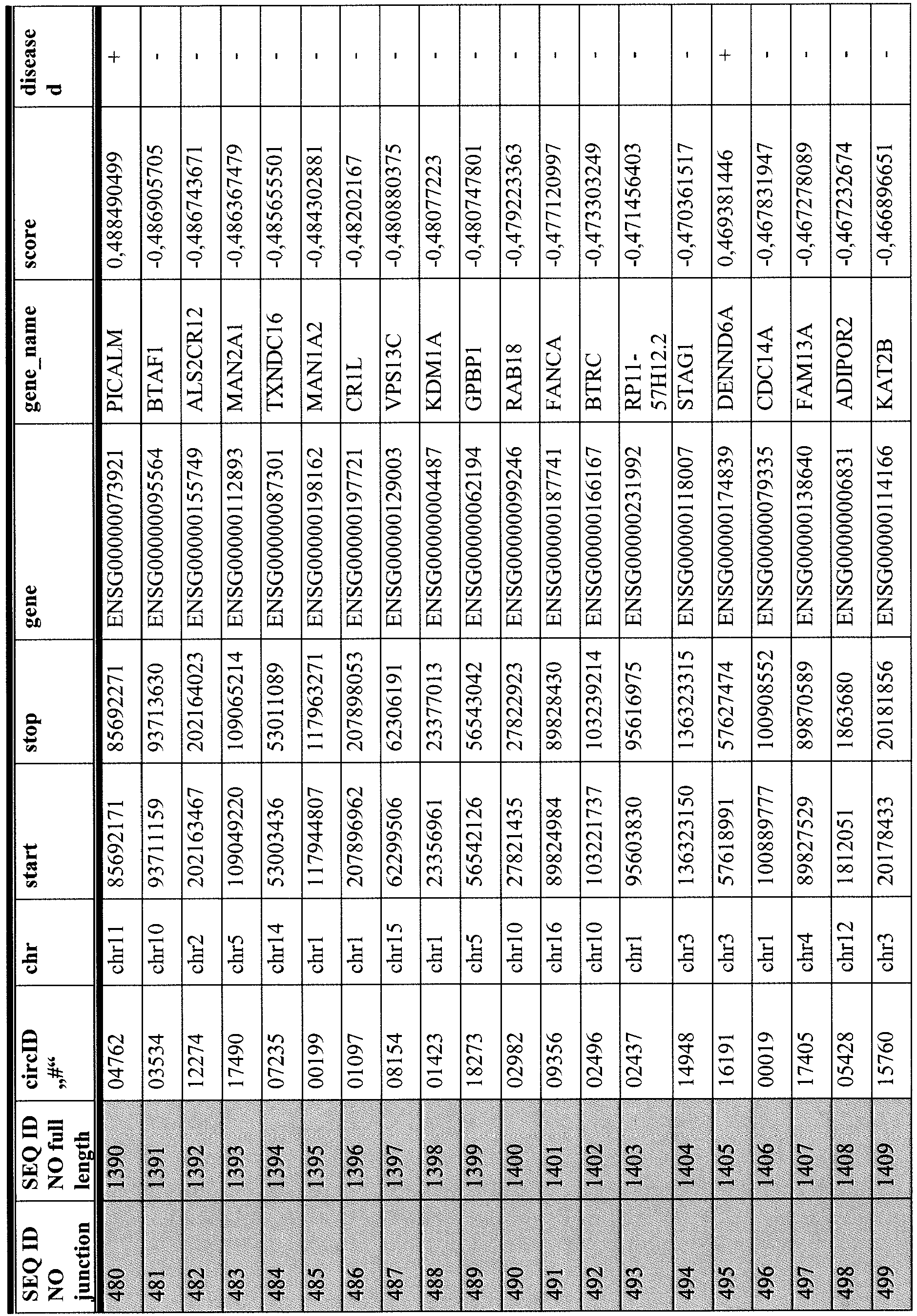

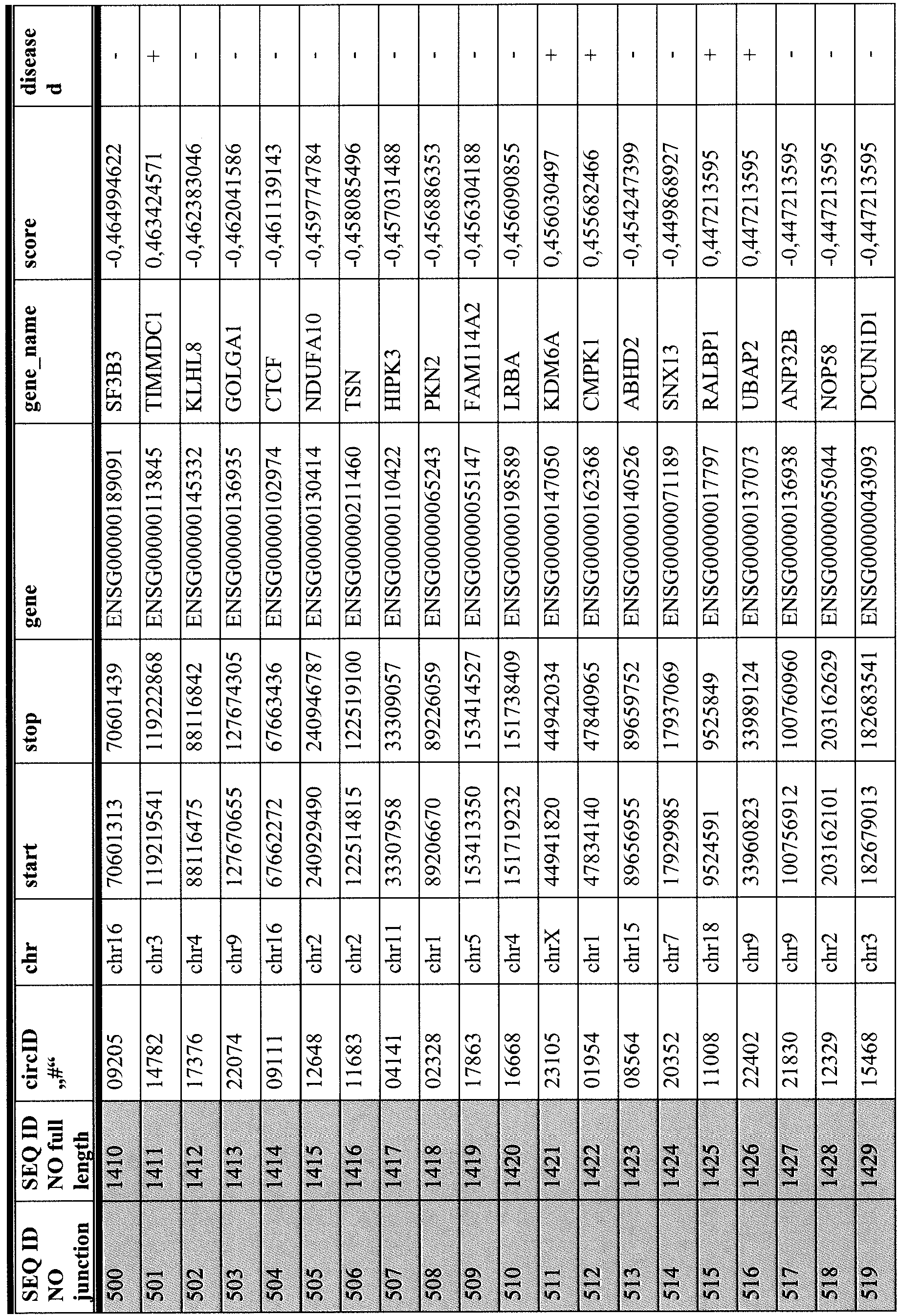

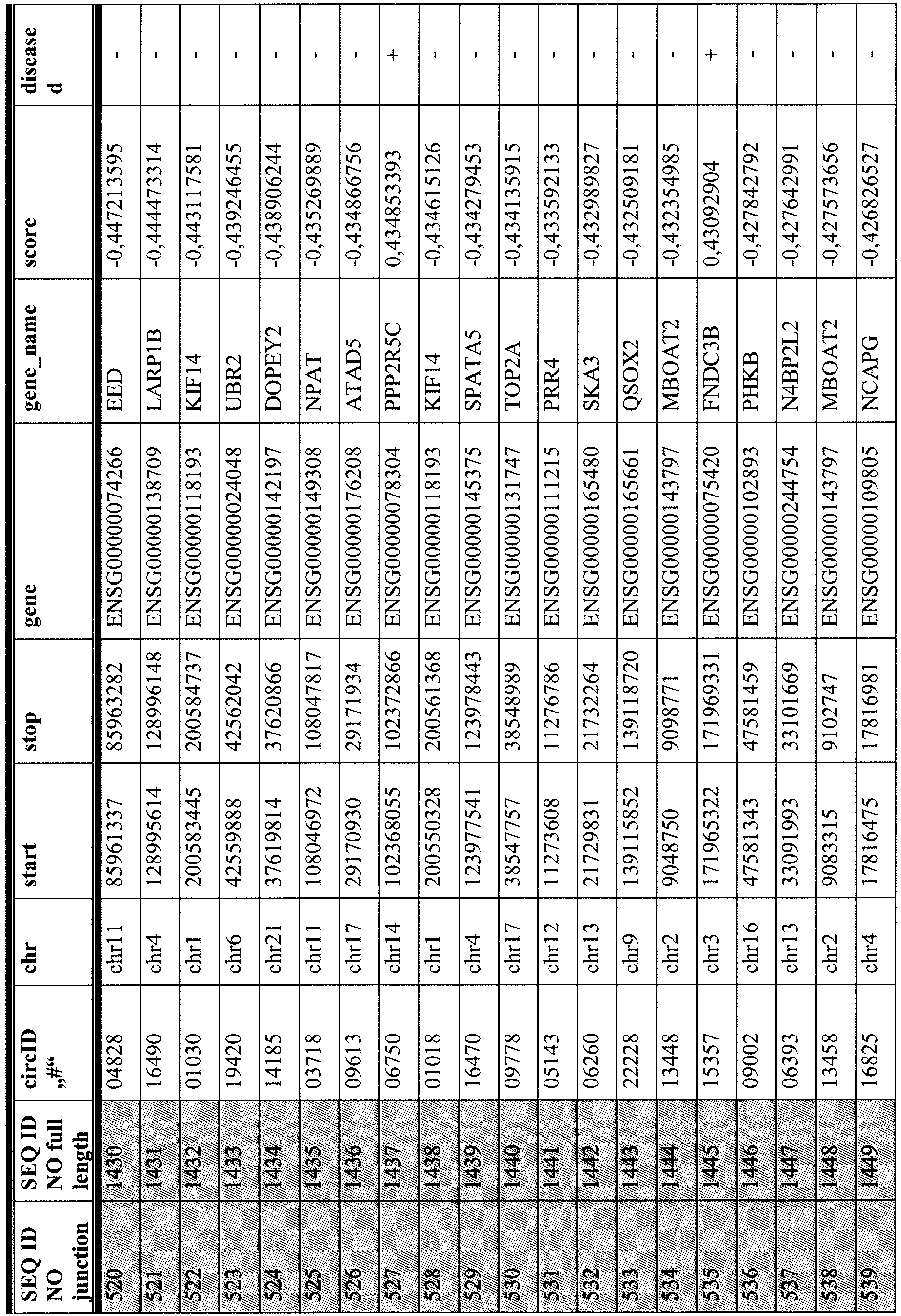

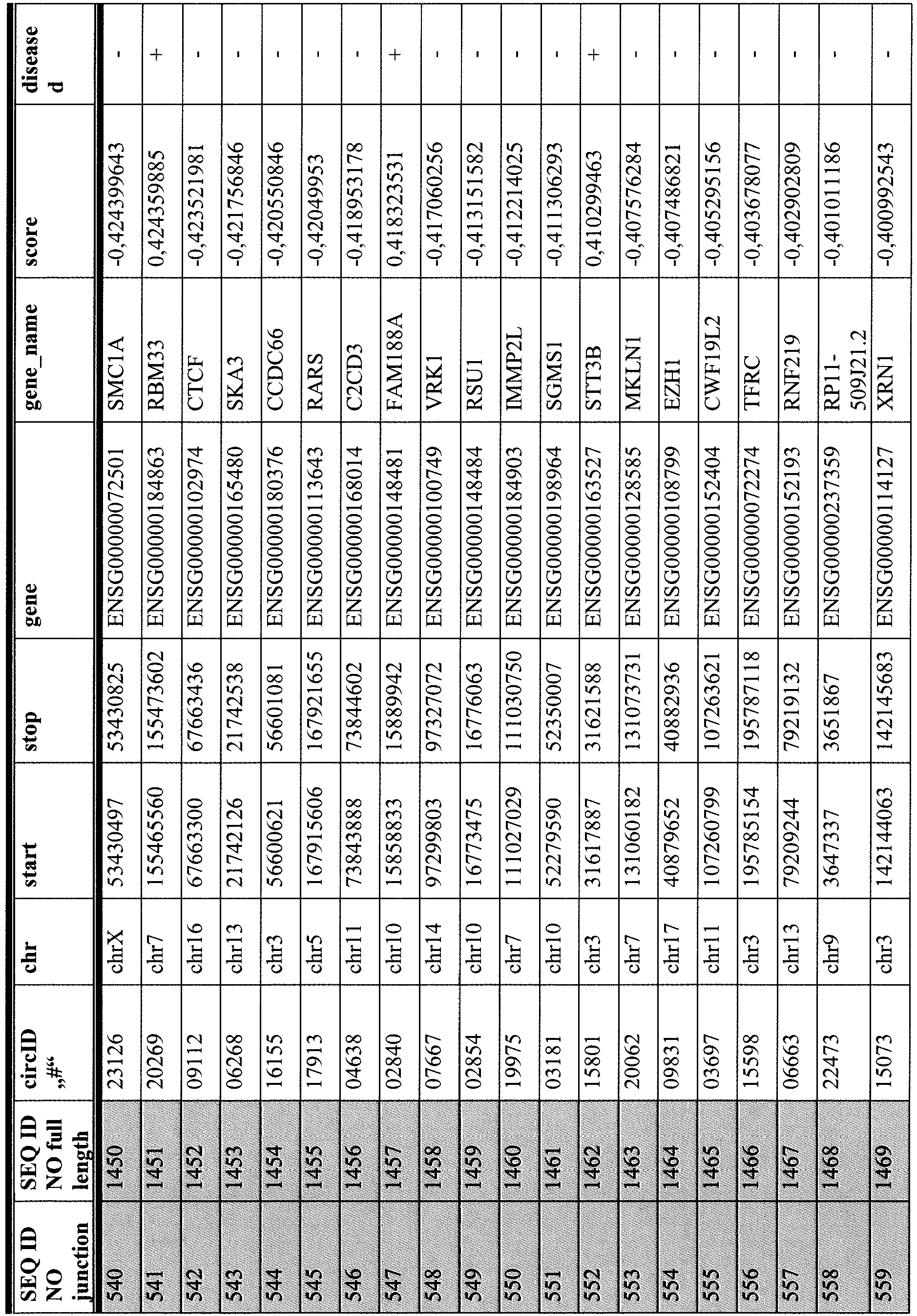

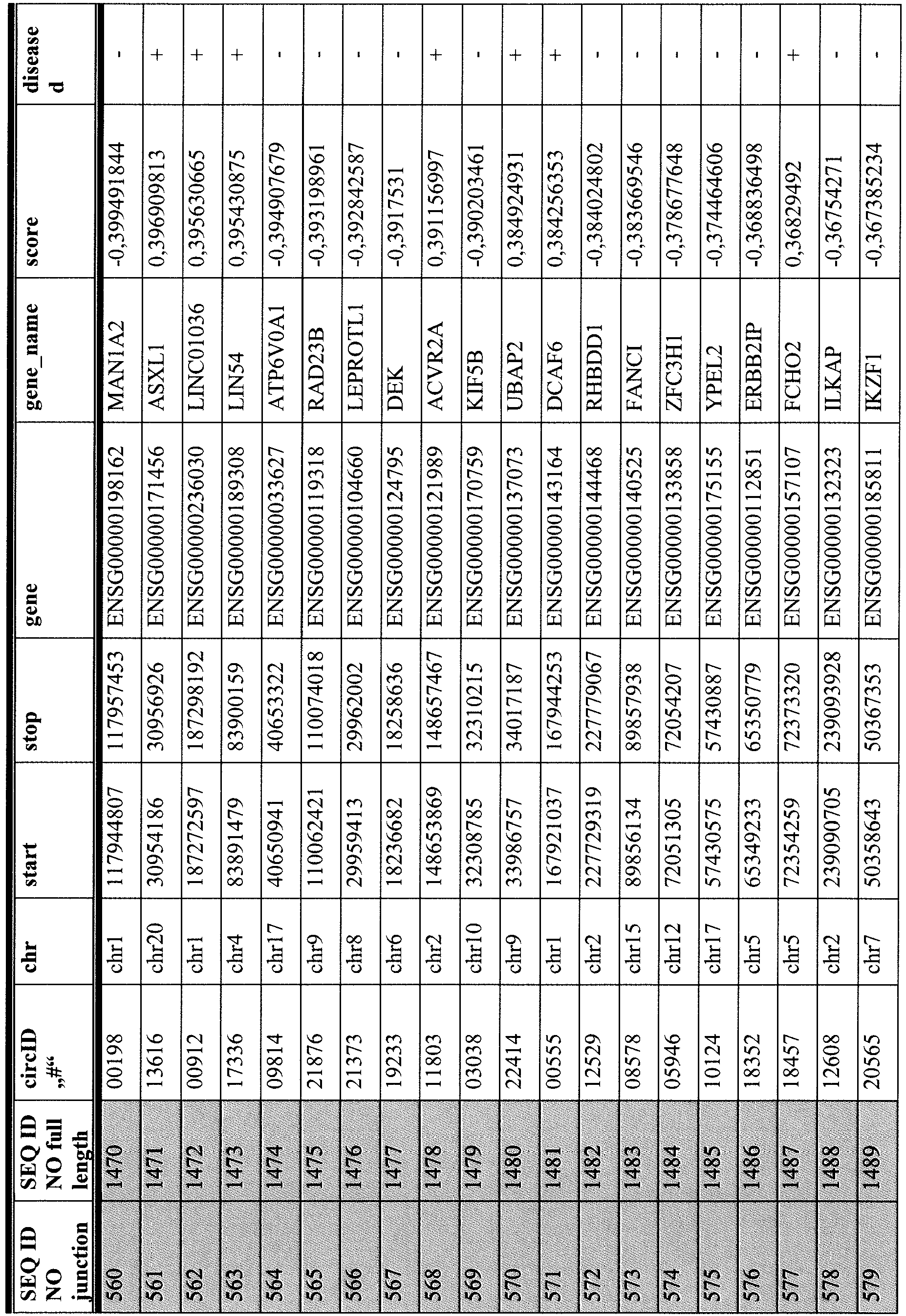

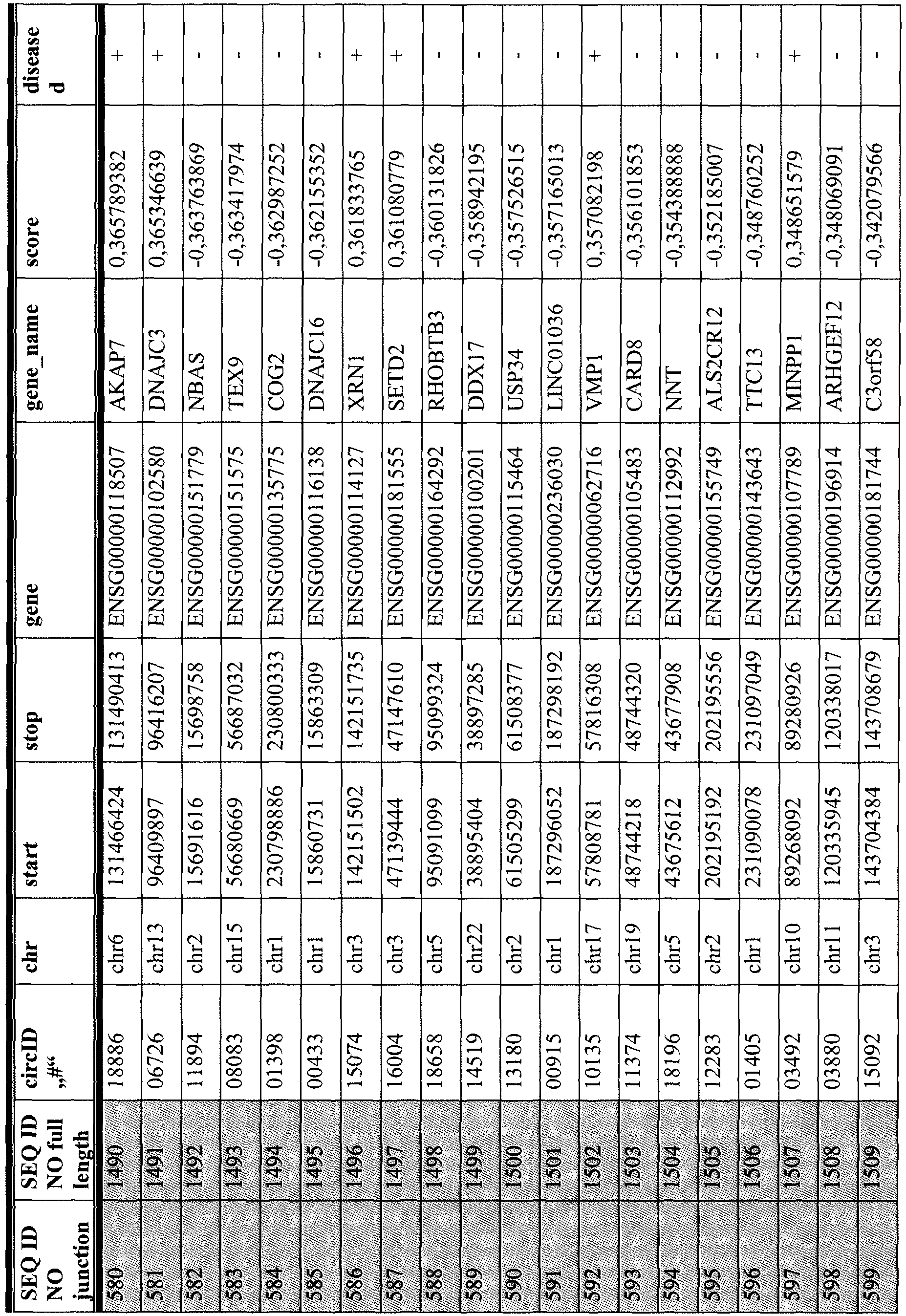

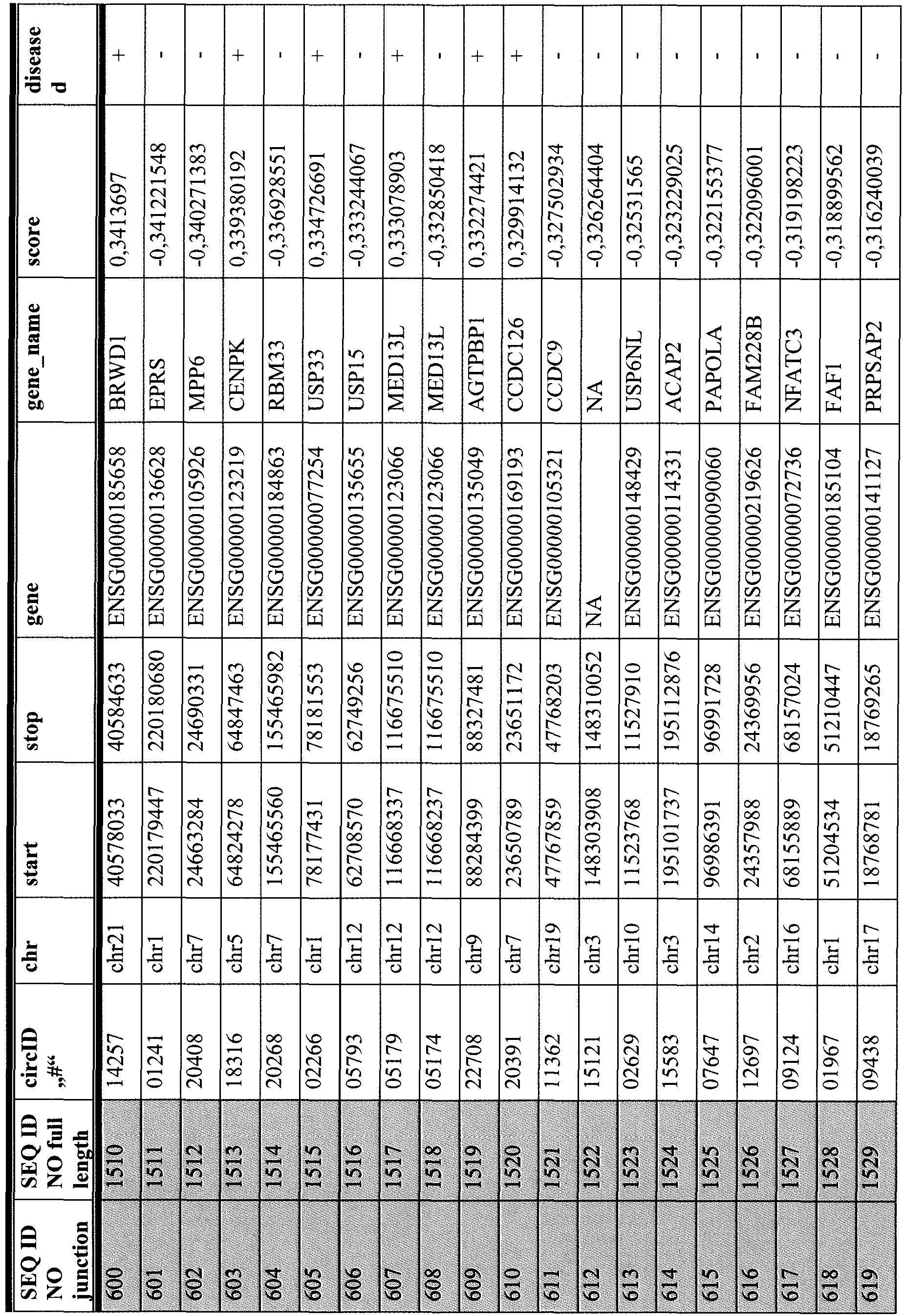

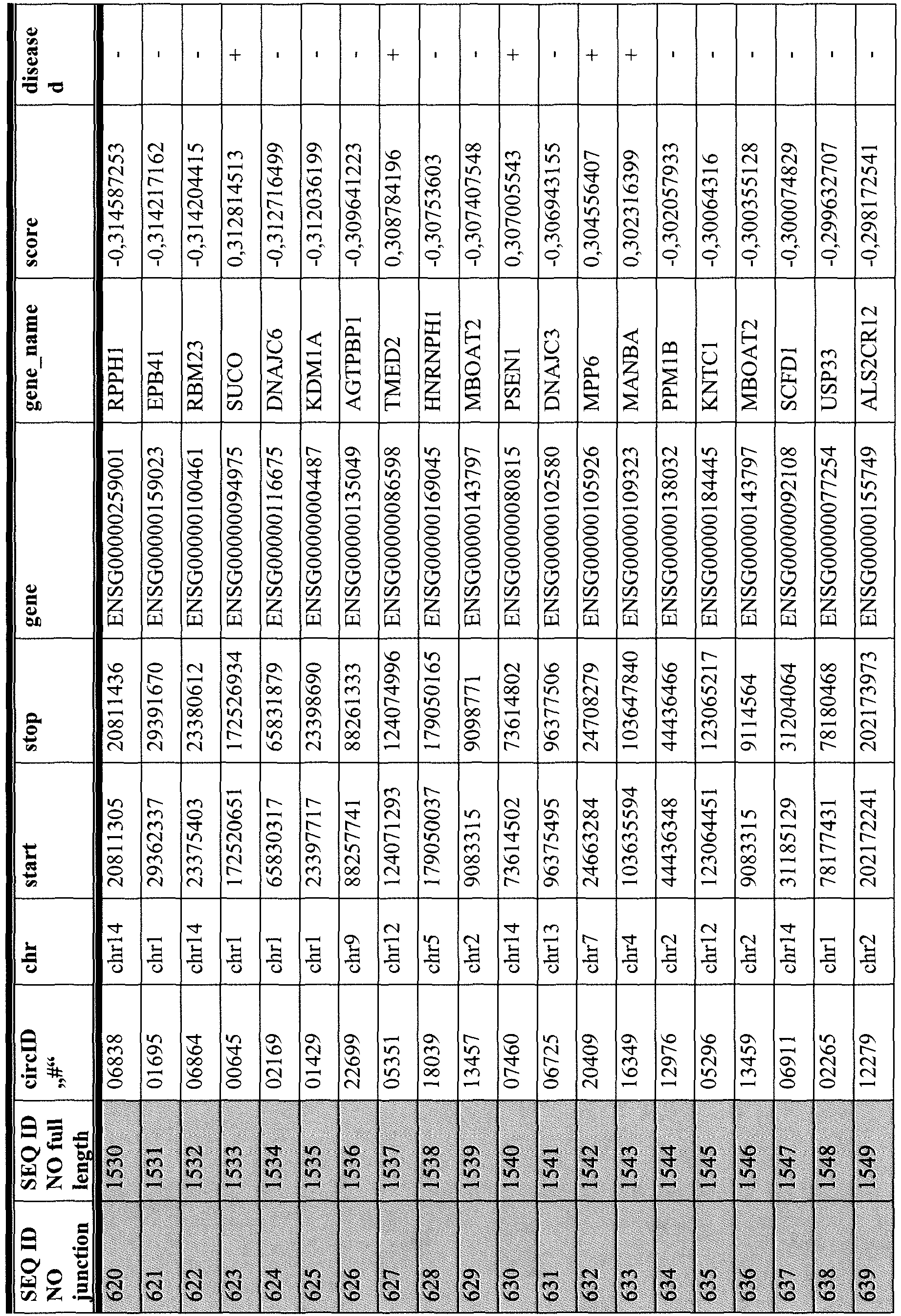

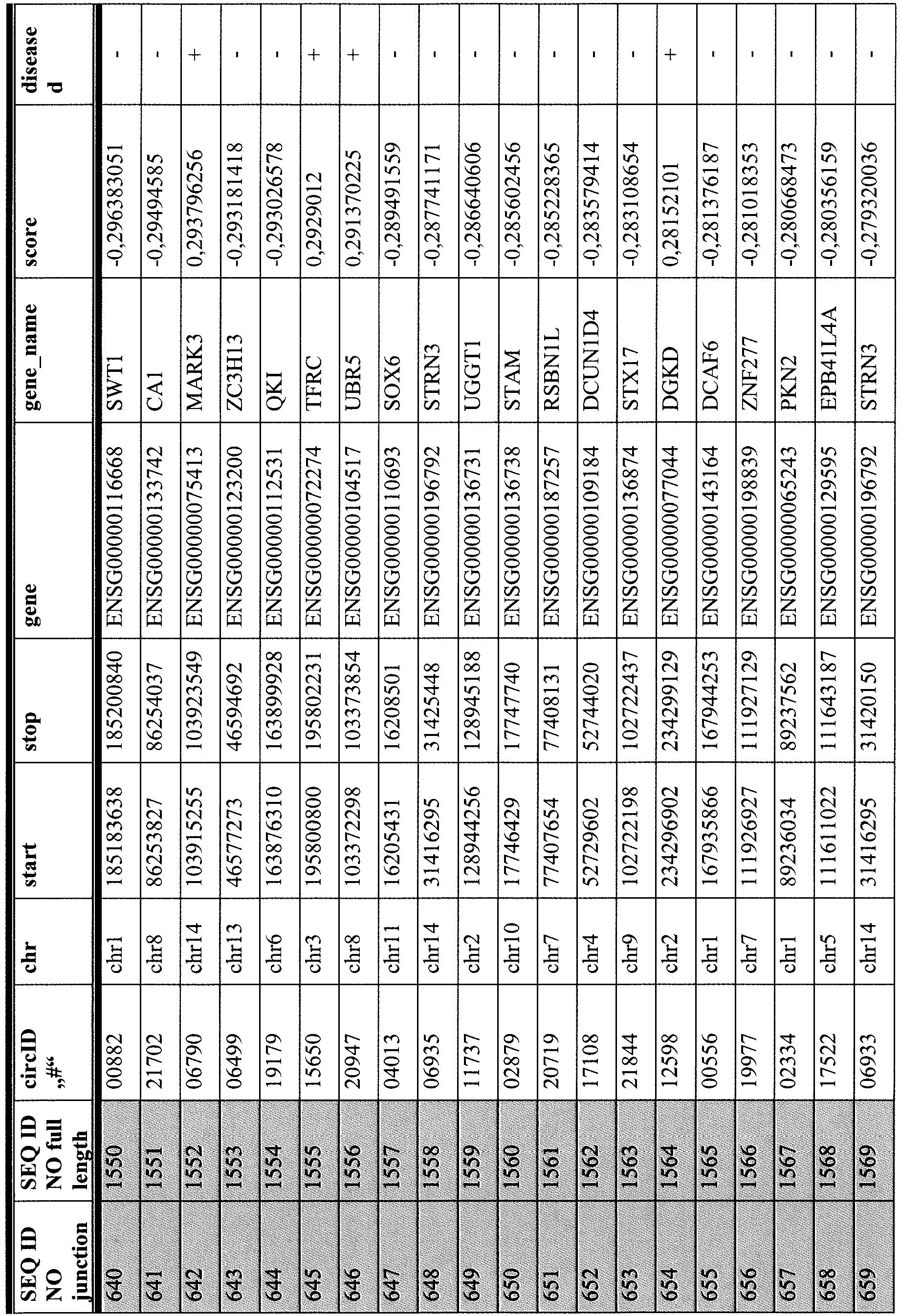

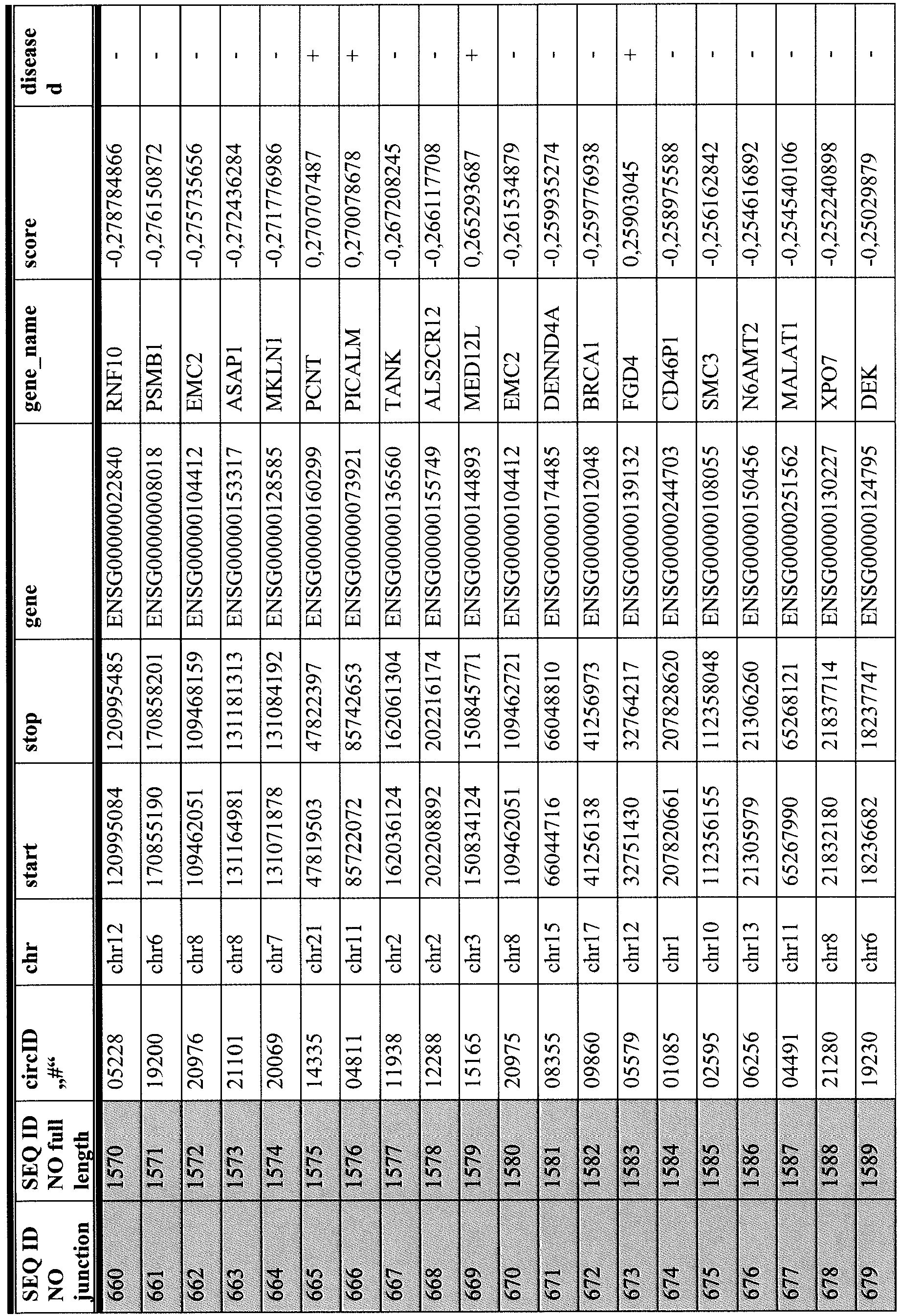

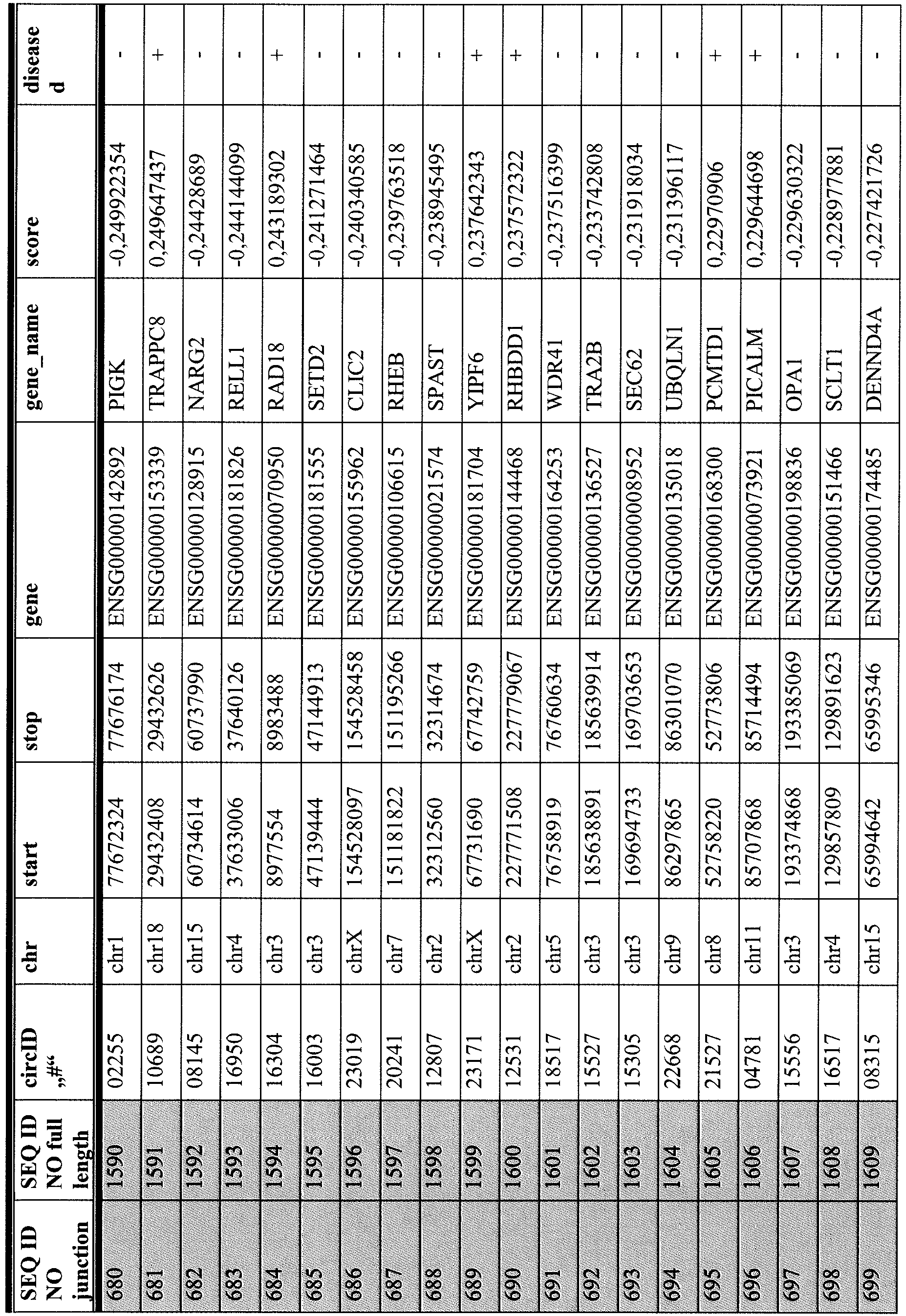

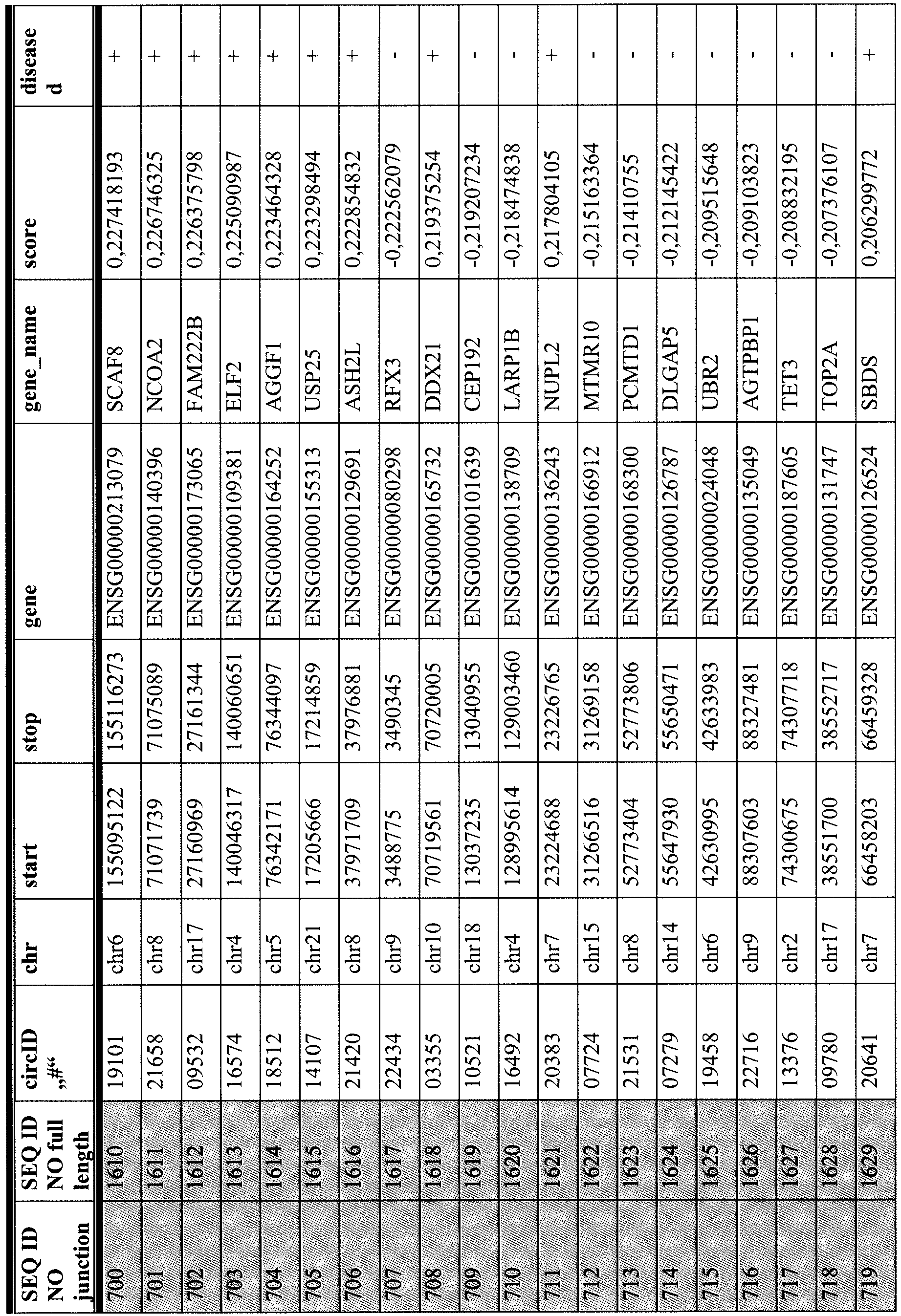

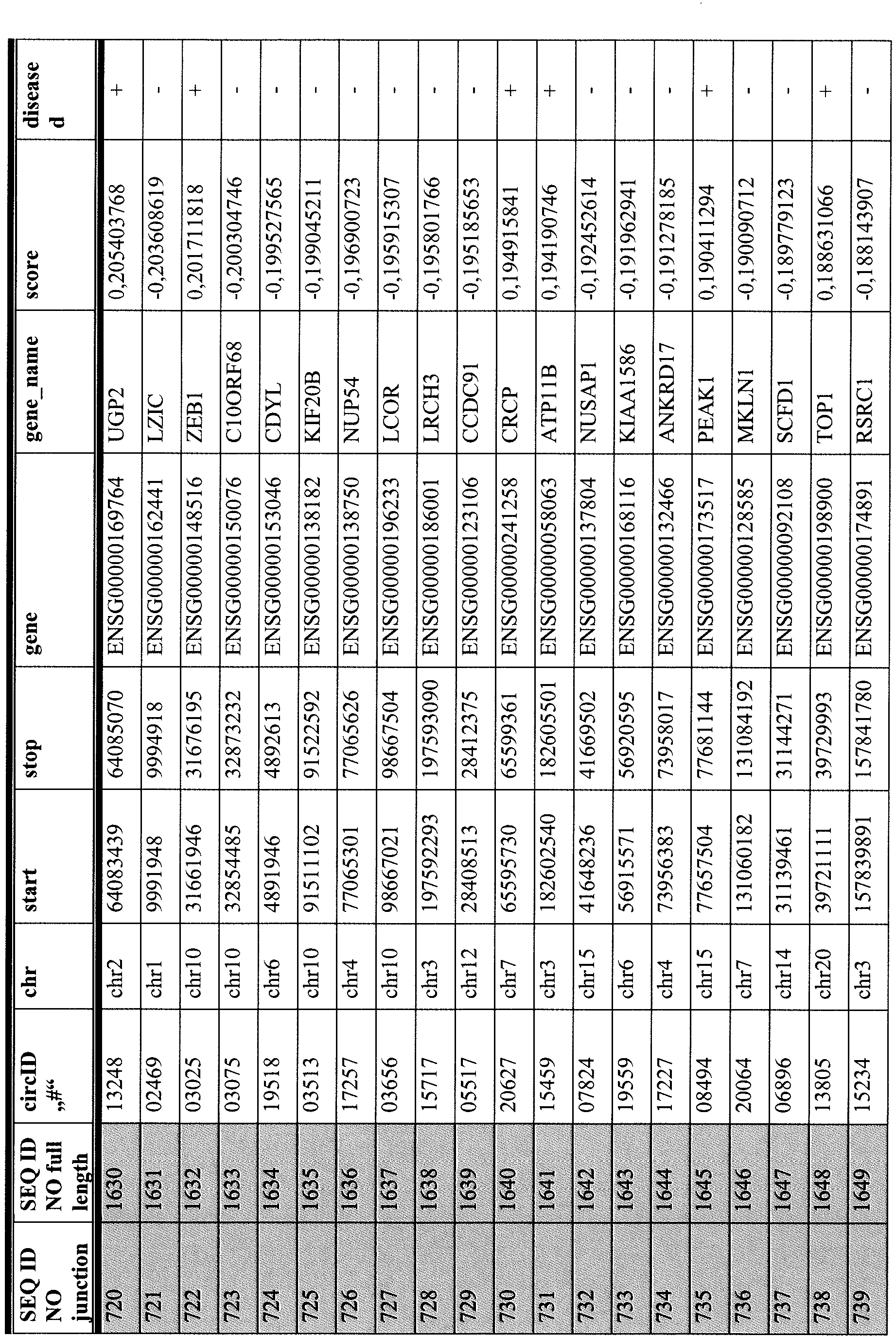

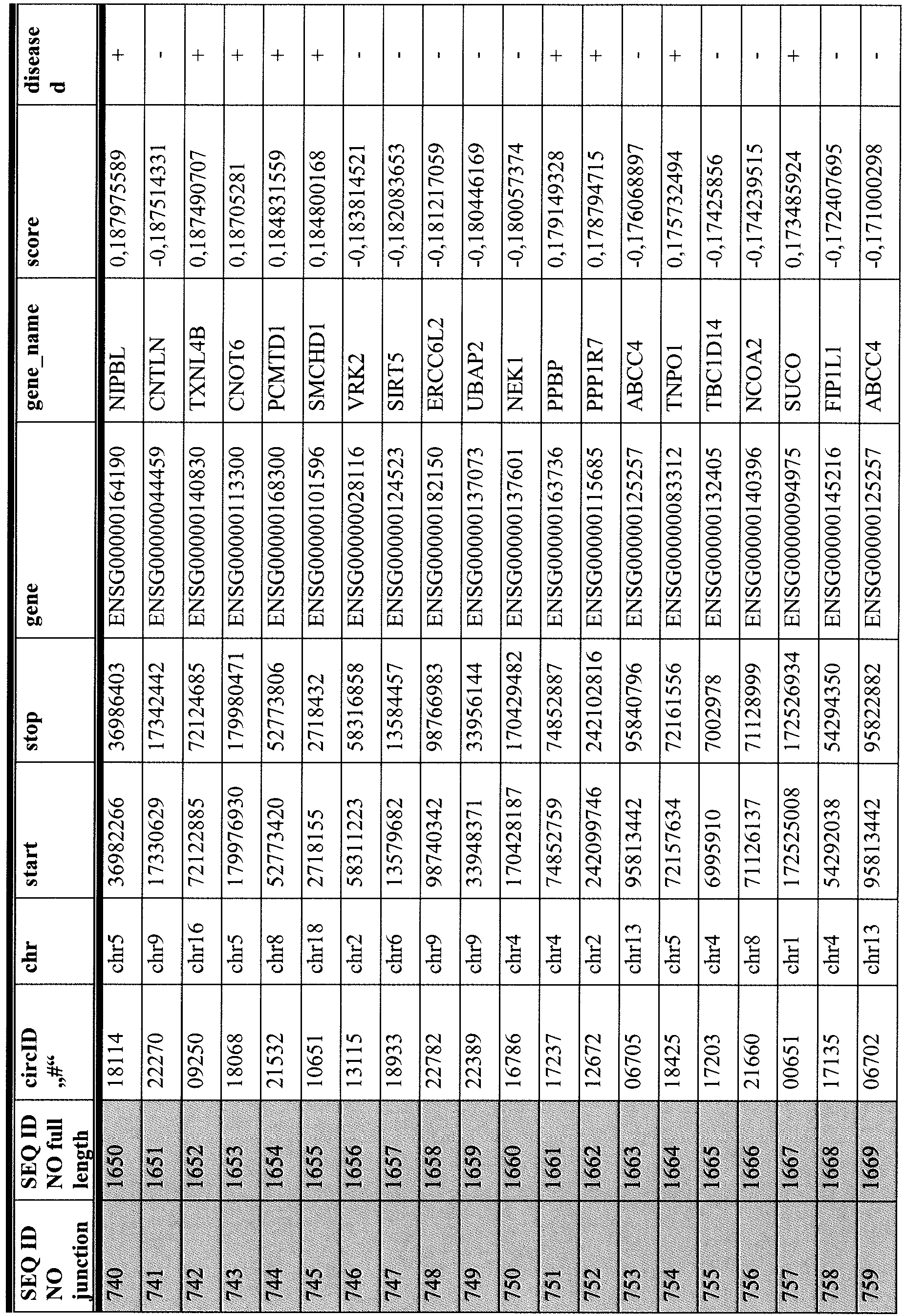

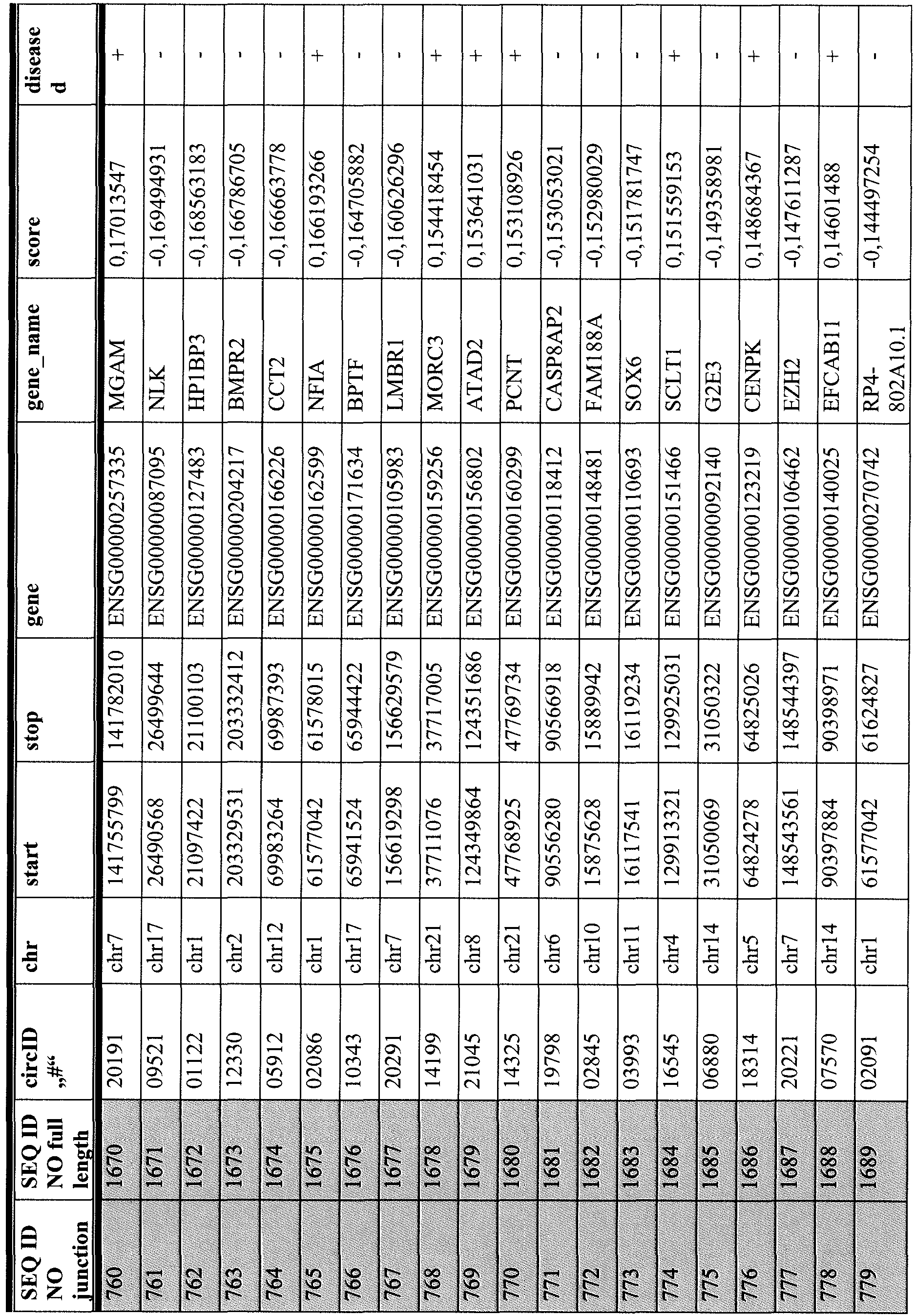

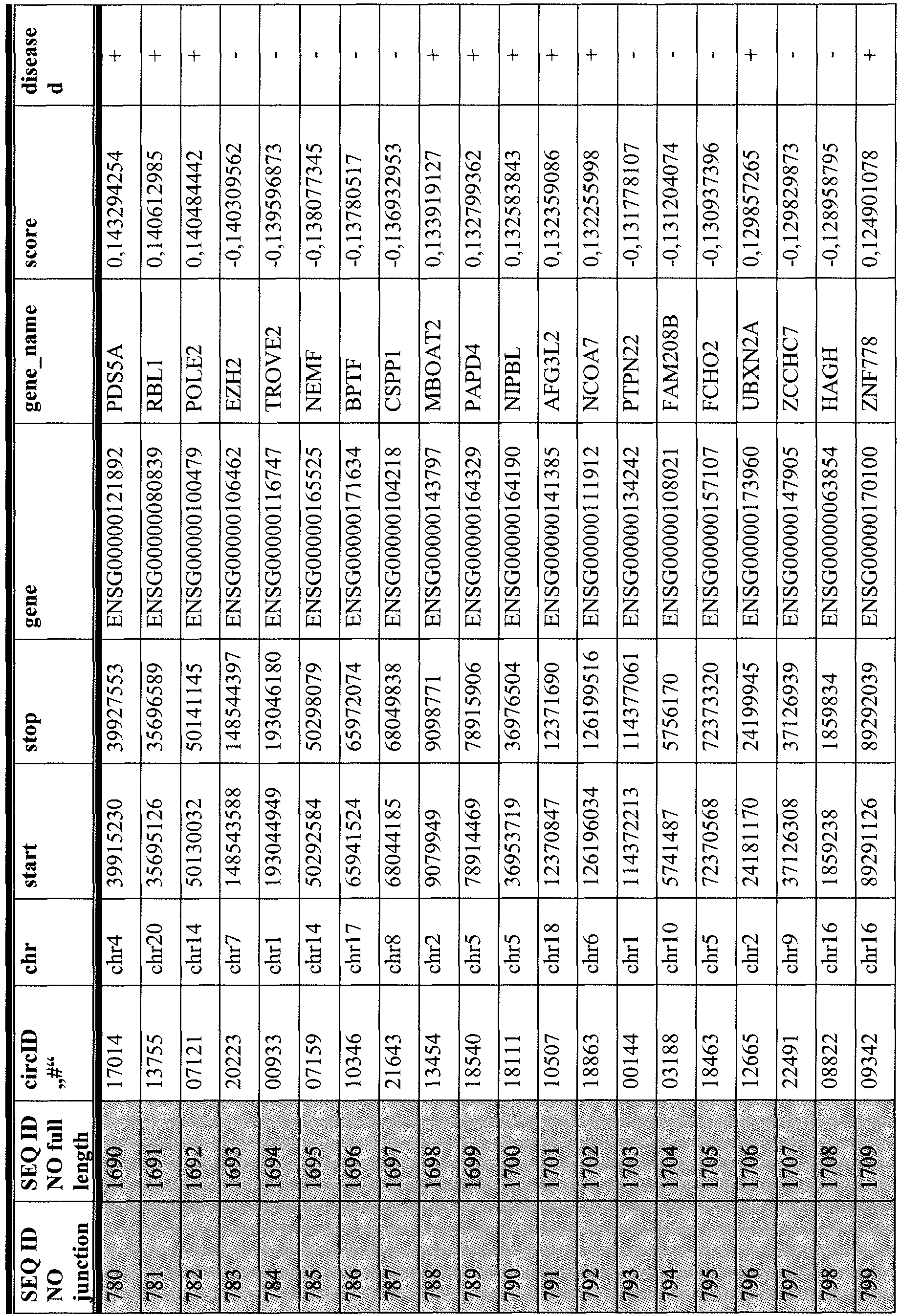

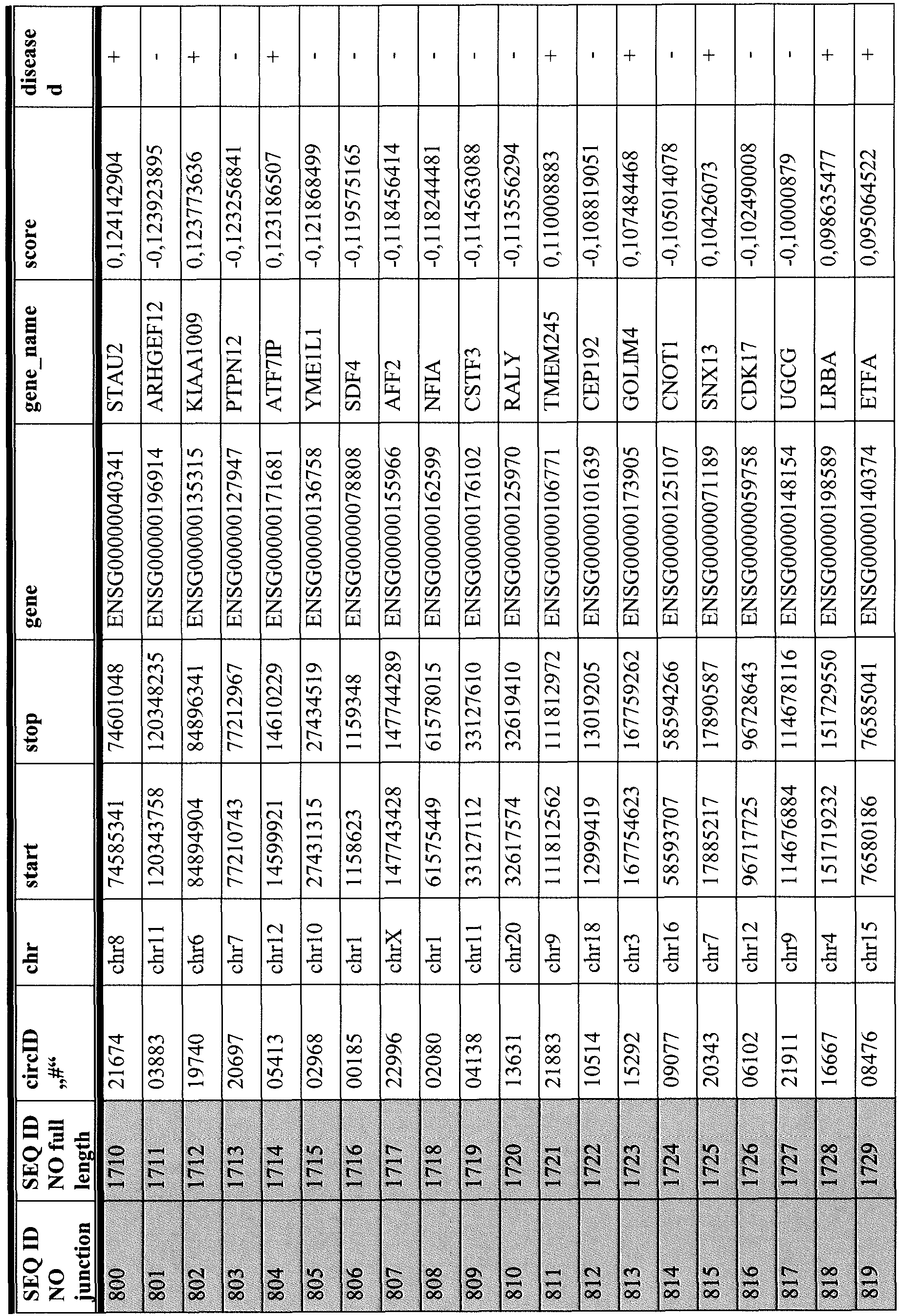

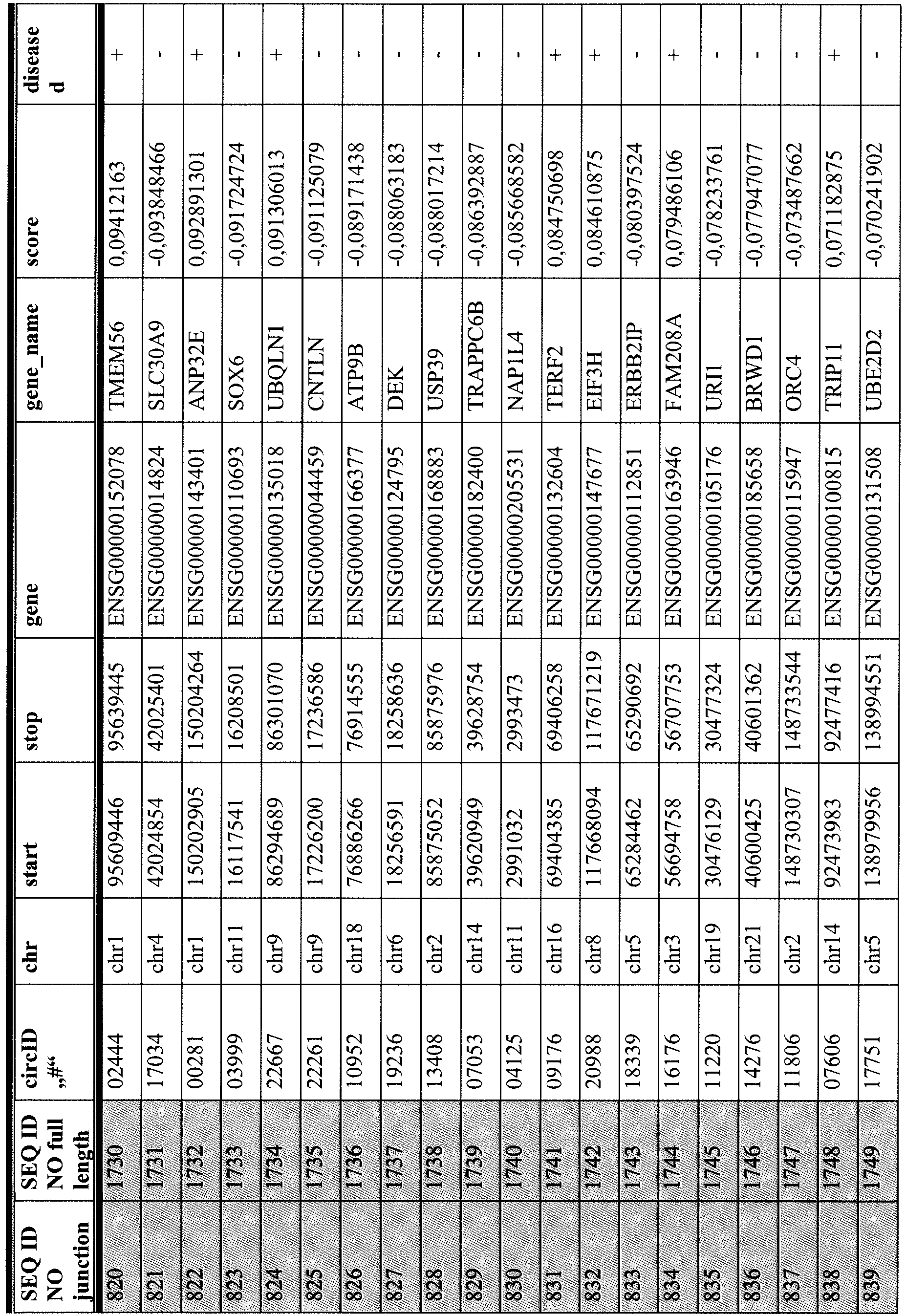

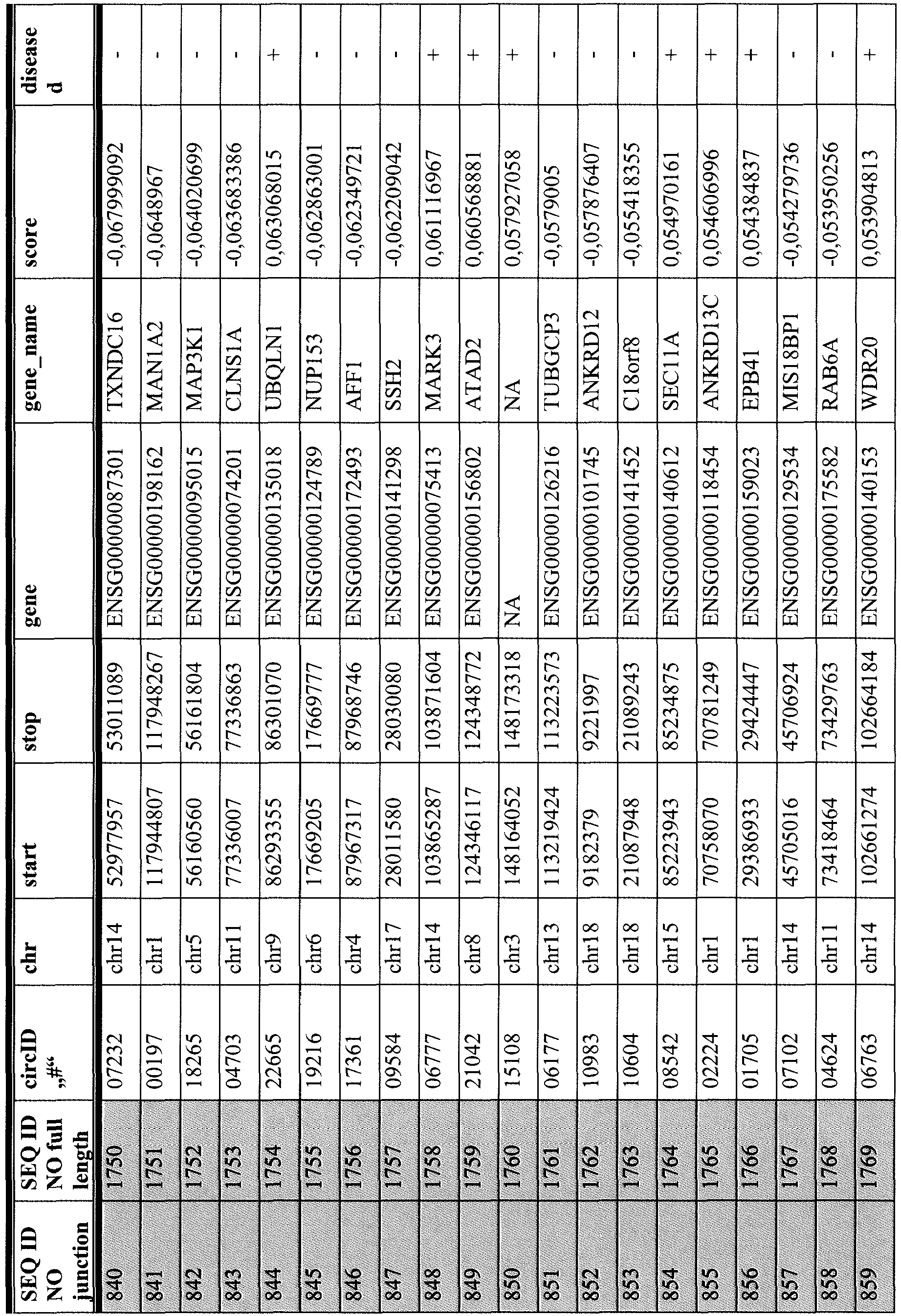

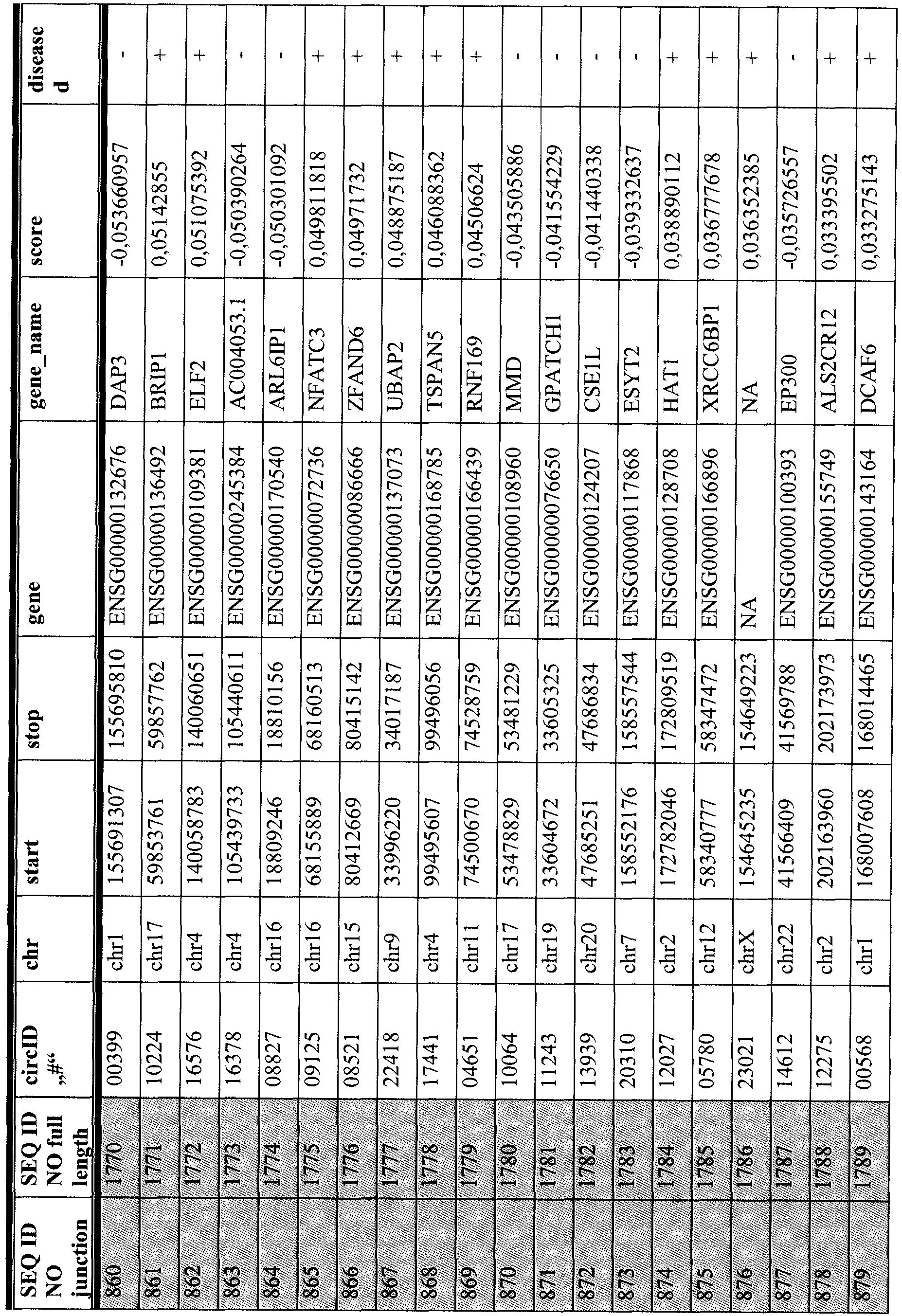

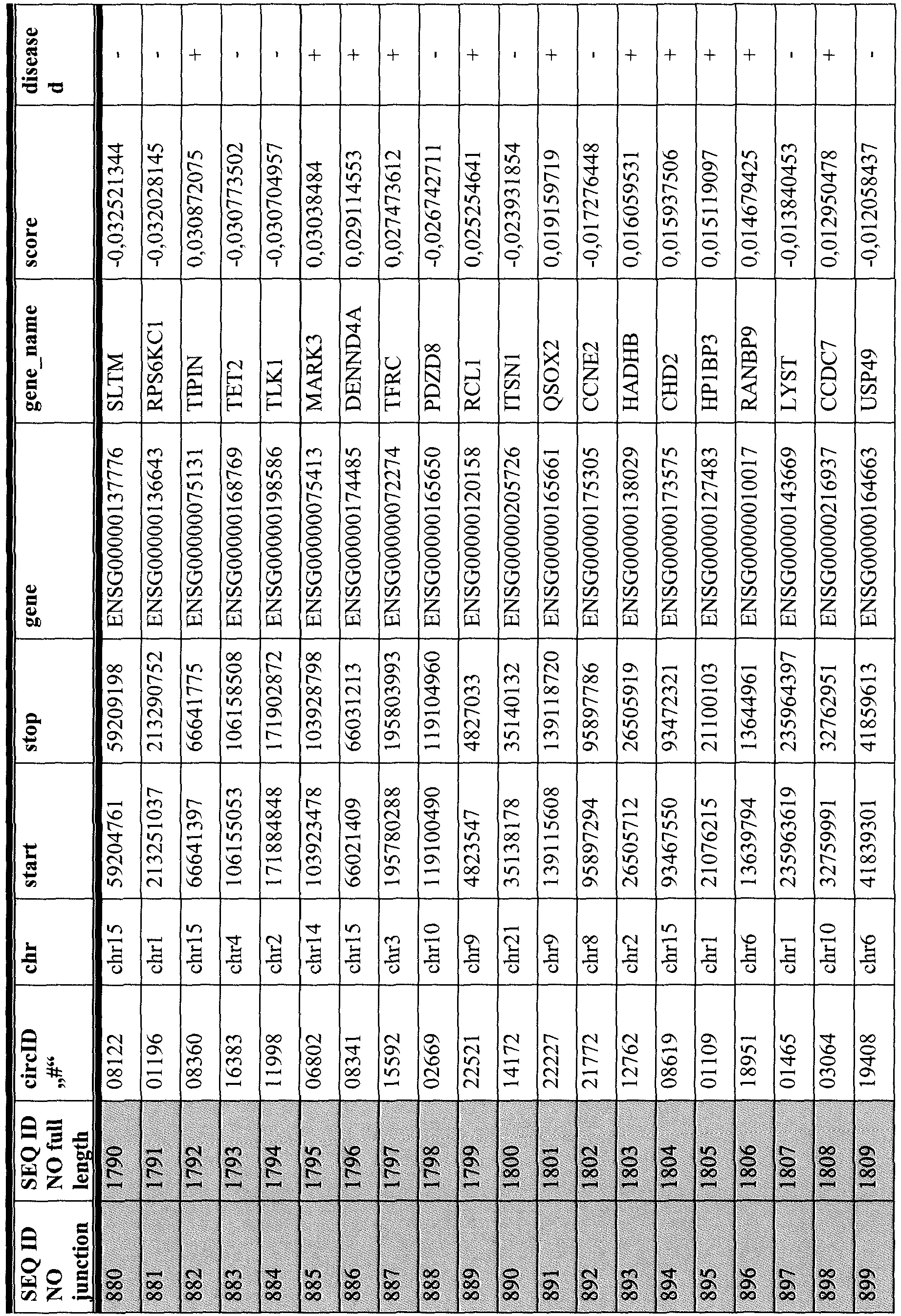

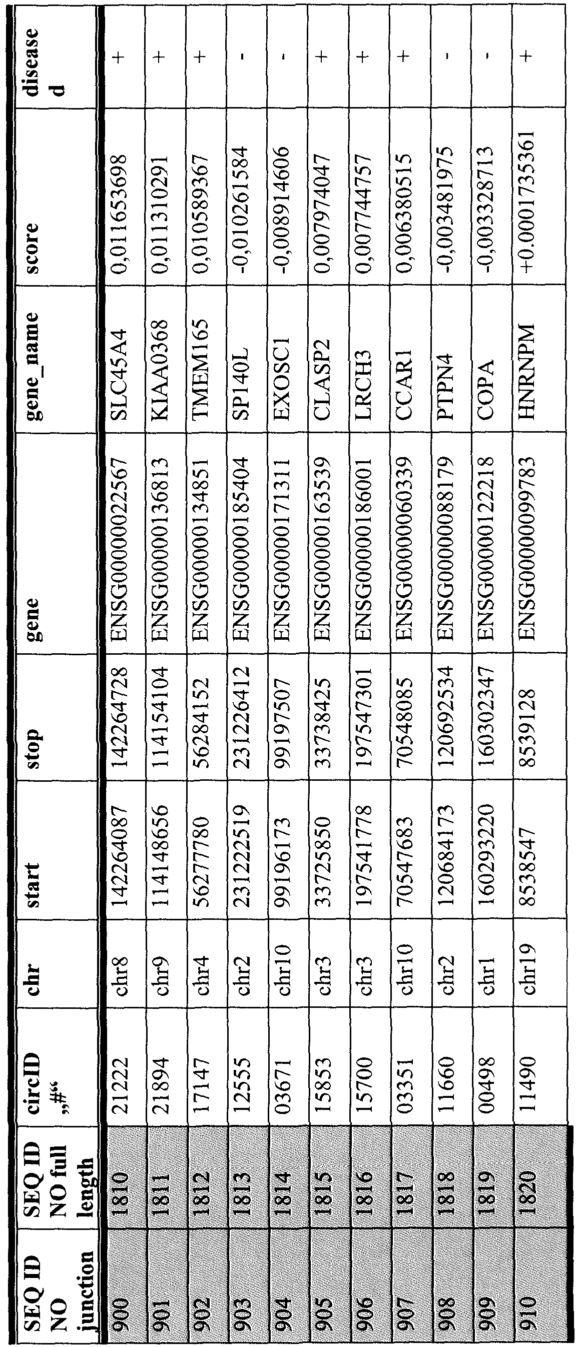

As outlined herein, it may be desirable to determine the presence or absence, or the level of more than on circRNA in order to increase the diagnostic significance of the method according to the present invention. Hence, in a preferred embodiment of the method for diagnosing a AD the levels of more than one circRNA comprising a sequence encoded by a sequence selected from the group consisting of SEQ ID NO:1 to SEQ ID NO:910, or a sequence having at least 70 % identity thereto, are determined and compared to the respective control level. The identity is preferably at least 80 %, more preferably at least 90 %, more preferably at least 95 %. In a preferred embodiment the levels of at least 100 circRNAs comprising a sequence encoded by a sequence selected from the group consisting of SEQ ID NO:1 to SEQ ID NO:910, or a sequence having at least 70 % identity thereto, are determined in a sample of a bodily fluid of said subject and controlled to the respective control level; preferably the levels of at least 150 circRNAs and more preferably the levels of at least 200 circRNAs comprising a sequence encoded by a sequence selected from the group consisting of nt 11 to 30 of any one of SEQ ID NO:1 to SEQ ID NO:910, or a sequence having at least 70 % identity thereto, are determined in a sample of a bodily fluid of said subject and controlled to the respective control level. The identity is preferably at least 80 %, more preferably at least 90 %, more preferably at least 95 % to the respective sequences in the SEQ ID NO:.

In one embodiment the circRNAs comprising a sequence encoded by SEQ ID NOs:1 to 910 have the sequence as determined by the inventors, i.e. have a sequence encoded by the sequence of any of SEQ ID NOs: 911 to 1820. Hence, in one embodiment of the method for diagnosing a neurodegenerative disease the levels of more than one circRNAs having a sequence being at least 70 % identical to any of the sequences as encoded by SEQ ID NO: 911 to 1820 are determined in a sample of a bodily fluid of said subject and controlled to the respective control level. Particularly preferred the levels of at least 100 circRNAs having a sequence being at least 70 % identical to any of the sequences as encoded by SEQ ID NOs: 911 to 1820 are determined in a sample of a bodily fluid of said subject and controlled to the respective control level, preferably the levels of at least 150, and more preferably the levels of at least 200 circRNAs having a sequence being at least 70 % identical to any of the sequences as encoded by SEQ ID NOs: 911 to 1820 are determined in a sample of a bodily fluid of said subject and controlled to the respective control level. In more preferred embodiments said sequence identity is at least 80 %, preferably at least 90 %, more preferably at least 95 %, yet more preferably at least 99 % to the outlined sequences. In a further preferred embodiment the circRNAs have the sequences as encoded by any one of SEQ ID NOs: 911 to 1820. The levels are preferably detected by using hybridization probes specifically hybridizing the sequences of nt 11 to 30 of SEQ ID NO:1 to 910 or specifically hybridizing to the sequences of SEQ ID NO:1 to 910, or an RNA sequence encoded by these sequences, or the respective reverse complements thereof.

The inventors found that the first 200 circRNAs as encoded by SEQ ID NO:1 to 910 have particular suited predictive and diagnostic values. Hence, in a preferred embodiment of the method for diagnosing a neurodegenerative disease said at least 100, preferably at least 150 more preferably at least 200 circRNAs comprise a sequence as encoded by any of SEQ ID NO:1 to SEQ ID NO:200, or a sequence having at least 70 % identity thereto, or the circRNAs have a sequence as encoded by any of SEQ ID NO: 911 to 1110, or a sequence having at least 70 % identity thereto. The identity is preferably at least 80 %, more preferably at least 90 %, more preferably at least 95 %. In a preferred embodiment of the method for diagnosing a neurodegenerative disease the levels of more than one circRNA comprising a sequence encoded by a sequence being at least 70 % identical to a sequence selected from the group consisting of the sequence of nt 11 to nt 30 of any one of SEQ ID NO: 1 to 200 are determined in a sample of a bodily fluid of said subject and controlled to the respective control level. Particularly preferred the levels of at least 100 circRNAs comprising a sequence encoded by a sequence being at least 70 % identical to a sequence selected from the group consisting of the sequence of nt 11 to nt 30 of any one of SEQ ID NO: 1 to 200 are determined in a sample of a bodily fluid of said subject and controlled to the respective control level, preferably of at least 150 and more preferably the levels of all 200 circRNAs comprising a sequence encoded by a sequence being at least 70 % identical to a sequence selected from the group consisting of the sequence of nt 11 to nt 30 of any one of SEQ ID NO: 1 to 200 are determined in a sample of a bodily fluid of said subject and controlled to the respective control level. The identity is preferably at least 80 %, more preferably at least 90 %, more preferably at least 95 %, yet more preferred 100 %. The levels are preferably detected by using hybridization probes specifically hybridizing the sequences of nt 11 to 30 of SEQ ID NO:1 to 200 or specifically hybridizing to the sequences of SEQ ID NO:1 to 200, or an RNA sequence encoded by these sequences, or the reverse complements thereof.

In one embodiment the circRNAs comprising a sequence encoded by any of the SEQ ID NOs:1 to 200 have the sequence as determined by the inventors, i.e. a sequence encoded by any of the SEQ ID NOs: 911 to 1110. Hence, in one embodiment of the method for diagnosing a neurodegenerative disease the levels of more than one circRNA having a sequence encoded by a sequence being at least 70 % identical to any of the sequences of SEQ ID NO: 911 to 1110 are determined in a sample of a bodily fluid of said subject and controlled to the respective control level. Particularly preferred the levels of at least 100 circRNAs having a sequence encoded by a sequence being at least 70 % identical to any of the sequences of SEQ ID NOs: 911 to 1110 are determined in a sample of a bodily fluid of said subject and controlled to the respective control level, preferably the levels of at least 150, and more preferably the levels of at least 200 circRNAs having a sequence encoded by a sequence being at least 70 % identical to any of the sequences of SEQ ID NOs: 911 to 1110 are determined in a sample of a bodily fluid of said subject and controlled to the respective control level. In more preferred embodiments said sequence identity is at least 80 %, preferably at least 90 %, more preferably at least 95 %, yet more preferably at least 99 % to the outlined sequences. In a further preferred embodiment the circRNAs have the sequences encoded by the sequences as set out in any one of SEQ ID NOs: 911 to 1110. The levels are preferably detected by using hybridization probes specifically hybridizing the sequences of nt 11 to 30 of SEQ ID NO:1 to 200 or specifically hybridizing to the sequences of SEQ ID NO:1 to 200, or an RNA sequence encoded by these sequences, or the reverse complements thereof.

The determination of percent identity between two sequences is accomplished using the mathematical algorithm of Karlin and Altschul (1993) Proc. Natl. Acad. Sci. USA 90: 5873-5877. Such an algorithm is incorporated into the BLASTN and BLASTP programs of Altschul et al. (1990) J. Mol. Biol. 215: 403-410. BLAST nucleotide searches are performed with the BLASTN program, score = 100, word length = 12, to obtain nucleotide sequences homologous to the nucleic acid sequences outlined herein. BLAST protein searches are performed with the BLASTP program, score = 50, wordlength = 3, to obtain amino acid sequences homologous to the EPO variant polypeptide, respectively. To obtain gapped alignments for comparative purposes, Gapped BLAST is utilized as described in Altschul et al. (1997) Nucleic Acids Res. 25: 3389-3402. When utilizing BLAST and Gapped BLAST programs, the default parameters of the respective programs are used.

In order to improve the diagnostic value, it may be desirable to determine the number of circRNAs showing increased or decreased levels being indicative for the neurodegenerative disease as outlined in Table 1. In a preferred embodiment the number of circRNAs showing increased or decreased levels being indicative for the neurodegenerative disease as outlined in Table 1 is above the 80% percentile of a control population, more preferably above the 90% percentile, yet more preferred above the 95% percentile. In a preferred embodiment of the method for diagnosing a neurodegenerative disease the presence of increased or decreased levels as defined in Table 1 under "disease" for the respective circRNA for at least 10, preferably at least 50, more preferably at least 100 circRNAs are indicative for the presence of a neurodegenerative disease, preferably Alzheimer's disease.

In one particular embodiment of the method for diagnosing a neurodegenerative disease the levels of all circRNAs comprising a sequence encoded by a sequence selected from the group consisting of nt 11 to 30 of any of SEQ ID NO:1 to SEQ ID NO:200, or a sequence having at least 70 % identity thereto; wherein preferably the presence of increased or decreased levels as defined in Table 1 under "disease" for at least 10, preferably at least 50, more preferably at least 100 of the respective circRNAs are indicative for the presence of a neurodegenerative disease, preferably Alzheimer's disease. Preferably, said method comprises the determination of the levels of all circRNAs comprising a sequence encoded by a sequence selected from the group consisting of any of SEQ ID NO:1 to SEQ ID NO:200 or a sequence having at least 70 % identity thereto, wherein preferably the presence of increased or decreased levels as defined in Table 1 under "disease" for at least 10, preferably at least 50, more preferably at least 100 of the respective circRNAs are indicative for the presence of a neurodegenerative disease, preferably Alzheimer's disease. Further preferred, all circRNAs with a sequence encoded by a sequence having at least 70 % identity to a sequence selected from the group consisting of SEQ ID NO:911 to SEQ ID NO:1110 are detected, wherein preferably the presence of increased or decreased levels as defined in Table 1 under "disease" for at least 10, preferably at least 50, more preferably at least 100 of the respective circRNAs are indicative for the presence of a neurodegenerative disease, preferably Alzheimer's disease. In more preferred embodiments said sequence identity is at least 80 %, preferably at least 90 %, more preferably at least 95 %, yet more preferably at least 99 % to the outlined sequences, yet more preferred the identity is 100%.

In one particular embodiment of the method for diagnosing a neurodegenerative disease the levels of all circRNAs comprising a sequence encoded by a sequence selected from the group consisting of nt 11 to 30 of any of SEQ ID NO:1 to SEQ ID NO:910, or a sequence having at least 70 % identity thereto; wherein preferably the presence of increased or decreased levels as defined in Table 1 under "disease" for at least 10, preferably at least 50, more preferably at least 100 of the respective circRNAs are indicative for the presence of a neurodegenerative disease, preferably Alzheimer's disease. Preferably, said method comprises the determination of the levels of all circRNAs comprising a sequence encoded by a sequence selected from the group consisting of any of SEQ ID NO:1 to SEQ ID NO:910 or a sequence having at least 70 % identity thereto, wherein preferably the presence of increased or decreased levels as defined in Table 1 under "disease" for at least 10, preferably at least 50, more preferably at least 100 of the respective circRNAs are indicative for the presence of a neurodegenerative disease, preferably Alzheimer's disease. Further preferred, all circRNAs with a sequence encoded by a sequence having at least 70 % identity to a sequence selected from the group consisting of SEQ ID NO:911 to SEQ ID NO:1820 are detected, wherein preferably the presence of increased or decreased levels as defined in Table 1 under "disease" for at least 10, preferably at least 50, more preferably at least 100 of the respective circRNAs are indicative for the presence of a neurodegenerative disease, preferably Alzheimer's disease. In more preferred embodiments said sequence identity is at least 80 %, preferably at least 90 %, more preferably at least 95 %, yet more preferably at least 99 % to the outlined sequences, yet more preferred the identity is 100%.

As outlined herein above, the circRNAs may be specifically detected through their unique sequence at the exon-exon junction in the head-to-tail arrangement. Hence, the disclosure also relates to a nucleic acid probe specifically hybridizing to a sequence of nucleotide (nt) 11 to nt 30 of any of the sequences of SEQ ID NO:1 to 910, or specifically hybridizing to a RNA sequence encoded by these sequences, or specifically hybridizing to a reverse complement sequences thereof, preferably specifically binding to any of the sequences of SEQ ID NO:1 to 910, or specifically hybridizing to a RNA sequence encoded by these sequences, or specifically hybridizing to a reverse complement sequences thereof. In a very preferred embodiment the nucleic acid probe spans the sequence of nt 15 to nt 35 of the respective SEQ ID NO: 1 to 910, of RNA sequence encoded by these sequences, or the reverse complement sequences thereof.

Nucleic acid probes may be prepared using any suitable method, such as, for example, the phosphotriester and phosphodiester methods or automated embodiments thereof. In one such automated embodiment diethylophosphoramidites are used as starting materials and may be synthesized as described by Beaucage et al., Tetrahedron Letters, 22:1859-1862 (1981). One method for synthesizing oligonucleotides on a modified solid support is described in U.S. Pat. No. 4,458,006. It is also possible to use a nucleic acid probes which has been isolated from a biological source (such as a restriction endonuclease digest). Preferred nucleic acid probes have a length of from about 15 to 500, more preferably about 20 to 200, most preferably about 25 to 60 bases.

The nucleic acid probe according to the present disclosure may be hybridization probe or as a primer for amplification reactions. In both cases the nucleic acid probe may comprise fluorescent dyes. Such fluorescent dyes may for example be FAM (5-or 6-carboxyfluorescein), VIC, NED, fluorescein, FITC, IRD-700/800, CY3, CY5, CY3.5, CY5.5, HEX, TET, TAMRA, JOE, ROX, BODIPY TMR, Oregon Green, Rhodamine Green, Rhodamine Red, Texas Red, Yakima Yellow, Alexa Fluor, PET and the like (*see* e.g. https://www.micro-shop.zeiss.com/us/us_en/spektral.php). In the context of the present invention, fluorescent dyes may for example be FAM (5-or 6-carboxyfluorescein), VIC, NED, fluorescein, fluorescein isothiocyanate (FITC), IRD-700/800, cyanine dyes, auch as CY3, CY5, CY3.5, CY5.5, Cy7, xanthen, 6-carboxy-2',4',7',4,7-hexachlorofluorescein (HEX), TET, 6-carboxy-4',5'-dichloro-2',7'-dimethodyfluorescein (JOE), N,N,N',N'-Tetramethyl-6-carboxyrhodamine (TAMRA), 6-carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, coumarines such as Umbelliferone, benzimides, such as Hoechst 33258; phenanthridines, such as Texas Red, Yakima Yellow, Alexa Fluor, PET, ethidium bromide, acridinium dyes, carbazol dyes, phenoxazine dyes, porphyrine dyes, polymethin dyes, and the like.

In a preferred embodiment, the nucleic acid probe specifically hybridizing to the sequence of nucleotide 11 to 30 of a sequence selected from the group consisting of the sequences listed in Table 1, or specifically hybridizing to a RNA sequence encoded by these sequences, or specifically hybridizing to a reverse complement sequences thereof; preferably specifically hybridizing to a sequence selected from the group consisting of the sequences listed in Table 1, or specifically hybridizing to a RNA sequence encoded by these sequences, or specifically hybridizing to a reverse complement sequences thereof.

The invention furthermore relates to a kit as defined in the appendent claims.

The disclosure furthermore relates to a kit for specifically detecting one or more, preferably more than one nucleic acids comprising a sequence selected from the group consisting of nt 11 to nt 30 of any one of SEQ ID NO: 1 to 910, or a sequence selected from the group consisting of SEQ ID NO:1 to 910, or SEQ ID NO:911 to SEQ ID NO:1820, or an RNA sequence encoded by any of these sequences. The kit is preferably a kit for diagnosing a neurodegenerative disease, comprising means for specifically detecting one or more nucleic acid sequence selected from the group consisting of SEQ ID NO:1 to 910 or SEQ ID NO:911 to SEQ ID NO:1820, or an RNA sequence encoded by any of these sequences. In a preferred embodiment the kit comprises means for specifically detecting at least 100, preferably at least 150 more preferably at least 200 nucleic acid sequences selected from the group consisting of SEQ ID NO:1 to SEQ ID NO:910 or SEQ ID NO:911 to SEQ ID NO:1820, or an RNA sequence encoded by any of these sequences.

The means for detecting preferably are one or more of the nucleic acid probes according to the disclosure. Hence, in one embodiment the kit comprises one or more nucleic acid probes, preferably more than one nucleic acid probe specifically hybridizing to the sequence of nucleotide 11 to 30 of a sequence selected from the group consisting of SEQ ID NO:1 to SEQ ID NO:910, or an RNA sequence encoded by these sequences, or the reverse complements thereof; preferably the kit comprises a plurality of nucleic acid probes specifically hybridizing to the sequence of nucleotide 11 to 30 of at least 100, preferably at least 150, more preferably at least 200 nucleic acid sequences selected from the group consisting of SEQ ID NO:1 to SEQ ID NO:910, or a RNA sequence encoded by these sequences, or the reverse complements thereof. In a particular preferred embodiment the kit comprises a plurality of nucleic acid probes hybridizing to the sequence of nucleotide 11 to 30 of at least 100, preferably at least 150, more preferably at least 200 nucleic acid sequences selected from the group consisting of SEQ ID NO:1 to 200, or the RNA sequences encoded by these sequences, or the reverse complements thereof; preferably the kit comprises a plurality of nucleic acid probes hybridizing to the sequence of nucleotide 11 to 30 of all of the sequences of SEQ ID NO:1 to 200, , or the RNA sequences encoded by these sequences, or the reverse complements thereof.

In a further particular preferred embodiment the kit comprises a plurality of nucleic acid probes hybridizing to the sequence of at least 100, preferably at least 150, more preferably at least 200 nucleic acid sequences selected from the group consisting of SEQ ID NO:1 to 200, , or an RNA sequence encoded by these sequences, or the reverse complements thereof; preferably the kit comprises a plurality of nucleic acid probes hybridizing to the sequence of all of the sequences of SEQ ID NO:1 to 200,, or the RNA sequences encoded by these sequences, or the reverse complements thereof.

The kit may further comprise means for handling and/or preparation of a bodily fluid sample, preferably for cerebrospinal fluid or whole blood. In a preferred embodiment the kit comprises a container for collecting whole blood, said container comprising stabilizing agents, preferably selected from the group consisting of chelating agents, EDTA, K₂EDTA, formulations like RNAlater (Qiagen) or such, or combinations thereof. In a particular preferred embodiment the kit comprises a K₂EDTA coated container.

As used herein, a kit is a packaged combination optionally including instructions for use of the combination and/or other reactions and components for such use.

Furthermore, the disclosure relates to an array for determining the presence or level of a plurality of nucleic acids, said array comprising a plurality of probes, wherein the plurality of probes specifically hybridize to the sequence of nucleotide 11 to 30 of a sequence selected from the group consisting of SEQ ID NO:1 to SEQ ID NO:910, or an RNA sequence encoded by these sequences, or the reverse complements thereof, preferably the plurality of probes comprises probes specifically hybridizing to the sequence of nucleotide 11 to 30 of at least 100, preferably at least 150 more preferably at least 200 nucleic acid sequences selected from the group consisting of SEQ ID NO:1 to SEQ ID NO:910, or the RNA sequences encoded by these sequences, or the reverse complements thereof. Preferably the plurality of probes comprises probes specifically hybridizing to the sequence of nucleotide 11 to 30 of SEQ ID NO:1 to SEQ ID NO:200, or the RNA sequences encoded by these sequences, or the reverse complements thereof.

Herein an "array" is a solid support comprising one or more nucleic acids attached thereto. Arrays, such as microarrays (e.g. from Affimetrix®) are known in the art Schena M¹, Shalon D, Davis RW, Brown PO(1995); Quantitative monitoring of gene expression patterns with a complementary DNA microarray.Science 270(5235):467-70. The solid support may be made of different nature including, but not limited to, those made of plastics, resins, polysaccharides, silica or silica-based materials, functionalized glass, modified silicon, carbon, metals, inorganic glasses, membranes, nylon, natural fibers such as silk, wool and cotton, and polymers. In some embodiments, the material comprising the solid support has reactive groups such as carboxy, amino, hydroxy, etc., which are used for attachment of, e.g. nucleic acid probes. Polymers are preferred, and suitable polymers include, but are not limited to, polystyrene, polyethylene glycol tetraphthalate, polyvinyl acetate, polyvinyl chloride, polyvinyl pyrrolidone, polyacrylonitrile, polymethyl methacrylate, polytetrafluoroethylene, butyl rubber, styrenebutadiene rubber, natural rubber, polyethylene, polypropylene, (poly)tetrafluoroethylene, (poly)vinylidenefluoride, polycarbonate and polymethylpentene. Preferred polymers include those outlined in U.S. Patent No. 5,427,779. The nucleic acid probes are preferably covalent attachment to the solid support of the array. Attachment may be performed as described below. As will be appreciated by those in the art, either the 5' or 3' terminus may be attached to the support using techniques known in the art. The arrays of the disclosure comprise at least two different covalently attached nucleic acid probes, with more than two being preferred. By "different" oligonucleotide herein is meant an oligonucleotide that has a nucleotide sequence that differs in at least one position from the sequence of a second oligonucleotide; that is, at least a single base is different, preferably their hybridization specificity is as outlined herein above.

Furthermore, the disclosure particularly relates to the use of a nucleic acid probe specifically hybridizing to the sequence of nucleotide 11 to 30 of a sequence selected from the group consisting of SEQ ID NO:1 to SEQ ID NO:910, and the RNA sequences encoded by these sequences, or hybridizing to the reverse complement thereof for the diagnosis of a neurodegenerative disease, preferably for the diagnosis of Alzheimer's disease The invention also relates to the use of a kit according to the invention for the diagnosis of Alzheimer's disease. Also described is the use of an array according to the disclosure for the diagnosis of a neurodegenerative disease, preferably for the diagnosis of Alzheimer's disease.

It will be apparent that the methods and components of the present invention, as well as the uses as substantially described herein or illustrated in the description and the examples, are also subject of the present invention and claimed herewith. In this respect, it is also understood that the embodiments as described in the description and/or any one of the examples, can be independently used and combined with any one of the embodiments described hereinbefore and claimed in the appended claims set. Thus, these and other embodiments are disclosed and encompassed by the description and examples of the present invention.

The invention is further illustrated by the following non-limiting Examples and Figures.

### Examples

### 1. Methods

### 1.1 Whole blood sample collection

Blood sampling was approved by the Charité ethics committee, registration number EA4/078/14 and all participants gave written informed consent. 5 mL blood were drawn from subjects by venipuncture and collected in K₂EDTA coated Vacutainer (BD, #368841) and stored on ice until used for RNA preparation. For downstream RNA analysis by sequencing or qPCR assays presented here, 100 µL blood (> 1 µg total RNA) is sufficient.

### 1.2 RNA isolation and RNase R treatment

Total RNA was isolated from fresh whole blood samples. Blood was diluted 1:3 in PBS and 250 µL of the dilution were used for RNA preparation using 750 µL Trizol LS reagent (Life Technology). Samples were homogenized by gentle vortexing and 200 µL chloroform was added. After centrifugation at 4 °C, 15 min at full speed in a table top centrifuge, the aqueous phase was collected to a new tube (typically 400 µL). RNA was precipitated by adding an equal volume of cold isopropanol and incubation for ≥ 1 hour at -80 °C. RNA pellets were recovered by spinning at 4 °C, 30 min at full speed in a table top centrifuge. RNA pellets were washed with 1 mL 80 % EtOH and subsequently air dried at room temperature for 5 min. The RNA was resuspended in 20 µL RNase-free water and treated with DNase I (Promega) for 15 min at 37 °C with subsequent heat inactivation for 10 min at 65 °C. HEK293 total RNA was prepared in the same way but using 1 mL Trizol on cell pellets. For sequencing experiments the RNA preparations were additionally subjected to two rounds of ribosomal RNA depletion using a RiboMinus Kit (Life Technologies K1550-02 and A15020). Total RNA integrity and rRNA depletion were monitored using a Bioanalyzer 2001 (Agilent Technologies). For qPCR analysis the samples were treated with RNase R (Epicentre) for 15 min at 37 °C at a concentration of 3 U/µg RNA. After treatment 5 % *C*. *elegans* total RNA was spiked-in followed by phenol-chloroform extraction of the RNA mixture. For controls the RNA was mock treated without the enzyme.

### 1.3 cDNA library preparation for Deep Sequencing

cDNA libraries were generated according to the Illumina TruSeq protocol. Sample RNA was fragmented, adaptor ligated, amplified and sequenced on an Illumina HiSeq2000 in 1x 100 cycle runs.

### 1.4 Quantitative PCR (qPCR)

Total RNA was reverse transcribed using Maxima reverse transcriptase (Thermo Scientific) according to the manufacturer's protocol. qPCR reactions were performed using Maxima SYBR Green/Rox (Thermo Scientific) on a StepOne Plus System (Applied Biosystems). Primer sequences are available in the Table 7. RNase R assays were normalized to *C*. *elegans* RNA spike-in RNA.

### 1.5 Sanger Sequencing

PCR products were size separated by agarose gel electrophoresis, amplicons were extracted from gels and Sanger sequenced by standard methods (Eurofins).

### 1.6 Detection and annotation of circRNAs

The detection of circular RNA was based on a previously published method *(see* Memczak S, Jens M, Elefsinioti A, et al. Circular RNAs are a large class of animal RNAs with regulatory potency. Nature. 2013;495(7441):333-338) with the following details. Human reference genome hg19 (Feb 2009, GRCh37) was downloaded from the UCSC genome browser *(see* Kent WJ, Sugnet CW, Furey TS, et al. The human genome browser at UCSC. Genome Research. 2002;12(6):996-1006) and was used for all subsequent analysis. bowtie2 (version 2.1.0 *(see* Langmead B, Salzberg SL. Fast gapped-read alignment with Bowtie 2. Nature Methods. 2012;9(4):357-359) was employed for mapping of RNA sequencing reads. Reads were mapped to ribosomal RNA sequence data downloaded from the UCSC genome browser. Reads that do not map to rRNA were extracted for further processing. In a second step, all reads that mapped to the genome by aligning the whole read without any trimming (end-to-end mode) were neglected. Reads not mapping continuously to the genome were used for circRNA candidate detection. From those 20 nucleotide terminal sequences (anchors) were extracted and re-aligned independently to the genome. The anchor alignments were then extended until the full read sequence was covered. Consecutively aligning anchors indicate linear splicing events whereas alignment in reverse orientation indicates head-to-tail splicing as observed in circRNAs (Figure 1A). The resulting splicing events were filtered using the following criteria 1) GT/AG signal flanking the splice sites 2) unambiguous breakpoint detection 3) maximum of two mismatches when extending the anchor alignments 4) breakpoint no more than two nucleotides inside the alignment of the anchors 5) at least two independent reads supporting the head-to-tail splice junction 6) a minimum difference of 35 in the bowtie2 alignment score between the first and the second best alignment of each anchor 7) no more than 100 kilobases distance between the two splice sites.

### 1.7 circRNA annotation

Genomic coordinates of circRNA candidates were intersected with published gene models (ENSEMBL, release 75 containing 22,827 protein coding genes, 7484 lincRNAs and 3411 miRNAs). circRNAs were annotated and exon-intron structure predicted as previously described (*see* Memczak S, Jens M, Elefsinioti A, et al. Circular RNAs are a large class of animal RNAs with regulatory potency. Nature. 2013;495(7441):333-338). Known introns in circRNAs were assumed to be spliced out. Each circRNA was counted to a gene structure category if it overlaps fully or partially with the respective ENSEMBL feature (Figure 1C, Table 1).

### 1.8 Published RNA data sets

In this study rRNA depleted RNA-seq data from whole blood samples (own data), fetal cerebellum (ENCODE accession: ENCSR000AEW) fetal liver (ENCODE accession: ENCSR000AFB) and HEK293 (Table 1; *see* Ivanov A, Memczak S, Wyler E, et al. Analysis of Intron Sequences Reveals Hallmarks of Circular RNA Biogenesis in Animals. CellReports. 2015;10(2):170-177) was used. Expression values, coordinates and other details of the circRNAs reported here and all associated scripts will be made available at www.circbase.org (*see* Glažar P, Papavasileiou P, Rajewsky N. circBase: a database for circular RNAs. RNA. 2014;20(11):1666-1670).

### 1.9 Quantification of circRNA and host gene expression

The number of reads that span a particular head-to-tail junction were used as a measure for circRNA expression. To allow comparison of expression between samples, raw read counts were normalized to sequencing depth by dividing by the number of reads that map to protein coding gene regions and multiply by 1,000,000 (Figure 1B left, Figure 4 C-D, Figure 6 and 10 A,C). To estimate host gene expression, RNA-seq data were first mapped to the reference genome with STAR (*see* Dobin A, Davis CA, Schlesinger F, et al. STAR: ultrafast universal RNA-seq aligner. Bioinformatics. 2013;29(1):15-21). htseq-count (*see* Anders S, Pyl PT, Huber W. HTSeq - A Python framework to work with high-throughput sequencing data. 2014) was employed to count hits on genomic features of ENSEMBL gene models. The measure transcripts per million (TPM) was calculated for each transcript and sample in order to compare total host gene expression between samples (Figure 1B, right).

Circular-to-linear ratios were calculated for each circRNA by dividing raw head-to-tail read counts by the median number of reads that span linear spliced junctions of the respective host gene. For both measures one pseudo count was added to avoid division by zero. CircRNAs from host genes without annotated splice junctions according to the ENSEMBL gene annotation, were not considered in this analysis.

For analysis in Figure 3D a permutation test with 1000 Monte-Carlo replications was performed on pooled biological replicate data to approximate the exact conditional distribution. To adjust for different dataset sizes the respective larger data set of each comparison was randomly subsampled.

### 1.10 Principal Component Analysis and Clustering

To perform principal component analysis (PCA) of circRNA expression in whole blood samples of different donors, variance stabilizing transformation was first performed on raw head-to-tail spliced read counts using the R package DESeq2 (*see* Love MI, Huber W, Anders S. Moderated estimation of fold change and dispersion for RNA-seq data with DESeq2. Genome Biology. 2014;15(12):550). Only circRNAs with a transformed expression value of at least 6.7 (n=910, Figure 4A) in one of the samples were considered for the analysis. PCA was performed on all remaining circRNAs using the prcomp function of R's stats package. All genes that give rise to these circRNAs and have at least one known splice junction were considered for PCA of the linear host gene expression. Same procedure was used for PCA using the median number of linear spliced reads as a proxy for linear expression. 200 circRNAs with the highest weight in PC2 were considered for clustering. Raw head-to-tail spliced read counts for each circRNA (nᵢ) were normalized to sequencing depth by dividing by the number of reads that map to protein coding gene regions multiplied by 1,000,000. Whole blood samples of different donors were clustered on log₂ transformed normalized circRNA expression profiles (log₂(nᵢ + 1)). Hierarchical, agglomerative clustering was performed with complete linkage and by using Spearman's rank correlation as distance metric (1 - {corr[log(nᵢ + 1)]}). The same procedure was used for linear host gene expression using the median number of linear spliced reads for all genes that give rise to these 200 circRNAs and have at least one known splice site.

### 2. Results

### 2.1 Thousands of circRNAs are reproducibly detected in human peripheral whole blood

First it was determined whether circRNAs are present in standard clinical blood specimen. To this end, total RNA was prepared from two biologically independent human peripheral whole blood samples and depleted ribosomal RNAs (*see* Methods, *supra*). The samples were reverse transcribed using random primers to allow for circRNA detection and sequencing libraries were produced (Figure 1A). The raw reads were fed into our *in silico* circRNA detection pipeline (*see* Memczak S, Jens M, Elefsinioti A, et al. Circular RNAs are a large class of animal RNAs with regulatory potency. Nature. 2013;495(7441):333-338). In short, the program filters reads that map continuously to the genome but saves unmapped reads. From those, terminal 20-mer anchors are extracted and independently aligned to the genome. If the anchors map in reverse orientation and can be extended to cover the whole read sequence, they are flagged as head-to-tail junction spanning, *i.e.* indicative for circRNAs. Anchors that aligned consecutively were used to determine linear splicing as an internal library quality control and to assess linear RNA isoform expression (Table 2).

From the RNA of two human donors we identified 4550 and 4105 unique circRNA candidates, respectively, by at least two independent reads spanning a head-to-tail splice junction (Figure 1B). In both datasets the number of total reads and linear splicing events were respectively similar, indicating reproducible sample preparation (Table 2, Table 5). When considering RNAs found in both samples, we observed a high correlation of expression for both linear (R=0.98) as well as circRNAs (R=0.80, Figure 1B). Between the two samples 1265 circRNAs (55 %) with more than 5 reads overlap and 2442 (39 %) circRNAs supported by at least 2 reads are shared (Table 1, Figure 15, Figure 5, technical reproducibility is shown in Figure 6). The later set will be considered as reproducibly detected circRNAs in the following analysis. CircRNA candidates are derived from genes covering the whole dynamic range of RNA expression (Figure 1B, right panel). As observed in other human samples, we find that most circRNAs are derived from protein coding exonic regions or 5' UTR sequences (Figure 1C; *see* Memczak S, Jens M, Elefsinioti A, et al. Circular RNAs are a large class of animal RNAs with regulatory potency. Nature. 2013;495(7441):333-338, and Rybak-Wolf A, Stottmeister C, Glažar P, et al. Circular RNAs in the Mammalian Brain Are Highly Abundant, Conserved, and Dynamically Expressed. MOLCEL. 2015;1-17). GO term enrichment analysis on reproducibly detected, top expressed circRNAs and the same number of top linear RNAs showed significant enrichment of different biological function annotations (Figure 7). Together with the broad expression spectrum of corresponding host genes this finding argues that circRNA expression levels are largely independent of linear RNA isoform abundance.

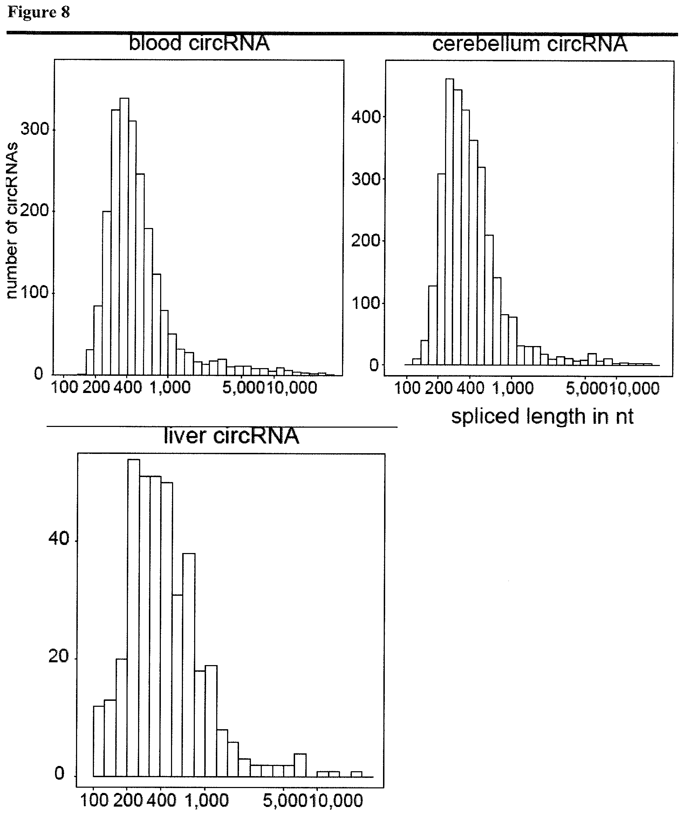

The predicted spliced length of blood circRNAs of 200-800 nt (median = 343 nt) is similar to that in liver or cerebellum (median = 394/448 nt) and previous observations in HEK293 cell cultures and other human samples (Figure 8 and see Memczak S, Jens M, Elefsinioti A, et al. Circular RNAs are a large class of animal RNAs with regulatory potency. Nature. 2013;495(7441):333-338). However, we observed a high number of circRNAs per gene, with 23 genes giving rise to more than 10 circRNAs ('circRNA hotspots', Figure ID).

To assess the reproducibility of the sequencing results we designed divergent, circRNA specific primers and measured relative abundances of the top eight expressed circRNAs compared to linear control genes in qPCR (Figure 1E). circRNA candidate 8 could not be unambiguously amplified from cDNA, most likely due to overlapping RNA isoforms and was therefore excluded from further analysis. For the remaining seven circRNA candidates, we tested circularity using previously established assays: 1) resistance to the 3'-5' exonuclease RNase R and 2) Sanger sequencing of PCR amplicons to confirm the sequence of predicted head-to-tail splice junctions. With these assays we validated 7/7 tested candidates suggesting that the overall false positive rate in our data sets is low (Figure 9). Interestingly, these circRNAs are expressed from gene loci that so far were not shown to have a specific blood related function (Table 3) but show expression levels that by far exceed expression of housekeeping genes such as VCL or TFRC (10-100fold, Figure 1E).

### 2.2 Circular-to-linear RNA expression is high in blood

When inspecting the read coverage in blood sequencing data, it was noticed that oftentimes the expression of circularized exons was outstandingly high compared to the coverage of neighboring exons expressed in linear RNA isoforms of the same gene. For example, it was observed that the two exons of circRNA candidate 5, which is product of the PCNT locus were densely covered with sequencing reads in the blood samples, while the upstream and downstream exons were barely detected (Figure 2A). This particular expression pattern was not observed in HEK293 cells, where all exons were equally covered. This observation was further investigated by qPCR, comparing linear to circular RNA expression with isoform specific primer sets in HEK293 and whole blood samples (Figure 2B, C). This independent assay confirmed dominant expression of the tested candidates which was found to be at least 30-fold higher than the cognate linear isoforms. In contrast, this circRNA domination was not found in HEK293 cells where the same RNAs were probed, which argues for a tissue-specific pattern.

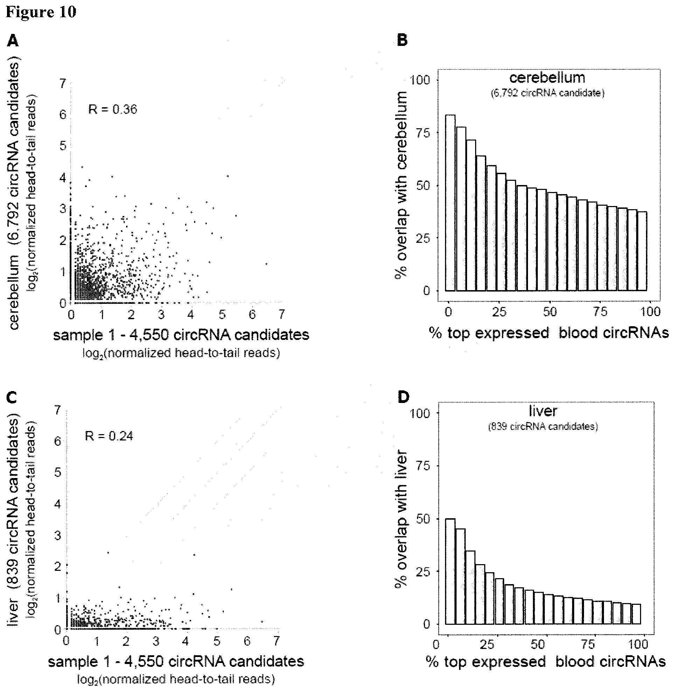

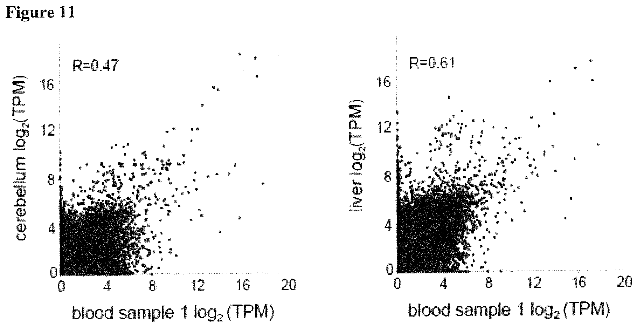

Thereafter, comparison of the blood data to published ENCODE project datasets from cerebellum, representative of neuronal tissues that in general have high circRNA expression *(see* Rybak-Wolf A, Stottmeister C, Glažar P, et al. Circular RNAs in the Mammalian Brain Are Highly Abundant, Conserved, and Dynamically Expressed. MOLCEL. 2015;1-17) and to a non-neuronal primary tissue, liver (Table 2) was performed. Approx. 30 % of blood circRNAs are also found in cerebellum while this fraction was around 10 % for liver with higher fractions for both cases when constraining the analysis to highly expressed blood circRNAs (Figure 10 A-D, comparison between total RNAs in Figure 11). In summary, circRNAs found in human whole blood in part overlap circRNAs expressed in cerebellum of liver, but also contain hundreds of other circRNAs.

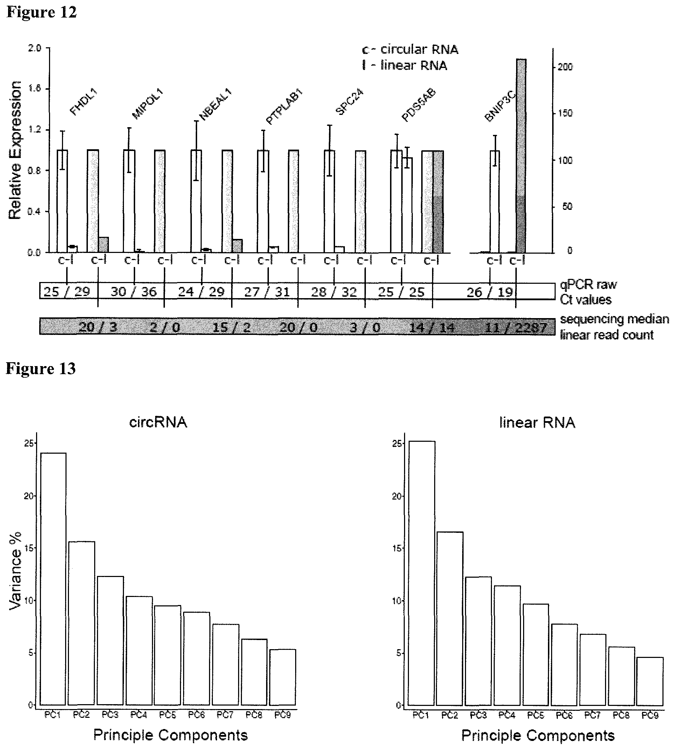

The relative circular to linear RNA isoform abundance on a transcriptome wide scale was then analyzed. To this end, read counts that span head-to-tail junctions and are therefore indicative of circRNAs were compared to the median number of read counts on linear splice site junctions on the same gene, the latter serving as a proxy for linear RNA expression (*see* Methods, *supra*). We observed that many blood circRNAs are highly expressed while corresponding linear RNAs show average or low abundances (Figure 3A), a finding that was recapitulated by qPCR assays validating our approach (Figure 12). For the control samples cerebellum and liver this pattern was not observed (Figure 3B, C) as revealed by comparing the mean circular-to-linear RNA ratio, which we found to be significantly higher in blood than in the tested control tissues (Figure 3D). In summary, blood has an outstanding general tendency to contain circRNAs at high levels while the corresponding linear transcripts are much more lowly expressed. This tendency was only found (to a much lower extent) in cerebellum but not in liver RNA as well as RNA from many other tissues or cell lines that we have analyzed.

### 2.3 circRNAs are putative biomarkers in Alzheimer's disease

The results show that circRNAs are reproducibly and easily detected in clinical standard blood samples and therefore are well suited to serve as a new class of biomarker for human diseases, like neurodegenerative diseases. Taking into account the high expression of circRNAs in neuronal tissues (*see* Rybak-Wolf A, Stottmeister C, Glažar P, et al. Circular RNAs in the Mammalian Brain Are Highly Abundant, Conserved, and Dynamically Expressed. MOLCEL. 2015;1-17) and the urgent need for biomarkers in neurological diseases, circular RNA expression in blood samples from Alzheimer's diseases patients and control subjects *(see* Methods, *supra,* Table 2, Table 4) was investigated. To this end, sequencing libraries from whole blood RNA from five individuals of each group were generated. In total 22,644 distinct circRNAs were detected in all samples combined. Then putative disease-specific circRNA expression were identified. Therefore, subsets of all detected circRNA candidates were defined and used these in a principle component analysis to detect expression differences between the two groups. Sorting of all circRNAs by expression and definition of sets for PCA analysis by increasing expression cut-offs was performed. In a range of the top 500 to top 900 circRNAs, a clear separation of control and diseased subjects was detected (Figure 4A, Figure 13). Interestingly, this is not observed when analyzing the corresponding linear RNAs, suggesting that there might be disease relevant information specifically encoded in the circular blood transcriptome (Figure 4B). When the circRNA were sorted out of this analysis by their weight in principle component 2 (PC2) and the data was subjected to unsupervised clustering, again controls and Alzheimer's patients were distinguishable. Importantly, the two main clusters do not reflect the gender or age of the subjects (*see* also Table 6). The findings of this analysis show that circRNA expression patterns in blood have a diagnostic value, that is not revealed by analyzing the expression of their cognate linear RNA isoforms.

### 3. Discussion

Recent publications show that circRNAs can be detected in plasma and saliva samples (*see* Koh W, Pan W, Gawad C, et al. Noninvasive in vivo monitoring of tissue-specific global gene expression in humans. Proceedings of the National Academy of Sciences. 2014;111(20):7361-7366; and Bahn JH, Zhang Q, Li F, et al. The Landscape of MicroRNA, Piwi-Interacting RNA, and Circular RNA in Human Saliva. Clinical Chemistry. 2014). However, in both specimens only few (10-70) circular RNAs with canonical splice sites were reported, which dramatically limits any further analysis. The circular transcriptome of whole blood presented here, demonstrates that the search for putative circRNA biomarker in peripheral blood is much more suitable to yield informative results. Using RNA-Seq of clinical standard samples showed reproducible detection of around 2400 circRNA candidates that are present in human whole blood. It will be interesting to determine the origin of blood circRNAs. Accumulating evidence suggests that circRNAs are specifically expressed in a developmental stage- and tissue-specific manner, rather than being merely byproducts of splicing reactions (*see* Memczak S, Jens M, Elefsinioti A, et al. Circular RNAs are a large class of animal RNAs with regulatory potency. Nature. 2013;495(7441):333-338; and Rybak-Wolf A, Stottmeister C, Glažar P, et al. Circular RNAs in the Mammalian Brain Are Highly Abundant, Conserved, and Dynamically Expressed. MOLCEL. 2015;1-17). Previously analyzed circRNA from neutrophils, B-cells and hematopoietic stem cells suggest that many circRNAs are constituents of hematocytes (*see* Salzman J, Gawad C, Wang PL, Lacayo N, Brown PO. Circular RNAs are the predominant transcript isoform from hundreds of human genes in diverse cell types. PLoS ONE. 2012;7(2):e30733). However, there is also the intriguing possibility of circRNA excretion into the extracellular space, e.g. by vesicles such as exosomes. Likewise, aberrant circRNA expression in disease may reflect, either a condition-specific transcriptome change in blood cells themselves, or a direct consequence of active or passive release of circRNA from diseased tissue.

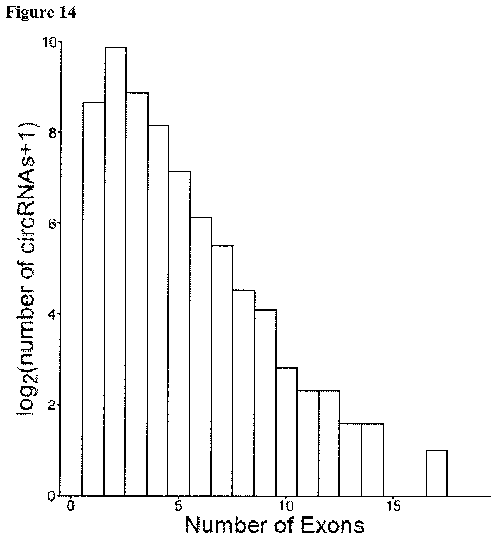

Further, we demonstrated that many circRNAs have a high expression compared to linear RNA isoforms from the same locus, a feature that distinguishes blood circRNAs from other primary tissues such as cerebellum or liver. Considering that this was observed for hundreds of blood circRNA candidates (Figure 3A, Table 1, Figure 15) and that further restricted the experimental setup to standard samples and preparation procedures. Gene products that are dominated by circRNAs which typically comprise 2-4 exons (example in Figure 2, Figure 14) will also dominate signals for the specific gene of interest in array assays, Northern Blots or qPCR experiments if the circularized exon expression is measured.

After reproducibly detecting thousands of oftentimes highly expressed circRNAs in blood, it was asked whether these might be instrumental in diagnosis of human disease. Therefore measurement of putatively specific circRNA abundances in Alzheimer's disease and control samples was performed.

It was observed that analyzing specific subsets of blood circular RNAs allows distinguishing Alzheimer's disease from control samples in a principal component analysis and unsupervised clustering. This distinction was not possible when analyzing linear RNA isoforms from the same genomic loci, demonstrating that circRNA expression data bear specific information.

Given the urgent need for non-invasive biomarker detection for many disease states, these findings show a way for biomarker detection in easily accessible bodily fluid samples; like whole blood and cerebrospinal fluid. These are not being limited to Alzheimer's disease or neurological condition in general, since blood circRNA expression might be specifically altered in many disorders and therefore exploitable as diagnostic tool in human diseases.

**Table 2. Sequencing statistic of analyzed libraries**

| **Sample** | **number of total reads (millions)** | **% map to rRNA** | **number of reads that map continously to genome (millions)** | **number of reads used for circRNA detection (millions)** | **number of linear splicing event s** | **reads mapping to protein coding genes (millions)** | **number of circRNA candidates** | **Ensembl accession ID** |
|---|---|---|---|---|---|---|---|---|
| H_1 | 57.85 | 11.52 | 41.75 | 9.45 | 77.367 | 8.47 | 4.550 | |
| rep_H_1 | 169.86 | 11.43 | 122.18 | 28.27 | 107.996 | 24.73 | 9.996 | |
| H_2 | 48.04 | 6.32 | 37.13 | 7.88 | 74.676 | 7.81 | 4.105 | |
| H_3 | 164.93 | 15.44 | 115.02 | 24.44 | 108.870 | 21.25 | 11.113 | |
| H_4 | 171.76 | 47.99 | 75.43 | 13.91 | 94.811 | 13.17 | 5.739 | |
| H_5 | 170.20 | 10.52 | 123.28 | 29.02 | 107.573 | 24.26 | 10.002 | |
| AD_1 | 110.76 | 18.74 | 74.07 | 15.93 | 88.932 | 13.85 | 5.837 | |
| AD_2 | 132.73 | 11.27 | 100.26 | 17.51 | 98.956 | 16.70 | 7.513 | |
| AD_3 | 131.62 | 15.78 | 93.46 | 17.39 | 91.823 | 17.22 | 6.867 | |
| AD_4 | 140.48 | 19.81 | 95.07 | 17.57 | 98.952 | 16.92 | 8.016 | |
| AD_5 | 122.04 | 13.88 | 88.91 | 16.18 | 96.942 | 17.41 | 6.404 | |
| Cerebellum_1 | 87.22 | 0.49 | 76.60 | 18.19 | 113,99 | 22.01 | 6.792 | ENCSR000AEW, ENCFF001ROL |
| Cerebellum_2 | 122.00 | 0.14 | 109.82 | 13.18 | 122.375 | 24.63 | 5.786 | ENCSR000AEW, ENCFF001RPH |
| Liver_1 | 86.12 | 3.35 | 72.72 | 10.52 | 101.147 | 30.14 | 839 | ENCSR000AFB, ENCFF001RNR |
| Liver_2 | 103.53 | 6.95 | 72.01 | 17.41 | 106.969 | 55.07 | 1.557 | ENCSR000AFB, ENCFF001RNX |

### Summary of RNA-sequencing results

Sequencing results for blood RNA from five controls (H), five Alzheimer patients (AD), cerebellum and liver control RNA samples. If not noted otherwise sample datasets were produced for this study.

**Table 3. Details on top expressed circRNA candidates.**

| **candidate** | | **host gene annoation** | **function** | **spliced length in nt** | **head-to-tail read counts** | **circBase ID** |
|---|---|---|---|---|---|---|
| 1 | MBOAT2 | membrane bound O-acyltransferase domain containing 2 | acyltransferase | 226 | 1367 | hsa_circ_0007334 |
| 2 | TMEM56 | Transmembrane Protein 56 | unknown | 264 | 676 | hsa_circ_0000095 |
| 3 | DNAJC6 | DnaJ Chaperone Homolog, Subfamily C, Member 6 | regulates chaperone activity | 302 | 513 | hsa_circ_0002454 |
| 4 | UBXN7 | UBX domain protein 7 | Ubiquitin-binding adapter | 183 | 485 | hsa_circ_0001380 |
| 5 | PCNT1 | Pericentrin-13 | component of the nuclear pore complex | 315 | 344 | hsa_circ_0002903 |
| 6 | MORC3 | MORC family CW-type zinc finger 3 | unknown | 249 | 333 | hsa_circ_0001189 |
| 7 | XPO1 | Exportin 1 | nuclear export of protein and RNAs | 207 | 333 | hsa_circ_0001017 |
| 8 | GSE1 | Coiled-Coil Protein Genetic Suppressor Element | unknown | 219 | 326 | hsa_circ_0000722 |

**Table 4. Patients overview.**

| **subject** | **age** | **diagnosis** | **stage** | **MMSE** | **sex** |
|---|---|---|---|---|---|
| H_1 | 31 | control | | | m |
| H_2 | 27 | control | | | m |
| H_3 | 71 | control | | | f |
| H_4 | 65 | control | | | f |
| H_5 | 62 | control | | | m |
| AD_1 | 75 | Alzheimer's Disease | mild | 24 | m |
| AD_2 | 81 | Alzheimer's Disease | mild | 23 | m |
| AD_3 | 73 | Alzheimer's Disease | mild | 25 | f |
| AD_4 | 69 | Alzheimer's Disease | medium | 18 | f |
| AD_5 | 68 | Alzheimer's Disease | severe | 8 | m |

| | | | | | |
|---|---|---|---|---|---|
| MMSE: Mini Mental State Examination | | | | | |

**Table 5. Raw reads mapping to hemoglobin genes for blood sample 1 and 2.**

| | **sample 1** | **sample 2** |
|---|---|---|
| total reads | 57,853,921 | 48,035,915 |
| | | |
| HBA1 | 2,429,755 | 2,016,794 |
| HBA2 | 3,330,428 | 1,891,626 |
| HBB | 3,964,389 | 3,703,140 |
| HBD | 1,016 | 936 |
| sum | 9,725,588 | 7,612,496 |
| % of total | 16.81 | 15.85 |

**Table 6. Rate of reproducibility after sub-sampling circRNAs in Fig 4a 1000 times.**

| % **circRNAs** | % clustering reproduced | % **linear RNAs** | % main cluster as in **Figure 4A** |
|---|---|---|---|
| 90 | 52.2 | 90 | 0 |
| 70 | 31.4 | 70 | 0 |
| 50 | 17.9 | 50 | 0 |

**Table 7. List of oligonucleotides used in the Examples**

| **SEQ ID NO:** | **Name** | **Sequence** |
|---|---|---|
| 1821 | hsaRTvinculinfwd | CTCGTCCGGGTTGGAAAAGAG |
| 1822 | hsaRTvinculinrev | AGTAAGGGTCTGACTGAAGCAT |
| 1823 | hsaTFRCfwd | ACCATTGTCATATACCCGGTTCA |
| 1824 | hsaTFRCrev | CAATAGCCCAAGTAGCCAATCAT |
| 1825 | celegansEIF3D_fwd | CGCCTTGAACATGGATAACTGCTGGG |
| 1826 | celegansEIF3D_rev | GATCGTCATCCGAGTTCTCCTCGTCG |
| 1827 | hsaMBOAT2div_fwd | AGTGCAAGATAAAGGCCCAAA |
| 1828 | hsaMBOAT2div_rev | TGATCATCATAGGAGTGGAGAACA |
| 1829 | hsaMBOAT2con_fwd | TACTCCACAGGTAATGTTGTAC |
| 1830 | hsaMBOAT2con_rev | ACTTTCATTGAAGGCAGATCATACCA |
| 1831 | hsaDNAJC6div_fwd | CCAGACATCTTGACCACTACACA |
| 1832 | hsaDNAJC6div_rev | ATGTGTCTTTGAGGGTGTCTTT |
| 1833 | hsaDNAJC6con_fwd | TCTCTACTCTACTCCTGGCCCAG |
| 1834 | hsaDNAJC6con_rev | GTAGGTCACACATATAGCCCAGGT |
| 1835 | hsaTMEM56div_fwd | CATCATTGTGCGTCCCTGTATG |
| 1836 | hsaTMEM56div_rev | GCTGAGACTATTGAAACCTGGAGA |
| 1837 | hsaTMEM56con_fwd | GCTGGCATACATTGGGAATTT |
| 1838 | hsaTMEM56con_rev | CAA TCCGCACGATGAAGAATAC |
| 1839 | hsaUBXN7div_fwd | ACCAGTATTTCCTGCTTTTGAGG |
| 1840 | hsaUBXN7div_rev | CTACCCTTGCAGATCTATTCCGG |
| 1841 | hsaUBXN7con_fwd | AGAAATCCCGTCACTTGGTCCAA |
| 1842 | hsaUBXN7con_rev | TGACAGTGAGGAAGGTCAGAGA |
| 1843 | hsaGSEldiv_fwd | CATCCTCCAGCTTTGCCGCCG |
| 1844 | hsaGSE1div_rev | CTGGTCGCGGTGGAAAGCATC |
| 1845 | hsaGSE1con_fwd | AGCTCAGTTGTGCAGGATTC |
| 1846 | hsaGSE1con_rev | CTTCTCAGGTAGTCCTCGGT |
| 1847 | hsaMORC3div_fwd | CA TCCTACGTGGACAGAAAGTGAA |
| 1848 | hsaMORC3div_rev | CTGTTCCGTGGAAAACAGAGAAT |
| 1849 | hsaMORC3con_fwd | CAGTGCAGTTGCTGAATTAATAG |
| 1850 | hsaMORC3con_rev | TCCCATTGTCGGTGAATGTC |
| 1851 | hsaPCNT1div_fwd | CCGGTGTTTAGAAGACTTGGAGTT |
| 1852 | hsaPCNT1div_rev | TGCAGACAGTTCTTTGCGTAGATT |
| 1853 | hsaPCNT1con_fwd | TTGCCATTACTGACCTGGAGAGC |
| 1854 | hsaPCNT1con_rev | CCGTCAATGCCGTCTCCTTCTC |
| 1855 | hsaXPO1div_fwd | TGAAATCAAGCAGCTGACGA |
| 1856 | hsaXPO1div_rev | AGATTCTTCCAAGGAACCAGTG |
| 1857 | hsaXPO1con_fwd | GCCAGGGACAGACATTTGA |
| 1858 | hsaXPO1con_rev | GCTCAAGTAAAGCTCTTTGTGAC |

## Claims

1. A method for diagnosing a disease of a subject, comprising the step of:
- determining the presence or absence of one or more circular RNA (circRNA) in a sample of a bodily fluid of said subject;
wherein the presence or absence of said one or more circRNA is indicative of the disease,
wherein the disease is Alzheimer's disease, and
wherein said bodily fluid is blood, preferably whole blood.

2. The method according to claim 1, wherein the determination step comprises:
- determining the level of said one or more circRNA;
- comparing the determined level to a control level of said one or more circRNA;
wherein differing levels between the determined and the control level are indicative of the disease.

3. The method according to claim 2, wherein said one or more circRNA is differentially expressed between the diseased and non-diseased state.

4. The method according to any one of claims 1 to 3, wherein the circRNA is detected by detection of an exon-exon-junction in a head-to-tail arrangement.

5. The method according to claim 4, wherein circRNA is detected using a method selected from the group consisting of probe hybridization based methods, nucleic acid amplification based methods, and nucleic acid sequencing.

6. The method according to any one of claims 1 to 5, wherein the sample is treated with RNase R before determination of the circRNA.

7. The method according to any one of claims 1 to 6, wherein more than one circRNAs from a panel of circRNAs are determined.

8. The method according to claim 7, wherein said panel comprises a plurality of circRNAs that have been identified as being present at differing levels in bodily fluid samples of patients having the disease and patients not having the disease, preferably identified by principle component analysis or clustering.

9. The method according to any one of claims 2 to 8, wherein said one or more circRNA comprises a sequence encoded by a sequence being at least 70 % identical to a sequence selected from the group consisting of nucleotides 11 to 30 of any of the sequences of SEQ ID NO:1 to SEQ ID NO:910, preferably wherein the presence of increased or decreased levels as defined in Table 1 under "disease" for the circRNA comprising the respective sequence are indicative of the presence of Alzheimer's disease.

10. The method according to claims 9, wherein said one or more circRNA has a sequence encoded by a sequence having at least 70 % identity to a sequence selected from the group consisting of SEQ ID NO:911 to SEQ ID NO:1820, preferably wherein the presence of increased or decreased levels as defined in Table 1 under "disease" for the circRNA having the respective sequence are indicative of the presence of Alzheimer's disease.

11. The method according to any of claims 2 to 9, wherein the levels of at least 100, preferably at least 150 and more preferably at least 200 circRNAs comprising a sequence encoded by a sequence being at least 70 % identical to a sequence selected from the group consisting of SEQ ID NO:1 to SEQ ID NO:910 are determined in said sample of a bodily fluid of said subject and controlled to the respective control level.

12. The method according to claim 11, wherein said at least 100, preferably at least 150 and more preferably at least 200 circRNAs have a sequence encoded by a sequence selected from the group consisting of SEQ ID NO:911 to 1820, or a sequence being at least 70 % identical thereto.

13. The method according to claim 11 or 12, wherein said at least 100, preferably at least 150 more preferably at least 200 circRNAs comprise a sequence encoded by a sequence having at least 70 % identity to a sequence selected from the group consisting of SEQ ID NO:1 to SEQ ID NO:200, or said circRNAs have a sequence having at least 70 % identity to a sequence selected from the group consisting of SEQ ID NO: 911 to 1110.

14. The method according to claim 11, wherein the presence of increased or decreased levels as defined in Table 1 under "disease" for the respective circRNA for at least 10, preferably at least 50, more preferably at least 100 of said at least 100, preferably at least 150 and more preferably at least 200 circRNAs are indicative of the presence of Alzheimer's disease.

15. The method according to any one of claims 9 to 12, wherein the levels of all circRNAs comprising a sequence encoded by a sequence having at least 70 % identity to a sequence selected from the group consisting of SEQ ID NO:1 to SEQ ID NO:910 or all circRNAs having a sequence encoded by a sequence having at least 70 % identity to a sequence selected from the group consisting of SEQ ID NO:911 to SEQ ID NO:1820 are detected, wherein preferably the presence of increased or decreased levels as defined in Table 1 under "disease" for at least 10, preferably at least 50, more preferably at least 100 of the respective circRNAs are indicative of the presence of Alzheimer's disease.

16. A kit for diagnosing Alzheimer's disease, comprising means for specifically detecting a nucleic acid sequence encoded by SEQ ID NO:1 or SEQ ID NO:911, or a sequence being at least 70 % identical to the recited sequences,
wherein the kit further comprises a container for collecting whole blood, said container comprising stabilizing agents preferably selected from the group consisting of chelating agents, EDTA, K₂EDTA, or combinations thereof.

17. Use of a kit according to claim 16 for the diagnosis of Alzheimer's disease.

## Patentansprüche

1. Verfahren für die Diagnose einer Erkrankung eines Individuums, umfassend den Schritt:
- Bestimmen des Vorliegens oder des Fehlens einer oder mehrerer circulärer RNAs (circRNA) in einer Probe einer Körperflüssigkeit des Individuums;
wobei das Vorliegen oder das Fehlen der einen oder mehreren circRNAs ein Anzeichen für die Erkrankung ist, wobei die Erkrankung Alzheimer-Krankheit ist, und
wobei die Körperflüssigkeit Blut, bevorzugt Vollblut ist.

2. Verfahren nach Anspruch 1, wobei der Bestimmungsschritt umfasst:
- Bestimmen des Spiegels der einen oder mehreren circRNAs;
- Vergleichen des Spiegels, der bestimmt wurde, mit einem Kontrollspiegel der einen oder mehreren circRNAs;
wobei Unterschiede zwischen dem bestimmten Spiegel und dem Kontrollspiegel ein Anzeichen für die Erkrankung sind.

3. Verfahren nach Anspruch 2, wobei die eine oder die mehreren circRNAs im Zustand der Erkrankung und der Nicht-Erkrankung differentiell exprimiert sind.

4. Verfahren nach einem der Ansprüche 1 bis 3, wobei die circRNA durch den Nachweis einer Exon-Exon-Junction in einer Head-to-Tail-Anordnung nachgewiesen wird.

5. Verfahren nach Anspruch 4, wobei circRNA unter Verwendung eines Verfahrens nachgewiesen wird, das ausgewählt ist aus der Gruppe bestehend aus Verfahren auf Grundlage der Sondenhybridisierung, Verfahren auf der Grundlage der Nucleinsäureamplifizierung und Nucleinsäuresequenzierung.

6. Verfahren nach einem der Ansprüche 1 bis 5, wobei die Probe vor der Bestimmung der circRNA mit RNase R behandelt wird.

7. Verfahren nach einem der Ansprüche 1 bis 6, wobei mehr als eine circRNA von einem circRNA-Panel bestimmt wird.

8. Verfahren nach Anspruch 7, wobei der Panel eine Vielzahl an circRNAs umfasst, die als in verschiedenen Spiegeln in Körperflüssigkeitsproben von Patienten, die die Erkrankung haben, und Patienten, die die Erkrankung nicht haben, vorliegend identifiziert wurden, bevorzugt durch Hauptkomponentenanalyse (principle component analysis) oder Clustering.

9. Verfahren nach einem der Ansprüche 2 bis 8, wobei die eine oder mehreren circRNAs eine Sequenz umfasst/umfassen, die von einer Sequenz codiert wird, die zu mindestens 70% identisch mit einer Sequenz ist, die aus der Gruppe bestehend aus den Nucleotiden 11 bis 30 einer der Sequenzen von SEQ ID NO:1 bis SEQ ID NO:910 ausgewählt ist, bevorzugt wobei das Vorliegen erhöhter oder verringerter Spiegel, wie in Tabelle 1 unter "Erkrankung" für die circRNA, die die entsprechende Sequenz umfasst, definiert, ein Anzeichen für das Vorliegen der Alzheimer-Erkrankung ist.

10. Verfahren nach Anspruch 9, wobei die eine oder mehreren circRNAs eine Sequenz aufweist/aufweisen, die von einer Sequenz codiert wird, die mindestens 70% Identität mit einer Sequenz aufweist, die aus der Gruppe bestehend aus SEQ 10 NO:911 bis SEQ ID NO:1820 ausgewählt ist, bevorzugt wobei das Vorliegen von erhöhten oder verringerten Spiegeln, wie in Tabelle 1 unter "Erkrankung" für die circRNA, die die entsprechende Sequenz aufweist, definiert, ein Anzeichen für das Vorliegen der Alzheimer-Krankheit ist.

11. Verfahren nach einem der Ansprüche 2 bis 9, wobei die Spiegel von mindestens 100, bevorzugt mindestens 150 und stärker bevorzugt mindestens 200 circRNAs, die eine Sequenz umfassen, die von einer Sequenz codiert wird, die zu mindestens 70% identisch mit einer Sequenz ist, die aus der Gruppe bestehend aus SEQ ID NO:1 bis SEQ ID NO:910 ausgewählt ist, in der Probe einer Körperflüssigkeit des Individuums bestimmt werden und mit dem entsprechenden Kontrollspiegel kontrolliert werden.

12. Verfahren nach Anspruch 11, wobei mindestens 100, bevorzugt mindestens 150 und stärker bevorzugt mindestens 200 circRNAs eine Sequenz aufweisen, die von einer Sequenz codiert wird, die aus der Gruppe bestehend aus SEQ ID NO:911 bis SEQ ID NO:1820 ausgewählt ist, oder eine Sequenz, die zu mindestens 70% identisch mit dieser ist.

13. Verfahren nach einem der Ansprüche 11 oder 12, wobei mindestens 100, bevorzugt mindestens 150, stärker bevorzugt mindestens 200 circRNAs eine Sequenz umfassen, die von einer Sequenz codiert wird, die mindestens 70% Identität mit einer Sequenz aufweist, die aus der Gruppe bestehend aus SEQ ID NO:1 bis SEQ ID NO:200 ausgewählt ist, oder die circRNAs eine Sequenz aufweisen, die mindestens 70% Identität mit einer Sequenz aufweisen, die aus der Gruppe bestehend aus SEQ ID NO:911 bis 1110 ausgewählt ist.

14. Verfahren nach Anspruch 11, wobei das Vorliegen erhöhter oder verringerter Spiegel, wie in Tabelle 1 unter "Erkrankung" für die entsprechende circRNA definiert, für mindestens 10, bevorzugt mindestens 50, stärker bevorzugt mindestens 100 der mindestens 100, bevorzugt mindestens 150 und stärker bevorzugt 200 circRNAs ein Anzeichen für das Vorliegen der Alzheimer-Krankheit ist.

15. Verfahren nach einem der Ansprüche 9 bis 12, wobei die Spiegel aller circRNAs nachgewiesen werden, die eine Sequenz umfassen, die von einer Sequenz codiert wird, die mindestens 70% Identität mit einer Sequenz aufweist, die aus der Gruppe bestehend aus SEQ ID NO:1 bis SEQ ID NO:910 ausgewählt ist, oder aller circRNAs, die eine Sequenz aufweisen, die von einer Sequenz codiert wird, die mindestens 70% Identität mit einer Sequenz aufweist, die aus der Gruppe bestehend aus SEQ ID NO:911 bis SEQ ID NO:1820 ausgewählt ist, wobei bevorzugt das Vorliegen erhöhter oder verringerter Spiegel wie in Tabelle 1 unter "Erkrankung" für mindestens 10, bevorzugt mindestens 50, stärker bevorzugt mindestens 100 der entsprechenden circRNAs definiert, ein Anzeichen für das Vorliegen der Alzheimer-Krankheit ist.

16. Kit für die Diagnose der Alzheimer-Krankheit, umfassend Mittel zum spezifischen Nachweis einer Nucleinsäuresequenz, die von SEQ ID NO:1 oder SEQ ID NO:911 codiert wird, oder einer Sequenz, die mindestens 70% identisch mit den aufgeführten Sequenzen ist,
wobei der Kit des Weiteren einen Behälter zum Sammeln von Vollblut umfasst,
wobei der Behälter stabilisierende Agentien umfasst, die bevorzugt aus der Gruppe bestehend aus Chelatbildnern, EDTA, K₂EDTA oder Kombinationen daraus ausgewählt sind.

17. Verwendung eines Kits nach Anspruch 16 zur Diagnose der Alzheimer-Krankheit.

## Revendications

1. Procédé pour diagnostiquer une maladie d'un sujet, comprenant l'étape de :
- détermination de la présence ou de l'absence d'un ou plusieurs ARN circulaires (ARNcirc) dans un échantillon d'un fluide corporel dudit sujet ;
dans lequel la présence ou l'absence desdits un ou plusieurs ARNcirc est indicatif de la maladie,
dans lequel la maladie est la maladie d'Alzheimer, et
dans lequel ledit fluide corporel est le sang, de préférence le sang total.

2. Procédé selon la revendication 1, dans lequel l'étape de détermination comprend :
- la détermination du niveau desdits un ou plusieurs ARNcirc ;
- la comparaison du niveau déterminé à un niveau témoin desdits un ou plusieurs ARNcirc ;
dans lequel des niveaux différents entre le niveau déterminé et le niveau témoin sont indicatifs de la maladie.

3. Procédé selon la revendication 2, dans lequel lesdits un ou plusieurs ARNcirc est ou sont exprimés de manière différente entre l'état pathologique et l'état non pathologique.

4. Procédé selon l'une quelconque des revendications 1 à 3, dans lequel l'ARNcirc est détecté par détection d'une jonction exon-exon dans un arrangement tête à queue.

5. Procédé selon la revendication 4, dans lequel l'ARNcirc est détecté au moyen d'un procédé choisi dans le groupe consistant en les procédés basés sur l'hybridation de sonde, les procédés basés sur l'amplification d'acide nucléique et le séquençage d'acide nucléique.

6. Procédé selon l'une quelconque des revendications 1 à 5, dans lequel l'échantillon est traité avec la RNase R avant la détermination de l'ARNcirc.

7. Procédé selon l'une quelconque des revendications 1 à 6, dans lequel plus d'un ARNcirc provenant d'une série d'ARNcirc sont déterminés.

8. Procédé selon la revendication 7, dans lequel ladite série comprend une pluralité d'ARNcirc qui ont été identifiés comme étant présents à des niveaux différents dans des échantillons de fluides corporels de patients ayant la maladie et de patients n'ayant pas la maladie, de préférence identifiés par analyse en composante principale ou regroupement.

9. Procédé selon l'une quelconque des revendications 2 à 8, dans lequel lesdits un ou plusieurs ARNcirc comprend ou comprennent une séquence codée par une séquence qui est identique à au moins 70 % à une séquence choisie dans le groupe consistant en les nucléotides 11 à 30 de l'une quelconque des séquences de SEQ ID NO : 1 à SEQ ID NO : 910, de préférence dans lequel la présence de niveaux augmentés ou diminués comme défini dans le tableau 1 sous « maladie » pour l'ARNcirc comprenant la séquence respective est indicatif de la présence de la maladie d'Alzheimer.

10. Procédé selon la revendication 9, dans lequel lesdits un ou plusieurs ARNcirc a ou ont une séquence codée par une séquence ayant au moins 70 % d'identité avec une séquence choisie dans le groupe consistant en SEQ ID NO : 911 à SEQ ID NO : 1820, de préférence dans lequel la présence de niveaux augmentés ou diminués comme défini dans le tableau 1 sous « maladie » pour l'ARNcirc ayant la séquence respective est indicatif de la présence de la maladie d'Alzheimer.

11. Procédé selon l'une quelconque des revendications 2 à 9, dans lequel les niveaux d'au moins 100, de préférence d'au moins 150 et de préférence encore d'au moins 200 ARNcirc comprenant une séquence codée par une séquence qui est identique à au moins 70 % à une séquence choisie dans le groupe consistant en SEQ ID NO : 1 à SEQ ID NO : 910 sont déterminés dans ledit échantillon d'un fluide corporel dudit sujet et contrôlés par rapport au niveau témoin respectif.

12. Procédé selon la revendication 11, dans lequel lesdits au moins 100, de préférence au moins 150 et de préférence encore au moins 200 ARNcirc ont une séquence codée par une séquence choisie dans le groupe consistant en SEQ ID NO : 911 à 1820, ou une séquence qui est identique à au moins 70 % à celle-ci.

13. Procédé selon la revendication 11 ou 12, dans lequel lesdits au moins 100, de préférence au moins 150 de préférence encore au moins 200 ARNcirc comprennent une séquence codée par une séquence ayant au moins 70 % d'identité avec une séquence choisie dans le groupe consistant en SEQ ID NO : 1 à SEQ ID NO : 200, ou lesdits ARNcirc ont une séquence ayant au moins 70 % d'identité avec une séquence choisie dans le groupe consistant en SEQ ID NO : 911 à 1110.

14. Procédé selon la revendication 11, dans lequel la présence de niveaux augmentés ou diminués comme défini dans le tableau 1 sous « maladie » pour l'ARNcirc respectif pour au moins 10, de préférence au moins 50, de préférence encore au moins 100 desdits au moins 100, de préférence au moins 150 et de préférence encore au moins 200 ARNcirc est indicatif de la présence de la maladie d'Alzheimer.

15. Procédé selon l'une quelconque des revendications 9 à 12, dans lequel les niveaux de tous les ARNcirc comprenant une séquence codée par une séquence ayant au moins 70 % d'identité avec une séquence choisie dans le groupe consistant en SEQ ID NO : 1 à SEQ ID NO : 910 ou tous les ARNcirc ayant une séquence codée par une séquence ayant au moins 70 % d'identité avec une séquence choisie dans le groupe consistant en SEQ ID NO : 911 à SEQ ID NO : 1820 sont détectés, dans lequel de préférence la présence de niveaux augmentés ou diminués comme défini dans le tableau 1 sous « maladie » pour au moins 10, de préférence au moins 50, de préférence encore au moins 100 des ARNcirc respectifs est indicatif de la présence de la maladie d'Alzheimer.

16. Kit pour diagnostiquer la maladie d'Alzheimer, comprenant des moyens pour détecter spécifiquement une séquence d'acide nucléique codée par SEQ ID NO : 1 ou SEQ ID NO : 911, ou une séquence qui est identique à au moins 70 % aux séquences citées,
dans lequel le kit comprend en outre un récipient pour recueillir du sang total, ledit récipient comprenant des agents stabilisants choisis de préférence dans le groupe consistant en les agents chélatants, l'EDTA, le K₂EDTA, ou leurs combinaisons.

17. Utilisation d'un kit selon la revendication 16 pour le diagnostic de la maladie d'Alzheimer.
